# EUROPEAN PATENT SPECIFICATION

(11) **EP 1 908 391 B1**
(45) Date of publication and mention of the grant of the patent: **01.09.2010**
(21) Application number: 06781450.9
(22) Date of filing: 21.07.2006
(51) Int. Cl.: A61B 1/00, A61B 1/012

(54) **ENDOSCOPE, TREATMENT DEVICE FOR ENDOSCOPE, AND ENDOSCOPE SYSTEM**
ENDOSKOP, BEHANDLUNGSVORRICHTUNG FÜR EIN ENDOSKOP UND ENDOSKOPSYSTEM
ENDOSCOPE, APPAREIL DE TRAITEMENT POUR ENDOSCOPE ET SYSTEME D'ENDOSCOPE

(30) Priority: 22.07.2005 JP 2005213482
(43) Date of publication of application: 09.04.2008
(73) Proprietor: Olympus Corporation, Tokyo 151-0072 (JP)
(72) Inventor: ICHIKAWA, Hiroaki, Kanagawa-ken (JP); KAWASHIMA, Koichi, 1920375 (JP)
(74) Representative: von Hellfeld, Axel
(86) International application number: PCT/JP2006/314528
(87) International publication number: WO 2007/011040

(56) References cited:
- EP-A- 0 072 689
- EP-A- 1 736 103
- WO-A-2006/124489
- DE-A1- 19 858 375
- JP-A- 6 054 801
- JP-A- 2003 093 393
- JP-A- 2003 290 125
- JP-A- 2004 057 815
- JP-A- 2004 261 349
- US-A- 5 538 008

## Description

### TECHNICAL FIELD

The present invention relates to an endoscope, an endoscope instrument, and an endoscope system.

### Background Art

In conventionally known endoscopic measurements, an endoscope instrument, e.g., a forceps is inserted into a forceps channel formed in an endoscope inserted into a body. For example, an endoscope instrument has an elastic elongate insertion section. A distal end therapeutic section capable of opening and closing an autopsy cup is disposed on the distal end section. A maneuvering section maneuvered by a surgeon is disposed on the proximal end of the insertion section.

In an endoscope instrument for use in operations (hereinafter called serial biopsy) to obtain samples of living tissue from a body, an insertion section has a dual-tube structure in which an inner tube is disposed in a sheath so that the insertion section can undertake water-supply and suctioning. See, for example, Japanese Unexamined Patent Application, First Publication No. 2003-93393. The endoscope instrument of this type upon capturing a living tissue by the autopsy cup supplies water, e.g., normal saline solution to the autopsy cup through a gap between the sheath and the inner tube, and suctions the living tissue together with the normal saline solution from the inner tube; thus, the living tissue is collected by a tissue-grasping device disposed to a maneuvering section. Provided to the maneuvering section of the endoscope instrument for that purpose are the tissue-grasping device and a mouthpiece that receives a water-supplying syringe. Furthermore, the maneuvering section is connected to a suction device through a tube.

However, the conventional endoscope instrument of this type has problems as follows.
Complex structure including the dual-tubed insertion section and the tissue-grasping device attached to the maneuvering section causes increase in production cost. The increase in production cost is particularly a problem in a case of disposable endoscope instruments.
Operations of an assistant who must keep a living tissue in biopsy cups and conduct suctioning and water-supply are so complex that the assistant endures overload in operating the endoscope instrument.
There is a limit in narrowing the diameter of the insertion section while maintaining necessary water-supply and suctioning in amount, i.e., the diameter of a channel in an endoscope must increase in accordance with an increased diameter of the endoscope insertion section of the endoscope.
JP 06054801 A relates to an endoscope with multifunction treating implement, wherein in the tip of an inserting part, a treatment unit is provided, and its treatment unit consists of a freely curvable arm part, and plural end effectors attached to this arm part so as to be freely attachable, detachable and changeable. Also, this endoscope is provided with a driving means for operating the treatment unit, a mechanism for controlling an operation of the driving means in accordance with an input of an input means for instructing an operation executed by this driving means, and connecting and separating the arm part and the end effector, a carrying passage for a change treating implement for guiding a transfer of the end effector, and a transfer driving means for collection and supply between a treating implement changing mechanism for containing plural treating implements, and also, supplying the selected treating implement to the carrying passage and the treatment unit.

### DISCLOSURE OF INVENTION

The present invention was conceived in consideration of the aforementioned circumstances, and the primary an object thereof is operability and enable low cost serial biopsy.
A first invention for overcoming the above objects is an endoscope that includes: an endoscope insertion section inserted into a human body and used there; and an endoscope-maneuvering section maneuvered by a surgeon in the exterior of the human body. A channel that allows inertion of an endoscope instrument therethrough is formed from a distal end section of the endoscope insertion section to the endoscope-maneuvering section. A tissue-suctioning pipeline is connected to the vicinity of an opening of the distal end of the channel. The tissue-suctioning pipeline is capable of connecting to a suction source that suctions a living tissue captured by the endoscope instrument.
This endoscope collecting a living tissue using a tissue-suctioning pipeline provided in the endoscope is different from a conventional endoscope that collects a living tissue through the inside of an endoscope instrument. The endoscope instrument upon capturing the living tissue is retracted to the vicinity of the proximal end relative to the tissue-suctioning pipeline, and then, the living tissue separated from the endoscope instrument by a suctioning force of the suction source is collected through the tissue-suctioning pipeline.

In a second invention of the present appoication associated with the endoscope of the first invention, a space is formed in the vicinity of the opening of the distal end of the channel, and the space is capable of allowing a forceps section provided to the distal end of the endoscope instrument to open and close therein.
This endoscope upon capturing the living tissue with the forceps section and retracting the forceps section into the channel opens the forceps section. The living tissue suctioned by the suction source and separated from the forceps section is collected through the tissue-suctioning pipeline.

In a third invention of the present application associated with the endoscope of the second invention, a regulating section is provided for regulating the position of the forceps section of the endoscope instrument within the space.
The regulating section of the endoscope during the retraction of the forceps section into the channel subsequent to capturing of the living tissue regulates the forceps section to come to a predetermined position suitable for collecting the living tissue through the tissue-suctioning pipeline.

In a fourth invention of the present application associated with the endoscope of the third invention, the regulating section is a abutment section that is provided to project into the channel and allows a part of the endoscope instrument to abut thereon.
The endoscope fixes the position of the forceps section based on a position where the endoscope instrument abuts to the abutment section. The living tissue can be suctioned and collected reliably by opening the forceps section since this position is suitable for the tissue-suctioning pipeline to collect the living tissue.

In a fifth invention of the present application associated with the endoscope of the third invention, the regulating section is provided in the vicinity of the opening section of the channel of the endoscope-maneuvering section, and the regulating section is capable of engaging with a part of the endoscope instrument.
A part of the endoscope instrument in the endoscope fixed to the channel by engaging the regulating section to the endoscope instrument is a reference point to regulate the distal end of the forceps section to come to a position suitable for collecting the living tissue through the tissue-suctioning pipeline. The regulating section provided in the vicinity of the proximity end facilitates maneuvering for positioning thereof.

In a sixth invention of the present application associated with the endoscope of the third invention, the regulating section has an abutment surface capable of abutting to the endoscope instrument in a direction orthogonal to an axial line of the channel where the direction indicates retraction of the endoscope instrument.
A part of the endoscope instrument of the endoscope abutting to the abutment surface that is orthogonal to an axial line during the retraction of the forceps section into the channel subsequent to capturing of the living tissue regulates the forceps section to come to a position suitable for collecting the living tissue through the tissue-suctioning pipeline.

In a seventh invention of the present application associated with the endoscope of the third invention, the regulating section has a rasing stand provided to be capable of freely raising in the channel, a long length of sheath of the endoscope instrument can be inserted through the rasing stand, and an engageable notch is formed to the forceps section provided to the distal end of the sheath.
This endoscope upon capturing the living tissue with the forceps section raises the rasing stand prior to retracting of the forceps section into the channel. The retracted state of forceps section engages with the rasing stand and is suspended there while the sheath passes through the notce of the rasing stand. Opening this state of the forceps section positioned suitable for collecting the living tissue through the tissue-suctioning pipeline causes the living tissue to be suctioned and collected into the tissue-suctioning pipeline.

In an eighth invention of the present application associated with the endoscope of the second invention, a sensor for detecting the position of the forceps section of the endoscope instrument is provided in the channel, the sensor being directed to the inside of the channel.
The sensor provided to the endoscope can detect insertion amount of the endoscope instrument. Inspecting a detection signal, put out from the sensor, indicative of the suitable position of the forceps section to collect the living tissue allows the forceps section to be positioned to collect the living tissue through the tissue-suctioning pipeline.

In a ninth invention of the present application associated with the endoscope of the second invention, the space is formed by widening the distal end section of the channel.
The diameter of the whole endoscope insertion section will not be significant since only the distal end section of the channel is widened in this endoscope.

In a tenth invention of the present application associated with the endoscope of the second invention, the space is formed from a material harder than the other part of the channel.
The channel in this endoscope will not be deformed or damaged by a forceps section that has made contact with an inner periphery of the channel defining the space while opening or closing in the channel.

In an eleventh invention of the present application associated with the endoscope of the first invention, a tissue-grasping device for capturing a living tissue collected by the endoscope instrument is provided between the tissue-suctioning pipeline and the suction source.
The tissue-grasping device provided in a tissue-suctioning pipeline in the endoscope can capture the living tissue on a capturing surface of the tissue-grasping device by merely suctioning the living tissue into the tissue-suctioning pipeline.

In a twelfth invention of the present application associated with the endoscope of the first invention, a tissue water-supply line is provided that is capable of connecting to a water-supplying tank and supplying a liquid stored in the water-supplying tank to the channel.
In this endoscope, the distal end therapeutic section of the endoscope instrument upon capturing the living tissue is retracted into the channel, and then water is supplied from the water-supplying tank through the tissue water-supply pipeline to the channel. The supplied water separates the living tissue from the distal end therapeutic section. Both the supplied water and the living tissue suctioned into the tissue-suctioning pipeline are collected in the exterior of the human body.

A thirteenth invention of the present application associated with the endoscope of the twelfth invention has a synchronization structure that conducts water-supply through the tissue water-supply line and suctioning through the tissue-suctioning pipeline synchronously.
Activating the synchronization structure in the endoscope undertakes water-supply and suctioning, thus, the living tissue is collected. For example, the synchronization structure is configured to start water-supply and suctioning simultaneously, or to start suctioning prior to water-supply.

In a fourteenth invention of the present application associated with the endoscope of the first invention, suction amount of the tissue-suctioning pipeline is greater than water-supply amount of the tissue water-supply line.
The significant suction amount of the tissue-suctioning pipeline in the endoscope allows the liquid supplied to the channel and the living tissue separated from the forceps section to be suctioned into the tissue-suctioning pipeline reliably, thereby collecting the liquid and the tissue in the exterior of the human body.

The present application also discloses an endoscope instrument wherein a long length of insertion section that is inserted through a channel of an endoscope extends from a maneuvering section that undertakes maneuvering of a surgeon, a distal end therapeutic section for collecting a living tissue is provided to a distal end section of the insertion section, and a distal-end-regulating section for regulating the position of the distal end therapeutic section is provided in a retracting direction of the distal end therapeutic section subsequent to the insertion of the insertion section through the channel.
The endoscope instrument upon capturing the living tissue with the distal end therapeutic section retracts the living tissue into the endoscope. The living tissue can be collected at a position where this state of distal-end-regulating section fixes the position of the distal end therapeutic section at a predetermined position in the endoscope.

The present application is further associated with an endoscope instrument wherein the insertion section has an inner sheath and an outer sheath covering an outer periphery of the inner sheath. The outer sheath is capable of freely sliding on the outer periphery of the inner sheath. The outer sheath is configured to be capable of being positioned relative to the endoscope, and to be capable of engaging with the distal end therapeutic section in a retracting direction of the distal end therapeutic section.
The outer sheath is insertied through the channel of the endoscope of the endoscope instrument and is positioned relative to the endoscope. The current position of the distal end of the outer sheath is a reference position. Subsequently, the distal end therapeutic section retracted into the endoscope upon capturing the living tissue is abutted to the outer sheath. This allows the distal end to suspend at a position fixed through the outer sheath relative to the endoscope and to collect the living tissue at this position.

With the endoscope instrument, an engagement member is provided having an end section thereof fixed to the outer sheath and the other end section capable of engaging with the endoscope.
The endoscope instrument while being inserted into the channel of the endoscope fixes the distal end of the outer sheath relative to the endoscope by engaging the engagement member extending from the outer sheath with the endoscope. Consequently, the positioning to the distal end therapeutic section can be carried out based on the reference position of the outer sheath.

With the endoscope instrument, projections and depressions capable of engaging with the endoscope are provided to the outer sheath.
The endoscope instrument while being inserted into the channel of the endoscope fixes the distal end of the outer sheath relative to the endoscope by engaging the outer sheath with the endoscope using the projections and depressions. Varing the engagement position of the projections and depressions in a longitudinal direction of the insertion section can adjust the position of the outer sheath in a case where the projections and depressions are provided in the longitudinal direction of the insertion section, or a plurality of sections that engage with the projections and depressions are provided in a longitudinal direction of the channel.

With the endoscope instrument, the insertion section has an inner sheath and an outer sheath covering an outer periphery of the inner sheath. The outer sheath is capable of freely sliding on the outer periphery of the inner sheath. A freely-projectirlg-and-recessing section is provided to at least a part of the distal end section of the outer sheath, and the outer diameter of the insertion section is increased by projecting the freely-projecting-and-recessing section.
This endoscope instrument fixes the position of the distal end of the outer sheath by engaging the freely-projecting-and-recessing section of the outer sheath within the channel of the endoscope. Water-supply to the distal end therapeutic section is conducted by using a section free from this state of the freely-projecting-and-recessing section.

With the endoscope instrument, the distal end therapeutic section has a section having a diameter greater than the diameter of the insertion section. The distal end therapeutic section is capable of engaging with a notch formed to a rasing stand provided in the channel of the endoscope.
The endoscope instrument upon capturing the living tissue with the distal end therapeutic section raises the rasing stand, and then retracts the distal end therapeutic section into the endoscope.

The present application further discloses an endoscope instrument wherein a long length of insertion section that is inserted through a channel of an endoscope extends from a maneuvering section that undertakes maneuvering of a surgeon, a distal end therapeutic section for collecting a living tissue is provided to a distal end section of the insertion section, the insertion section has an identification member used for regulating insertion amount of the insertion section, the identification member is positioned so that the distal end therapeutic section is positioned in the vicinity of the proximal end relative to a point where a tissue-suctioning pipeline of the endoscope connected to a suction source is connected to the distal end section of the channel.
Making use of the identification member can allow the position of the distal end therapeutic section to be fixed since the insertion amount of the insertion section is recognized by the indentification member during insertion into the endoscope or retraction upon capturing the living tissue.

The present application also discloses an endoscope instrument, wherein a long length of insertion section that is inserted through a channel of an endoscope extends from a maneuvering section that undertakes maneuvering of a surgeon, a distal end therapeutic section for collecting a living tissue is provided to a distal end section of the insertion section, and a tissue-grasping device is provided to the maneuvering section. The tissue-grasping device is capable of connecting to a tissue-suctioning pipeline provided in the endoscope in order to communicate to the distal end section of the channel. The tissue-grasping device capturs a living tissue conveyed from the distal end therapeutic section through the tissue-suctioning pipeline.
The endoscope instrument undertaking water-supply and suctioining using pipelines in the endoscope introduces the living tissue toward the maneuvering section of the endoscope instrument while collecting the living tissue, and then the living tissue is captured by the tissue-grasping device provided in the maneuvering section. This configuration free from the pipelines used for water-supply and suctioning simplifies the configuration of the insertion section.

The present application also discloses an endoscope instrument wherein a long length of insertion section that is inserted through a channel of an endoscope extends from a maneuvering section that undertakes maneuvering of a surgeon, a distal end therapeutic section for collecting a living tissue is provided to a distal end section of the insertion section, and a suctioning-and-water-supplying operation section is provided to the maneuvering section. The suctioning-and-water-supplying operation section undertakes water-supply operation and suctioning operation. The water-supply operation conducts supplying of water to the channel of the endoscope and separating of a living tissue from the distal end therapeutic section. The suctioning operation conducts suctioning of the separated living tissue.
In the endoscope instrument, the pipeline for supplying water to the living tissue and the pipeline for suctioning the living tissue are provided mainly in the endoscope, and the suctioning-and water-supplying operation section that undertakes water-supply and suctioning through these lines is provided in the endoscope instrument. Therefore, the surgeon who maneuvers the endoscope instrument can collect the living tissue in the exterior of the numan body.

With the endoscope instrument, a pair of biopsy cups are provided to the distal end therapeutic section that are capable of freely opening and closing. A slider that opens and closes the biopsy cups is provided to the maneuvering section. The slider is capable of advancing and retracting. The suctioning-and water-supplying operation section is configured to operate synchronously in accordance with the advancement and the retraction of the slider. The suctioning-and water-supplying operation section is in an operable state that conducts water-supply and suctioning when the slider is at a position that opens the biopsy cups. The suctioning-and water-supplying operation section is in a suspended state that suspends the water-supply and the suctioning conducted by the suctioning-and water-supplying operation section when the slider is at a position that closes the biopsy cups.
Extending and retracting of the slider of the maneuvering section of the endoscope instrument allow the biopsy cups to open and close, and simultaneously carry out water-supply to the distal end therapeutic section in the endoscope and suctioining from the channel. Therefore, operations conducted by the endoscope instrument can be simplified.

The present application further discloses an endoscope system that includes: an endoscope having an endoscope insertion section extending from the endoscope; and an endoscope instrument used to be inserted through a channel formed in the endoscope. The endoscope is inserted into a human body from an endoscope-maneuvering section maneuvered in the exterior of the human body by an surgeon. The endoscope instrument has a distal end therapeutic section for collecting a living tissue provided to the distal end section of a long length of insertion section. The endoscope instrument has a maneuvering section at the proximal end section of the insertion section. The endoscope is configured to separate a living tissue from the distal end therapeutic section retracted into the channel by supplying water to the channel. A tissue-suctioning pipeline that suctions the living tissue is connected to the vicinity of the distal end of the channel. A distal-end-regulating section is provided in the vicinity of the channel relative to a connection position of the tissue-suctioning pipeline. The distal-end-regulating section regulates the position of the distal end therapeutic section.
The endoscope system collects the living tissue captured by the endoscope instrument in the exterior of the human body by using the tissue-suctioning pipeline provided in the endoscope. Since this state of the distal end therapeutic section is regulated by the distal-end-regulating section and positioned there, the living tissue can be separated from the distal end therapeutic section and collected from the tissue-suctioning pipeline reliably.

With the endoscope system, the distal-end-regulating section is a projecting section formed to the endoscope instrument, and an abutment section abutting to the projecting section is formed in the channel.
This endoscope system fixes the position of the distal end therapeutic section relative to the endoscope by abutting a projecting section of the endoscope instrument to the abutment section.

The present application further discloses an endoscope system that includes: an endoscope having an endoscope insertion section extending from the endoscope; and an endoscope instrument used to be inserted through a channel formed in the endoscope. The endoscope is inserted into a human body from an endoscope-maneuvering section maneuvered in the exterior of the human body by an surgeon. The endoscope instrument has a distal end therapeutic section for collecting a living tissue provided to the distal end section of a long length of insertion section. The endoscope instrument has a maneuvering section at the proximal end section of the insertion section wherein the endoscope is configured to separate a living tissue from the distal end therapeutic section retracted into the channel by supplying water to the channel. A tissue-suctioning pipeline that suctions the living tissue is connected to the vicinity of the distal end of the channel. At least one of a tissue-grasping device and a suctioning-and-water-supplying operation section is provided in the endoscope. The tissue-grasping device collects the riving tissue through the tissue-suctioning pipeline. The suctioning-and-water-supplying operation section undertakes water-supply and suctioning for collecting the living tissue.
The endoscope system, upon retracting the endoscope instrument that has captured the living tissue into the channel of the endoscope, conducts water-supply and suctioning by using pipelines provided in the endoscope and collects the living tissue. The suctioning-and water-supplying operation section that undertakes this case of water-supply and suctioning in the endoscope allows operations to be shared by the endoscope and the endoscope instrument. In addition, providing the tissue-grasping device to the endoscope further simplifies the configuration of the endoscope instrument.

Since a pipeline for collecting the living tissue is provided in the endoscope or the endoscope system according to the present invention, the diameter of the channel can be narrower than that of a pipeline formed in a conventional endoscope instrument.
Cost of the endoscope instrument can be reduced. Configuration to acknowledge the position of the distal end therapeutic section, e.g., providing an abutment section to the endoscope permits reliable collection of the living tissue.
Such an endoscope instrument or endoscope system allows the distal end therapeutic section to be positioned in the endoscope can ensure operations, e.g., collecting of the living tissue using pipelines in the endoscope.

### BRIEF DESCRIPTION OF DRAWINGS

FIG. 1 is a general view of a structure of the endoscope system according to embodiments of the present invention.
FIG. 2 illustrates pipelines between an instrument of an endoscope system and an endoscope.
FIG. 3 is a diagram showing the structure of a first switching device.
FIG. 4 illustrates a leak hole of an air-and-water-supplying button that is blocked by a finger.
FIG. 5 illustrates an air-and-water-supplying button that undergoes first press.
FIG. 6 illustrates the air-and-water-supplying button that undergoes secondary press.
FIG. 7 is a diagram showing the structure of second switching device.
FIG. 8 illustrates the air-and-water-supplying button that undergoes first press.
FIG. 9 illustrates the air-and-water-supplying button that undergoes secondary press.
FIG. 10 is a perspective view illustrating arrangement of the air-and-water-supplying button and a suction button.
FIG. 11 is a side view of FIG. 10.
FIG. 12 illustrates an air-and-water-supplying button that alone undergoes a first press.
FIG. 13 illustrates the air-and-water-supplying button that alone undergoes first press.
FIG. 14 illustrates the air-and-water-supplying button and the air-and-water-supplying button that undergo secondary press.
FIG. 15 illustrates ordinary water-supplying pipeline.
FIG. 16 illustrates air-supplying paths for supplying air.
FIG. 17 illustrates ordinary suctioning paths.
FIG. 18 illustrates a distal end therapeutic section compressed to a mucosa.
FIG. 19 illustrates a living tissue, that is a part of the mucosa, grasped by the distal end therapeutic section.
FIG. 20 illustrates paths for supplying water to the grasped tissue and for suctioning the grasped tissue.
FIG. 21 is a schematic view illustrating how to suction the grasped tissue.
FIG. 22 illustrates pipelines between an instrument of an endoscope system and an endoscope.
FIG. 23 illustrates a distal end therapeutic section compressed to a mucosa.
FIG. 24 illustrates a living tissue, that is a part of the mucosa, grasped by the distal end therapeutic section.
FIG. 25 illustrates the retracted state of the whole instrument of FIG. 24.
FIG. 26 illustrates the whole instrument extended subsequently from the state of FIG. 25.
FIG. 27 illustrates the biopsy cups that subsequently open.
FIG. 28 is a schematic view that illustrates how to suction the grasped tissue by retracting the whole instrument while keeping the opening state of biopsy cups and by causing the biopsy cups to make contact with a gap section.
FIG. 29 illustrates pipelines between an instrument of an endoscope system and an endoscope.
FIG. 30 illustrates a distal end chip of the instrument making contact with an abutment section of a forceps channel.
FIG. 31 is a perspective view of the distal end chip.
FIG. 32 is a process of inserting the instrument through the forceps channel.
FIG. 33 illustrates the coil sheath and the distal end therapeutic section extruded from the outer sheath and compressed to the mucosa while the outer sheath remains .
FIG. 34 illustrates a living tissue, that is a part of the mucosa, grasped by the distal end therapeutic section.
FIG. 35 is a schematic view illustrating how to suction the grasped tissue.
FIG. 36 illustrates another example of a connecting state of a tissue-suctioning pipeline and a chamber.
FIG. 37 illustrates pipelines between an instrument of an endoscope system and an endoscope.
FIG. 38 is a diagram showing the structure of a first switching device.
FIG. 39 illustrates a leak hole of an air-and-water-supplying button that is blocked by a finger.
FIG. 40 illustrates a pressed air-and-water-supplying button.
FIG. 41 is a diagram showing the structure of second switching device.
FIG. 42 illustrates a pressed a suction button.
FIG. 43 is a view observed along an arrow A of FIG. 37.
FIG. 44 illustrates pipelines between an instrument of an endoscope system and an endoscope.
FIG. 45 illustrates a sutructure for fixing the outer sheath by inserting a key into a key hole of the forceps plug.
FIG. 46 illustrates a structure for fixing the outer sheath by a ratchet.
FIG. 47 illustrates a sutructure for fixing the outer sheath to the forceps plug by a freely slidable catching plate.
FIG. 48 illustrates the outer sheath passing through an increased diameter section of the catching plate.
FIG. 49 is a cross-section illustrateing arrangement shown in FIG. 48.
FIG. 50 illustrates the outer sheath engaging with a reduced-diameter section of the catching plate.
FIG. 51 is a cross-section illustrateing arrangement shown in FIG. 50.
FIG. 52 is a cross-section illustrating a structure for fixing the outer sheath by a cover fixed to the outer sheath.
FIG. 53 is a cross-sectional view taken along a line B-B of FIG. 52.
FIG. 54 is a cross-section illustrating the cover engaging with a forceps port.
FIG. 55 is a cross-sectional view taken along a line C-C of FIG. 54.
FIG. 56 is an exploded perspective view illustrating how to screw the outer sheath into a forceps channel.
FIG. 57 is a perspective view illustrating how to screw the outer sheath into a forceps channel.
FIG. 58 illustrates pipelines between an instrument of an endoscope system and an endoscope.
FIG. 59 is a diagram showing the structure of a first switching device.
FIG. 60 illustrates an air-and-water-supplying button that undergoes a first press.
FIG. 61 illustrates the air-and-water-supplying button that undergoes a secondary press.
FIG. 62 is a diagram showing the structure of second switching device.
FIG. 63 illustrates a pressed a suction button.
FIG. 64 is a diagram showing the shape of a connector.
FIG. 65 is an enlarged view of a maneuvering section of the instrument.
FIG. 66 illustrates a turned-on state of a switch pressed by a slider of the maneuvering section.
FIG. 67 is a diagram showing the structure of the instrument.
FIG. 68 illustrates an increased-diameter section formed by advancing the outer sheath.
FIG. 69 is a diagram showing the pipelines in the endoscope.
FIG. 70 illustrates a distal end therapeutic section compressed to a mucosa.
FIG. 71 illustrates paths for supplying water to the grasped tissue and for suctioning the grasped tissue.
FIG. 72 is a schematic view illustrating how to suction the grasped tissue.
FIG. 73 illustrates another forms of a connector of the instrument and a connector of the endoscope.
FIG. 74 is a diagram showing the structure of the instrument.
FIG. 75 illustrates an increased-diameter section formed by advancing the slider of the outer sheath.
FIG. 76 illustrates a structure that engages the outer sheath to the coil sheath.
FIG. 77 illustrates an increased-diameter section formed by extracting the coil sheath relative to the outer sheath.
FIG. 78 illustrates a structure of a instrument in which the slider synchronizes with the outer sheath.
FIG. 79 illustrates an increased-diameter section formed by retracting the slider and moving the outer sheath.
FIG. 80 illustrates an instrument having an increased-diameter section in which the slider synchronizes with the outer sheath.
FIG. 81 illustrates an increased-diameter section housed by moving the outer sheath.
FIG. 82 illustrates pipelines between an instrument of an endoscope system and an endoscope.
FIG. 83 is an enlarged view illustrating a rasing stand and a distal end section of the instrument.
FIG. 84 is a cross-section illustrating the distal end section of the endoscope.
FIG. 85 illutrates a rasing stand raised by extruding and pressing the distal end therapeutic section to the mucosa.
FIG. 86 illustrates collecting of a grasped tissue by retracting the instrument with the raised state of the rasing stand.
FIG. 87 illustrates pipelines between an instrument of an endoscope system and an endoscope.
FIG. 88 is a diagram showing an example of a monitor display section.
FIG. 89 illustrates a configuration for detecting an insertion position using an electrical contact.
FIG. 90 illustrates a configuration for detecging an insertion position using a mark exposed outwardly from a forceps plug.
FIG. 91 is a diagram showing another form of the instrument.
FIG. 92 is an enlarged perspective view of a distal end section of the instrument.
FIG. 93 illustrates an operation for pressing a cutter of the instrument onto the mucosa and rotating the cutter.
FIG. 94 illustrates a living tissue of the mucosa captured in the cutter.
FIG. 95 illustrates suctioning and collecting of the captured tissue pushed out of the cutter.
FIG. 96 illustrates pipelines between an instrument of an endoscope system and an endoscope.

### Explanation of Reference Numerals and Symbols

1, 101, 201, 301, 601, 801, 901, and 1101:endoscope system
2, 102, 202, 302, 602, 802, 902, and 1102: endoscope
3, 203, 603, 803, 903, 1003, and 1103: instrument (endoscope instrument)
4: endoscope-maneuvering section
5: endoscope insertion section
13: water-supplying tank
14: suction source
15, 115, and 215: forceps channel (channel, space)
17, 317: tissue-grasping device
28, 328, and 628: tissue water-supply pipeline
46, 646, 1146: tissue-suctioning pipeline
52: connecting member (connecting structure)
74: slider
75, 275: insertion section
76: coil sheath (sheath, inner sheath)
77, 277, and 877: distal end therapeutic section (forceps section)
78, 278, and 878: forceps distal end section (distal-end-regulating section)
79: biopsy cup
116: chamber (space)
117, 217: abutment section (regulating section)
117A: abutment surface
280, 480, and 680: outer sheath
281: distal end chip (distal-end-regulating section)
283: projecting section
286: engagement member (distal-end-regulating section)
287: hook section (regulating section)
375: suctioning-and-water-supplying switch (suctioning-and-water-supplying operation section)
416: forceps plug (regulating section)
415A: flange section (regulating section)
420: key (regulating section)
430: ratchet (regulating section)
440: slit (regulating section)
441: catching plate (regulating section)
482, and 482A: engagement section (distal-end-regulating section)
450: cover (distal-end-regulating section)
454: catching plate (distal-end-regulating section)
501: female thread section (regulating section)
510: male-thread section (distal-end-regulating section)
684, and 716: increased-diameter section (distal-end-regulating section, freely-projecting-and-recessing section)
714: wire (distal-end-regulating section, freely-projecting-and-recessing section)
747: increased-diameter section (distal-end-regulating section)
748: sheath distal end section (distal-end-regulating section)
880: rasing stand (regulating section)
882: slit (notch)
910: photo-senser (sensor, regulating section)
913, and 940: mark (distal-end-regulating section)
930: conductive material (distal-end-regulating section)
931: electrical contact (sensor, regulating section)

### BEST MODE FOR CARRYING OUT THE INVENTION

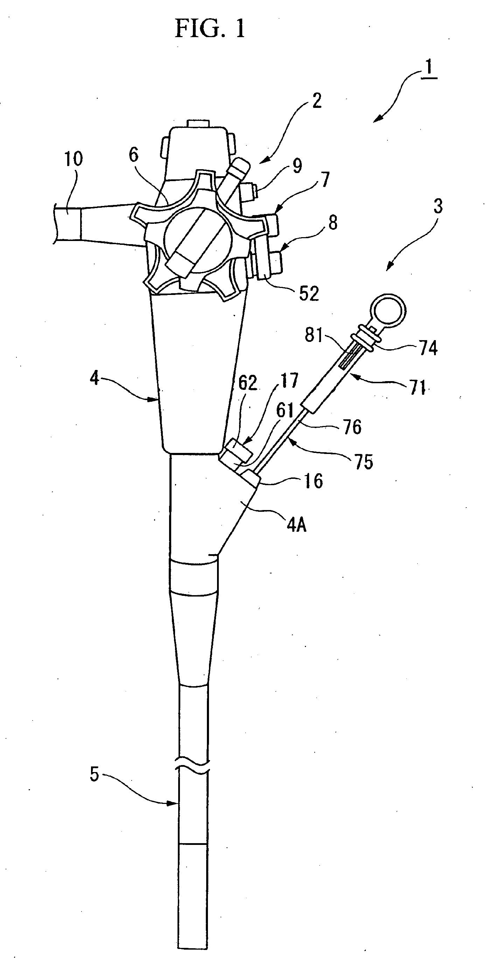

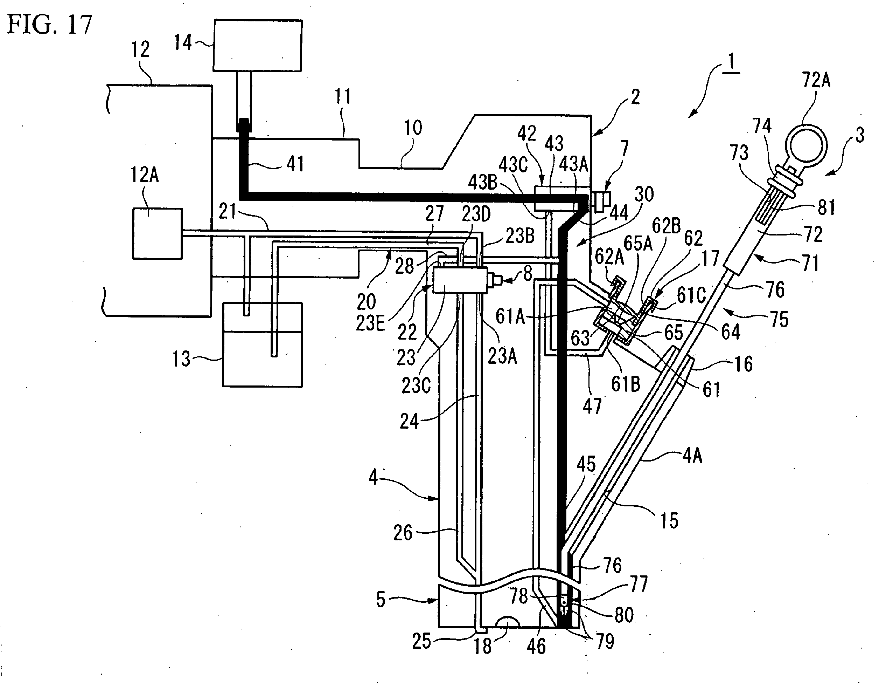

An embodiment according to the present invention is explained as follows in detail with reference to FIGS. 1 to 22.

### (First Embodiment)

FIG. 1 is a schematic view of an endoscope system according to a first embodiment of the present invention. An endoscope system 1 includes an endoscope 2; and an endoscope instrument 3 (hereinafter called instrument) inserted through a forceps channel of the endoscope 2.

The endoscope 2 has an endoscope-maneuvering section 4 grasped and maneuvered in the exterior of a human body by a surgeon. Extending from a lower end of the endoscope-maneuvering section 4 is an long length of elastic endoscope insertion section 5 that is inserted into the human body. Provided on an upper section of the endoscope-maneuvering section 4 are an angle knob 6 for adjusting the direction of the endoscope insertion section 5; and a plurality of various buttons 7, 8, and 9. Furthermore, a universal cable 10 extends from the endoscope-maneuvering section 4.

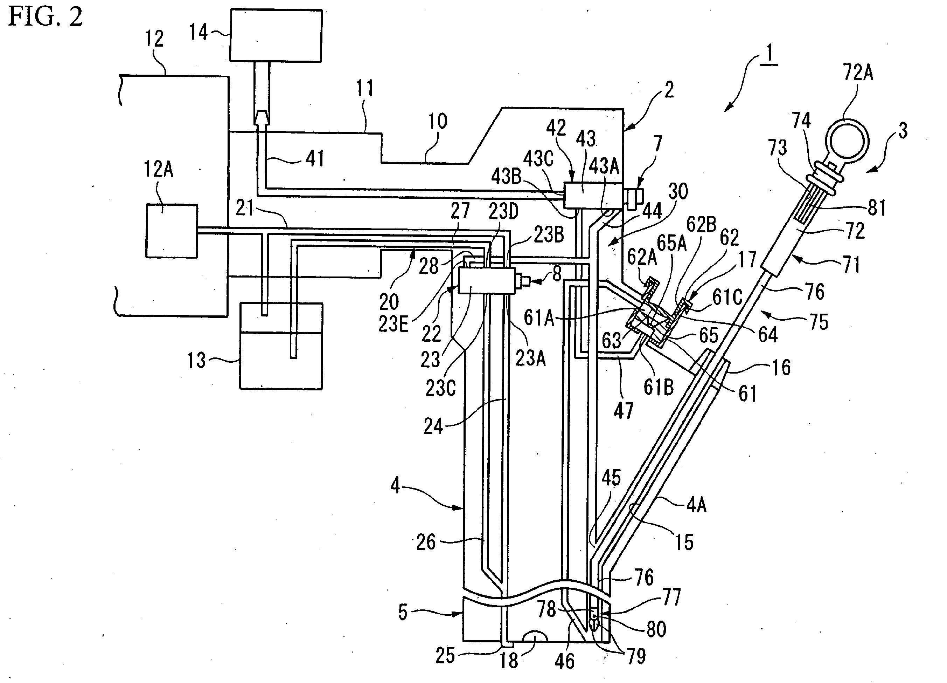

FIG. 2 schematically illustrates a connector 11 provided on an end section of the long length of universal cable 10. The endoscope 2 is connected to a controlling device 12, a water-supplying tank 13, and a suction source 14 via the connector 11. Provided on a lower lateral section 4A of the endoscope-maneuvering section 4 are a forceps plug 16 that covers the forceps port of the forceps channel 15; and a tissue-grasping device 17. The forceps channel 15 extends from the endoscope-maneuvering section 4 to the distal end of the endoscope insertion section 5 and has an opening on the distal end section of the endoscope insertion section 5. In addition, provided on the distal end section of the endoscope insertion section 5 are a lens 18 of an image-pickup section for capturing a body cavity; and a lighting device (not shown in the drawings).

Various pipelines, e.g., a water-supplying pipeline, a pipeline, and the forceps channel 15 are formed in the endoscope 2. At first, a first pipeline system 20 connected primarily to an air-supplying source 12A in the controlling device 12, or to a water-supplying tank 13 has an air-supplying pipeline 21 connected to the air-supplying source 12A. The air-supplying pipeline 21 branches off in the connector 11. One of the branched pipelines is inserted into the water-supplying tank 13 and has an opening at a position higher than a fluid level. Also, the other branched pipeline of the air-supplying pipeline 21 passing through the universal cable 10 is connected to a second port 23B of the first switching device 22.

A first switching device 22 having five ports switches flow paths. Connected to a first port 23A of the first switching device 22 is a air-supplying pipeline 24. The air-supplying pipeline 24 is connected to a nozzle 25 provided on the distal end section of the endoscope insertion section 5. The nozzle 25 has an opening directed to a lens 18 of an image-pickup section so that the lens 18 can be cleansed.
Connected to the vicinity of the distal end of the air-supplying pipeline 24 is a water-supplying pipeline 26 that undertakes liquid flow thereinside. The water-supplying pipeline 26 is connected to a third port 23C of the first switching device 22. Connected to a fourth port 23D of the first switching device 22 is a water-supplying pipeline 27, inserted into a water-supplying tank 13 through the universal cable 10 and the connector 11, has an opening at a lower position of the liquid level. Furthermore, connected to a fifth port 23E of the first switching device 22 is a tissue water-supply pipeline 28 that is connected to a second pipeline system 30 that is explained later.

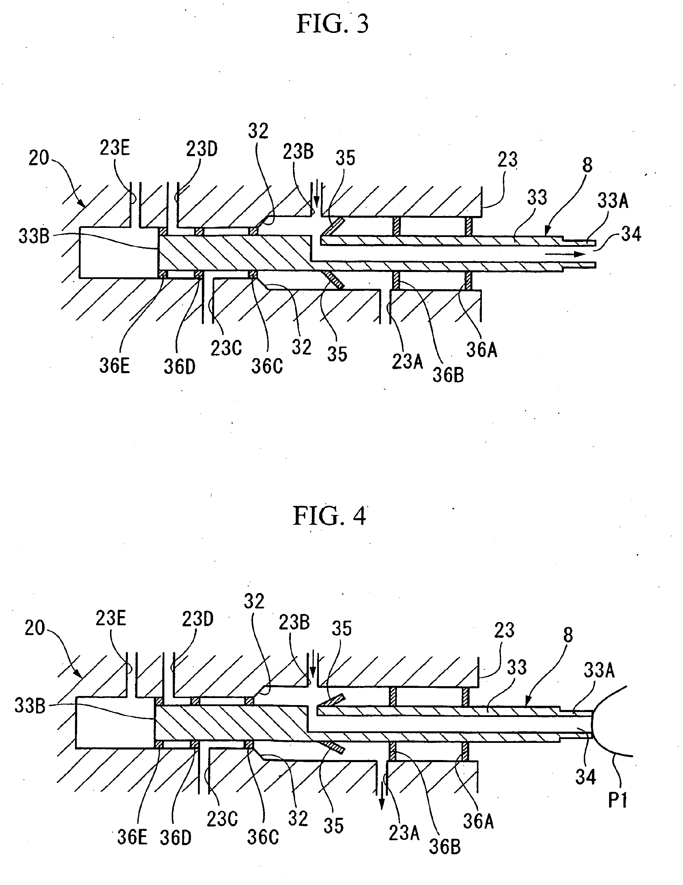

As illustrated in FIG. 3, the first switching device 22 has an elongate sleeve 23 having an opening section on an end section thereof. An air-and-water-supplying button 8 capable of freely extending and retracting is inserted from the opening section. The sleeve 23 is increased in diameter by a taper surface 32 that opens toward the opening section in a middle of a direction from a closed end to an opening end. Fored in an in an axial line direction of the sleeve 23 in an order from the opening section are a first port 23A, a second port 23B, a third port 23C, a fourth port 23D, and a fifth port 23E. It should be noted that the previously explained taper surface 32 is provided between the position where the second port 23B is formed and the position where the third port 23C is formed.

A air-and-water-supplying button 8 has an elongate button main unit 33. A head section 33A exposed outward of the button main unit 33 having a reduced diameter and has an opening, i.e., a leak hole 34. The leak hole 34 extends in parallel with an axial line of the button main unit 33 and has an opening reaching the distal end section 33B at a lateral section of the button main unit 33. The check valve 35 projects outward in a radial direction of the button main unit 33 close to a head section 33A relative to the opening of the leak hole 34.
A packing 36A and a packing 36B are fixed at a predetermined interval from each other close to the head section 33A relative to the check valve 35 in the button main unit 33 so that an airtight structure is formed among the packings 36A and 36B and the sleeve 23. A packing 36C, packing 36D, and a packing 36E are fixed in an axial line direction at a predetermined interval from each other close to the distale end section 33B relative to the check valve 35 so that a watertight structure is formed by the packings 36C to 36E and the sleeve 23.

FIG. 3 illustrates a retracted state of the air-and-water-supplying button 8 where the check valve 35 is positioned between a first port 23A and a second port 23B of the sleeve 23. The packing 36C is located between the second port 23B and the third port 23C. The packing 36D is located between the third port 23C and a fourth port 23D. The packing 36E is located between the fourth port 23D and a fifth port 23E.
Air supplied from the second port 23B to each of the non-communicating state of the ports 23A to 23E port 23E is discharged from the leak hole 34. Blocking the leak hole 34 by a finger P1 as illustrated in FIG. 4, air supplied from the second port 23B pushes to open the check valve 35, thereby causing the first port 23A to be communicated to the second port 23B.

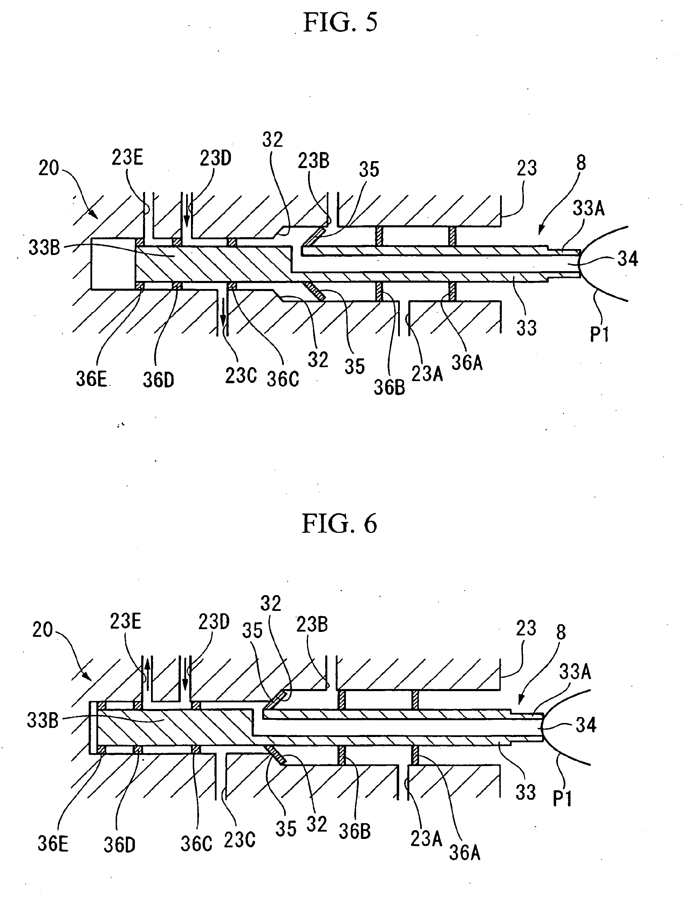

as illustrated in FIG. 5, a first press to the air-and-water-supplying button 8 causes the packing 36D to remain close to the opening section relative to the first port 23A while moving the packing 36B to inbetween the first port 23A and the second port 23B; and while moving the check valve 35 to inbetween the second port 23B and the third port 23C. The packing 36C remains between the second port 23B and the third port 23C, and the packing 36D moves to between the fourth port 23D and the fifth port 23E. This results in that the third port 23C communicates to the fourth port 23D.

As illustrated in FIG. 6, further pressing the air-and-water-supplying button 8, i.e., a secondary press to the air-and-water-supplying button 8 causes the packing 36A to remain close to the opening section relative to the first port 23A and the packing 36B to remain between the first port 23A and the second port 23B. The check valve 35 makes contact with a taper surface 32. The packing 36C moves to between the third port 23C and the fourth port 23D. The packing 36D ande packing 36E move close to the other end, i.e., the dead end of the sleeve 23 relative to the fifth port 23E. This results in that the fourth port 23D communicates to the fifth port 23E.
Blocking the leak hole 34 by the finger P1 does not cause the second port 23B to communicate to the third port 23C since an area of the check valve 35 that receives pressure upon making contact with the taper surface 32; therefore the leak hole 34 should be blocked by the finger P1 when pressing the air-and-water-supplying button 8.

As illustrated in FIG. 2, a second pipeline system 30 has a suction pipeline 41 that is connected to a suction source 14. The suction pipeline 41 passing through the connector 11 and the universal cable 10 is connected to a third port 43C of the second switching device 42. A second switching device 42 having three ports switches flow paths. Connected to a first port 43A of the second switching device 42 is a suction pipeline 44. The suction pipeline 44 has the tissue water-supply pipeline 28 of the previously-explained first pipeline system 20 on its way to a connecting point 45 where the suction pipeline 44 is connected to the forceps channel 15.
The forceps channel 15 has an opening at the distal end section of the endoscope insertion section 5. A tissue-suctioning pipeline 46 is connected to the vicinity of the opening section formed on the distal end of the forceps channel 15. The tissue-suctioning pipeline 46 connected to the forceps channel 15 in a slanting manner is further connected to a tissue-suctioning pipeline 47 via a tissue-grasping device 17 in the endoscope-maneuvering section 4. The tissue-suctioning pipeline 47 is connected to the second port 43B of the second switching device 42.

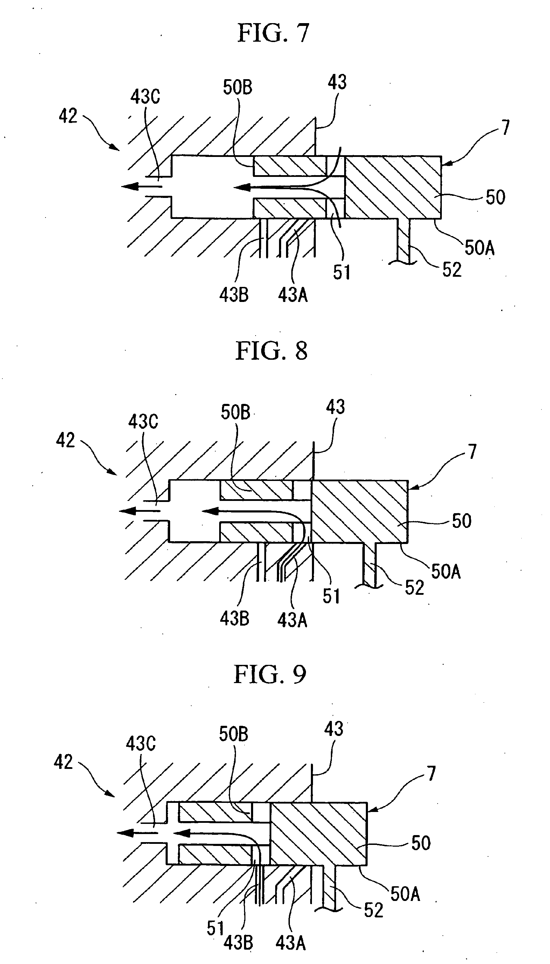

As illustrated in FIG. 7, the second switching device 42 has an elongate sleeve 43 having an opening section on an end section thereof. A suction button 7 maintaing an airtight structure and capable of freely extending and retracting is inserted into the opening section. The first port 43A and the second port 43B formed at a predetermined interval from each other in order from the opening section are disposed in an axial line direction toward a lateral part of the sleeve 43. A third port 43C is formed on the other end, i.e., the dead end of the sleeve 43.
The suction button 7 has an elongate button main unit 50. A communication hole 51 is formed on an outwardly exposed part of the most retracted state of button main unit 50. The opening of the communication hole 51 formed on the distal end section 50B of the button main unit 50 is directed to the other end of the sleeve 343. The button main unit 50 that is retracted the most in length is configured to block the first and second ports 43A and 43B.

As illustrated in FIG. 8, the suction button 7 in the first press causes the communication hole 51 pushed in the sleeve 43 to communicate to the first port 43A. This results in that the first port 43A communicates to the third port 43C. As illustrated in FIG. 9, further pressing the suction button 7,i.e., a secondary press to the suction button 7 blocks the first port 43A; thus the second port 43B communicates to the third port 43C through the communication hole 51.

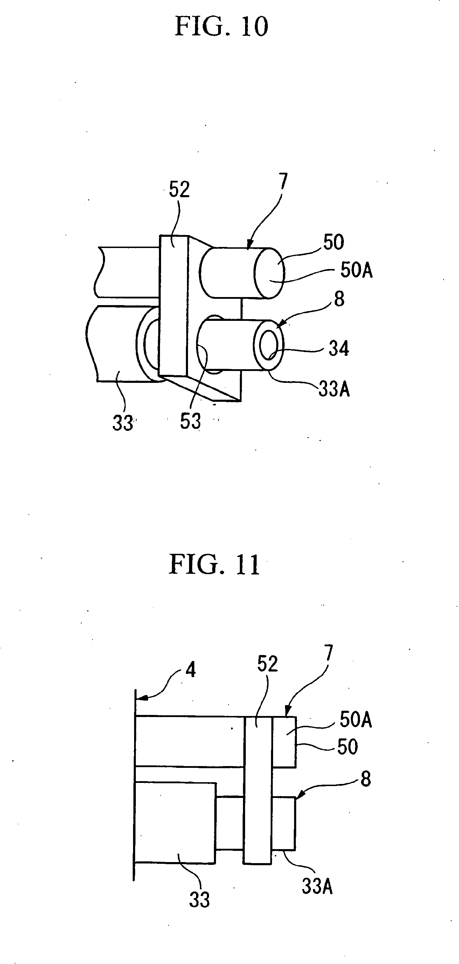

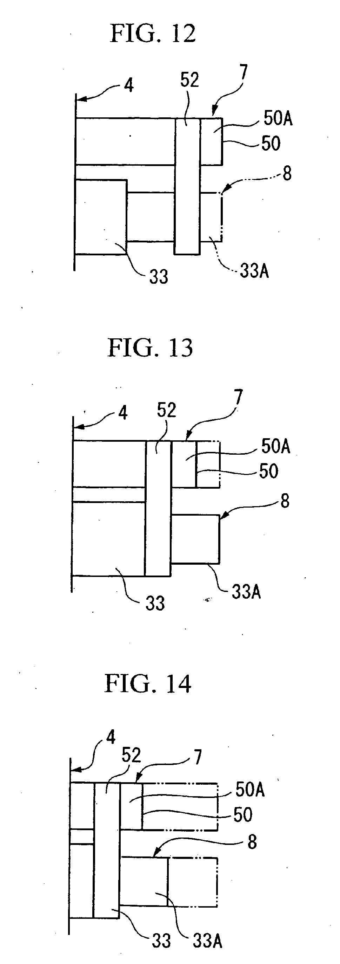

A connecting member 52 is a connecting mechanism fixed on a head section 50A of the button main unit 50. As illustrated in FIG. 10, the connecting member 52 of the suction button 7 has an opening section 53 having a head section 33A of the air-and-water-supplying button 8 inserted therethrough. The head section 33A is capable of freely extending and retracting.
The air-and-water-supplying button 8 can undertake a first press as illustrated in FIG. 12 as long as the buttons 7 and 8 are not pressed as illustrated in FIG. 11. The air-and-water-supplying button 8 cannot undertake a secondary press in this state, i.e., the head section 33A is substantially flush with the connecting member 52.

A gap is formed between a connecting member 52 and a step section formed by an increased-diameter section of the air-and-water-supplying button 8 as illustrated in FIG. 11 showing a non-pressed state of buttons 7 and 8. Accordingly the suction button 7 alone can undertake a first press as illustrated in FIG. 13, and this state of connecting member 52 makes contact with the step section of the air-and-water-supplying button 8.
Pressing the suction button 7 further, i.e., a secondary press to the suction button 7 provides a similar manner of secondary press to the air-and-water-supplying button 8 via the connecting member 52 as illustrated in FIG 14. It should be noted that the buttons 7 and 8 are configured to make stepwise movements by means of e.g., a spring to the positions as illustrated in FIGS. 3, 5, 6, and 7 to 9. These drawings do not show the spring.

As illustrated in FIGS. 1 and 2, the tissue-grasping device 17 has a cylindrical casing 61 fixed to a lateral section 4A of the endoscope-maneuvering section 4. The casing 61 has a lid 62 that blocks the opening of the casing 61. A tissue-suctioning pipeline 46 is connected to an opening section 61A of the lateral section of the casing 61. A tissue-suctioning pipeline 47 is connected to an opening section 61B of the bottom section of the casing 61.
An increased-diameter section, i.e., a flange 61C is formed on an outer periphery of the opening of the casing 61. A jaw section 62A provided to the lid 62 locks the lid 62 to the casing 61. A cylindrical lateral section 62B extends from the jaw section 62A along an inner periphery of the casing 61. The lateral section 62B extends to a position where the lateral section 62B does not interfere with the opening section 61 A of the casing 61. The lateral section 62B of the lid 62 is blocked by a lens 63. The lid 62 as a whole has a recessed shape in a cross-sectional view.
A seal member 64, e.g., an O-ring is inserted into a groove formed on an outer periphery of the lateral section 62B. The seal member 64 forms an airtight structure between the lid 62 and the casing 61. A filter 65 is inserted into a space formed between the lens 63 and the bottom section of the casing 61. Formed on the filter 65 is a tissue-grasping surface 65A that causes the opening section 61A to the opening section 6 1 B and captures a living tissue.

As illustrated in FIG. 2, the instrument 3 has a maneuvering section 71 that is maneuvered in the exterior of a patien's body by a surgeon. The maneuvering section 71 has an elongate maneuvering section main unit 72. A finger-hook ring 72A is formed on the proximal end of the maneuvering section main unit 72. A slit 73 is formed distally relative to the ring 72A. The slit 73 extends along an axial line direction of the maneuvering section main unit 72, and a slider 74 capable of freely extending and retracting is attached along the slit 73. A maneuvering wire 81 us fixed to the slider 74. The maneuvering wire 81 passing through the maneuvering section main unit 72 is extracted into an insertion section 75 provided on the distal end section of the maneuvering section main unit 72.

The insertion section 75 is configured so that the maneuvering wire 81 is capable of freely extending and retracting through an elastic and densely-wound long length of coil sheath 76. Provided to the distal end section of the insertion section 75 is a distal end-treating section 77 that is fixed to the distal end of the coil sheath 76.

The distal end-treating section 77 has a forceps distal end section 78 fixed to the coil sheath 76. A pair of autopsy cups 79 inserted into a slit formed on the distal end of the forceps distal end section 78 are capable of freely rotating and are supported by a pin 80. Recessed portions are formed on the distal end sections projecting from the forceps distal end section 78 of the autopsy cup 79. Also, the recessed sections of the autopsy cup 79 are disposed to direct to each other. s A maneuvering wire 81 is joined to a distal part of the autopsy cup 79 povotably supprted by the pin 80. In this configuration, retracting the slider 74 causes a pair of the autopsy cup 79 connected via the maneuvering wire 81 to close, and extending the slider 74 causes a pair of the autopsy cup 79 connected via the maneuvering wire 81 to open.

The width of the distal end-treating section 77 upon opening the autopsy cup 79 is less significant than an inner diameter of the forceps channel 15. That is, a space formed in the forceps channel 15 enabes to open or close the autopsy cup 79 between the opening of its distal end and a connecting point 45.

Operations in the present embodiment is explained next.
An endoscope insertion section 5 is inserted into a body of a patient, and an instrument 3 is inserted into a forceps channel 15. Buttons 7 and 8 are retracted most in the initial state, and air supplied from an air-supplying source 12A is dicharged from a leak hole 34 of the air-and-water-supplying button 8 to thereoutside. Air is suctioned from a communication hole 51 of the suction button 7 into a suction source 14.

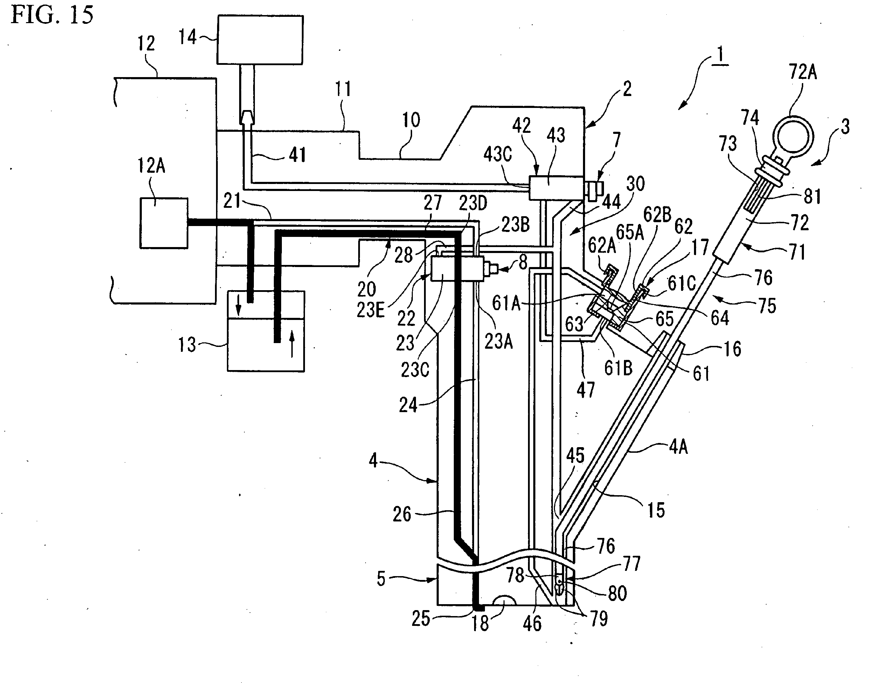

For example, as illustrated in FIGS. 5 and 12, incleansing a lens 18 of the endoscope insertion section 5, i.e., supplying water ordinarily from the distal end section of the endoscope insertion section 5 necessitates a first press to only the air-and-water-supplying button 8 to separate a second port 23B from ports 23A and 23C, thereby connecting a third port 23C to a fourth port 23D. Therefore, as illustrated in FIG. 15, the air supplied from the air-supplying source 12A and introduced to a water-supplying tank 13 presses down a liquid surface of the water-supplying tank 13. This results in supplying normal saline solution or distilled water from the water-supplying tank 13 to a water-supplying pipeline 27.
Communication between the fourth port 23D and the third port 23C causes liquid supplied from the water-supplying tank 13 through the water-supplying pipeline 26 and the nozzle 25 to be supplied to the lens 18, thereby cleansing the surface of the lens 18. Air does not flow to a air-supplying pipeline 24 since the first port 23A of the first switching device 22 does not communicate to the second port 23B. liquid is not supplied to a tissue water-supply pipeline 28 since a fifth port 23E does not communicate to the fourth port 23D.

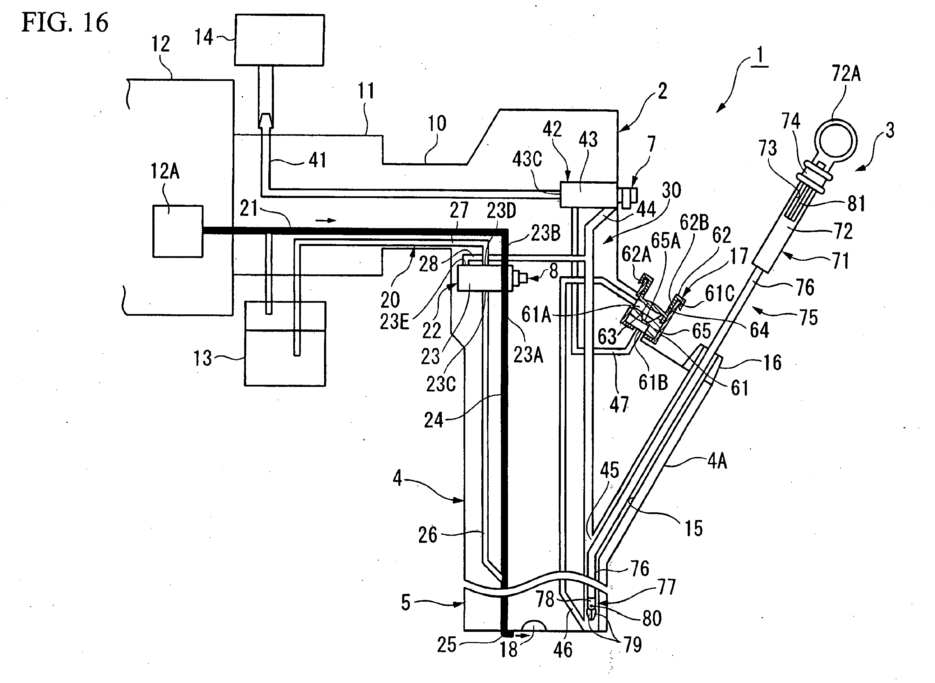

For example, as illustrated in FIGS. 4 and 11, blowing off moisture from the cleansed lens 18, i.e., ordinary air-supply from the distal end section of the endoscope insertion section 5 necessitates a finger to block the leak hole 34 while the air-and-water-supplying button 7 is in the most retracted state. Pressure of the air supplied from the second port 23B causes a check valve 35 to open, thereby communicating the second port 23B to the first port 23A.
On the other hand, liquid is not supplied to the third port 23C and the fourth port 23D since those ports are separated by a packing 36D. As illustrated in FIG. 16, this results in the air supplied from the air-supplying source 12A ans passing through the air-supplying pipeline 21, second port 23B, first port 23A, and air-supplying pipeline 24 is blew off from a nozzle 25.

As illustrated in FIGS. 8 and 13, ordinary suctioning operation, e.g., suctioning the once-supplied water necessitates a first press only to the suction button 7, thereby communicating the first port 43A of the second switching device 42 to the third port 43C. This resultsin suctioning conducted by means of the suction source 14, suction pipeline 41, third port 43C, first port 43A, suction pipeline 44, and the forceps channel 15.

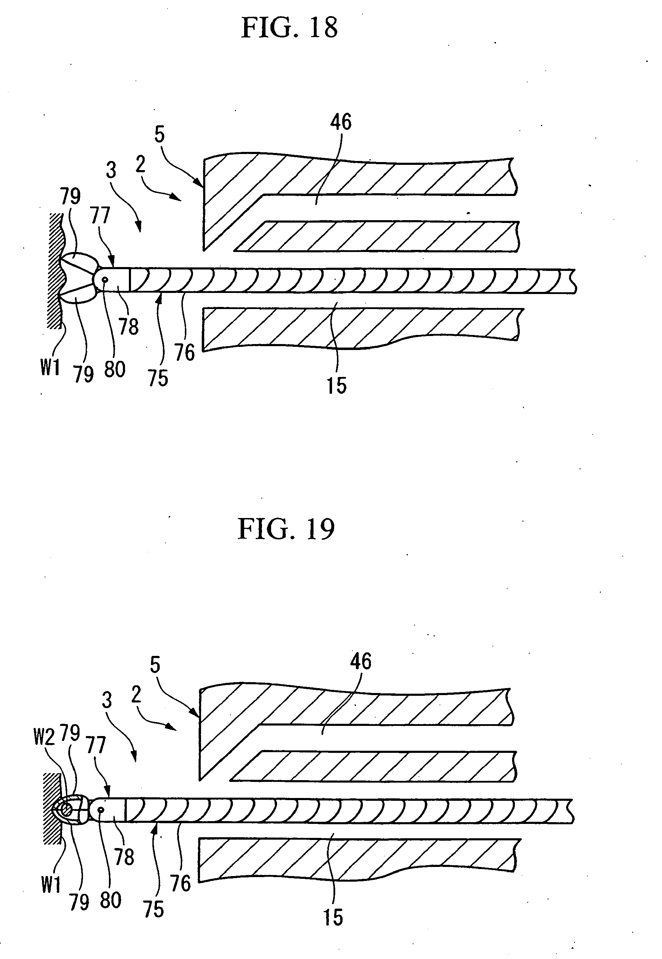

On the other hand, capturing a living tissue necessitates extending the whole instrument 3 and causing a distal end-treating section 77 to project from the distal end section of the endoscope 2. Further extending the slider 74 of the maneuvering section 71 causes a pair of the autopsy cups 79 connected to the maneuvering wire 81.
As illustrated in FIG. 18, the slider 74 of the maneuvering section 71 is retracted upon pressing this state of autopsy cups 79 onto an object position, e.g., a mucosa W1. A autopsy cups 79 connected to the maneuvering wire 81 closes. A part of the living tissue in this state of the mucosa W1 is put between the recessed sections of the autopsy cups 79 as illustrated in FIG. 19.

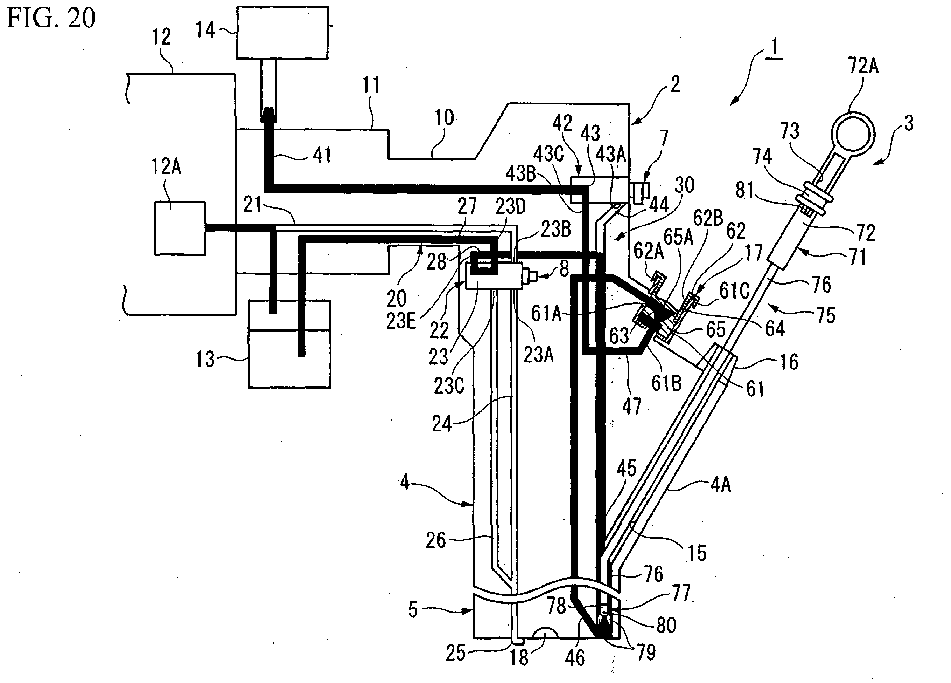

Retracting the whole instrument 3 while closing the autopsy cups 79 causes the living tissue grasped by the autopsy cups 79 to be torn from the mucosa W1, thereby obtaining a grasped tissue W2. A short distance from the mucosa W1 to the distal end section of the endoscope insertion section 5 causes the distal end-treating section 77 to be housed in the channel 15 of the endoscope 2 upon retracting the whole instrument 3.
Liquid-supply to the forceps channel 15 is commenced and suctioning from the tissue-suctioning pipeline 46 is commenced after positioning the distal end-treating section 77 distally relative to the proximal end of the tissue-suctioning pipeline 46 as illustrated in FIG. 20.

Details in liquid-supply to the forceps channel 15 and suctioning from the tissue-suctioning pipeline 46 are explained. A secondary press is provided to the suction button 7 as illustrated in FIG. 14. The air-and-water-supplying button 8 pressed by this state of the connecting member 52 also undergoes a secondary press. The second port 43B communicates to the third port 43C by switching the second switching device 42 in the second pipeline system 30. This results in the suction source 14 connected to the tissue-suctioning pipeline 47, thereby suctioniong the tissue-suctioning pipeline 46 via the tissue-grasping device 17.

On the other hand, the fourth port 23D communicates to the fifth port 23E by switching the first switching device 22 in the first pipeline system 20. This results in air supplied from the air-supplying source 12A to be introduced into the water-supplying tank 13, thus liquid therein is flown out. The liquid flowing out of the water-supplying tank 13 flows through the water-supplying pipeline 27, the fourth port 23D of the first switching device 22, and the fifth port 23E, and is introduced to a tissue water-supply pipeline 28. The liquid passing from the tissue water-supply pipeline 28 through a suction pipeline 44 further flows into the forceps channel 15. The liquid is suctioned from the tissue-suctioning pipeline 46 connected to the distal end section of the forceps channel 15.

Substantially all the liquid flowing into the forceps channel 15 is suctioned into the tissue-suctioning pipeline 46 since the endoscope 2 is adjusted so that suctioning by the suction source 14 exceeds liquid-supply from the water-supplying tank 13.
Preferable settings upon a secondary press to the suction button 7 may cause simultaneous suctioning and liquid-supply or suctioning in prior to liquid-supply. This facilitates suctioing and collecting of the liquid supplied to the forceps channel 15.

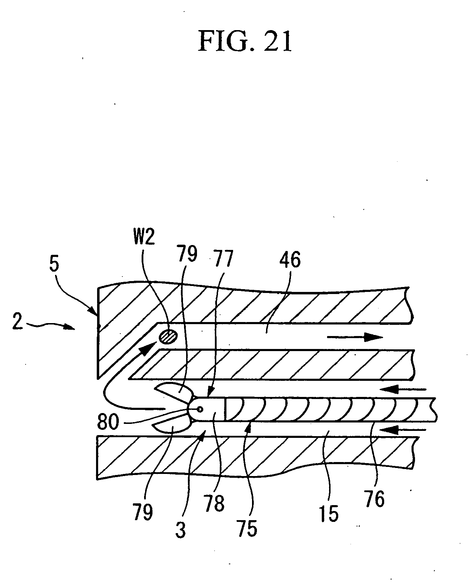

A pair of the autopsy cups 79 are opened by maneuvering the maneuvering section 71 of the instrument 3 in prior or subsequent to commencing liquid-supply and suctioning by using pipelines in the endoscope 2. The grasped tissue W2 as if it is washed by the liquid flowing through the forceps channel 15 separates from the autopsy cups 79, and as illustrated in FIG. 21, the grasped tissue W2 together with the liquid is suction from the tissue-suctioning pipeline 46 having an opening that is distally located relative to the autopsy cups 79. The grasped tissue W2 passing through the tissue-suctioning pipeline 46 are introduced into the tissue-grasping device 17, thereby captured by a tissue-grasping surface 65A of a filter 65.
The liquid passes through a filter 65, an opening section 6 1 B of a casing 61 of the tissue-grasping device 17, a tissue-suctioning pipeline 47, a second switching device 42, and a suction pipeline 41, and is drained from the suction source 14. An enlarged view of the grasped tissue W2 can be obtained by a lens 63 provided to a lid 62 of the tissue-grasping device 17. The grasped tissue W2 can be taken out by removing the lid 62. Serial biopsy is conducted by repeating the previously-explained operations while maintaining the liquid-supply and suctioning.

The present embodiment can simplify and downsize the instrument since the tissue water-supply pipeline and the tissue-suctioning pipeline are provided to an endoscope in contrast to a conventional configuration in which a tissue water-supply pipeline and a tissue-suctioning pipeline are provided to an instrument. In addition, the present embodiment can simplify and downsize the maneuvering section 71 of the instrument 3 by providing maneuvering-means such as a button or water-supplying button to the endoscope 2. This can reduce cost of the instrument 3.
The instrument 3 can be downsized and reduced in cost relative to a conventional case where a tissue-grasping device 17 is provided to an instrument since the tissue-grasping device 17 is provided to the endoscope 2. The whole configuration of the endoscope system 1 can be simplified and cost reduction can be achieved since the grasped tissue W2 can be captured by the endoscope 2 using: pipelines for use in cleansing the lens 18 and in an ordinary suctioning; buttons 7 and 8; and a part of the suction source 14. These factors can reduce cost per therapeutic maneuvering.

Workload to an assistant who maneuvers the instrument 3 can be reduced by assigning roles between a surgeon who maneuvers the encodscope 2 since operations associated with liquid-suupply and suctioning for captureing the grasped tissue W2 are conducted by an surgeon who maneuvers the endoscope 2.

### Second embodiment

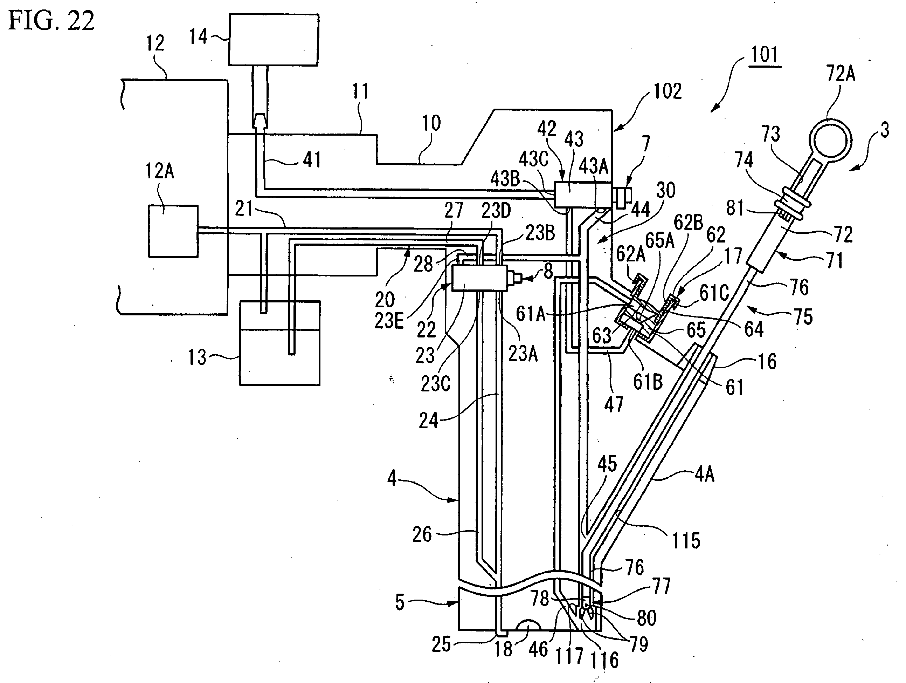

A second embodiment according to the present invention will be explained in detail with reference to FIGS. 22 to 28.
As illustrated in FIG. 22, an endoscope system 101 includes an endoscope 102 and an instrument 3 in configuration. A forceps channel 115 of the endoscope 102 according to the present embodiment is different from that of the first embodiment, and the rest of the components in the present embodiment is the same as those in the first embodiment.

Provided to the distal end section of the forceps channel 115 is a cylindrical chamber 116 having an enlarged diameter section. A tissue-suctioning pipeline 46 is connected to the chamber 116 in a slanting manner. The diameter of the chamber 116 is more significant than an open state of the autopsy cups 79 so that the autopsy cups 79 can be opened or closed there. The diameter of the forceps channel 115 except the chamber 116 is more significant than the outer diameter of the insertion section 75 and the outer diameter of the distal end-treating section 77 of the closed state of the autopsy cups 79, but less significant than the open state of the autopsy cups 79. Therefore, a gap formed by the chamber 116 is an abutment section 117 (regulating section) having a function that will be explained later.
The surface of the distal end of the abutment section 117 is an abutment surface 117A that is orthogonal to an axial line of the forceps channel 115. The distance between the abutment surface 117A and a position where the tissue-suctioning pipeline 46 is connected is more significant than the length of the autopsy cups 79.

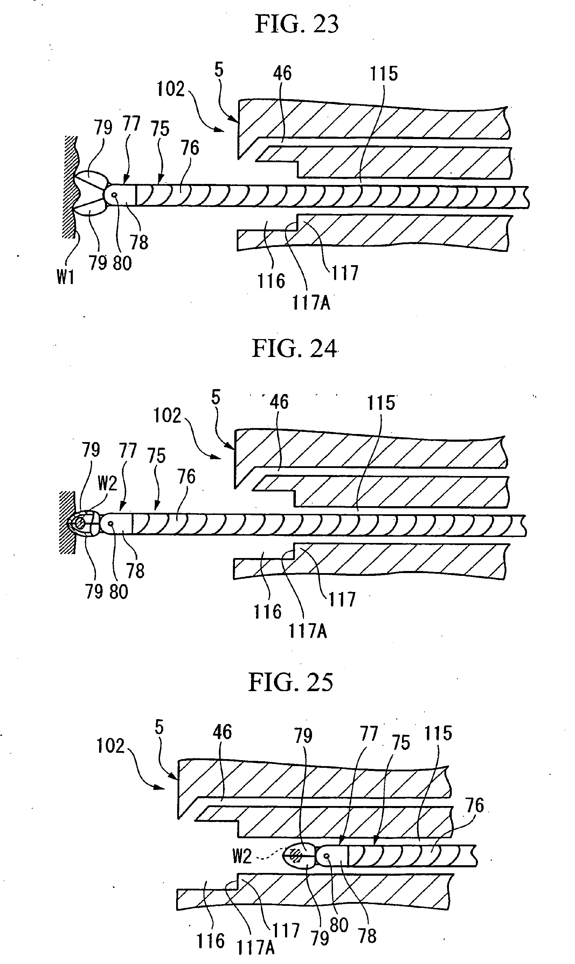

Operations of the endoscope system 101 are explained next.
As illustrated in FIG. 23, an opening state of the autopsy cups 79 of the distal end-treating section 77 projected from the distal end section of the endoscope 102 is pressed onto a mucosa W1, and then the autopsy cups 79 are closed by maneuvering the maneuvering section 71. As illustrated in FIG. 24, since the autopsy cups 79 grasp the living tissue (grasped tissue W2), the whole instrument 3 maintainig this state is retracted into the forceps channel 115 of the endoscope 102. As illustrated in FIG. 25, the grasped tissue W2 grasped by the autopsy cups 79 are dissected and the distal end-treating section 77 is housed in the endoscope 102.

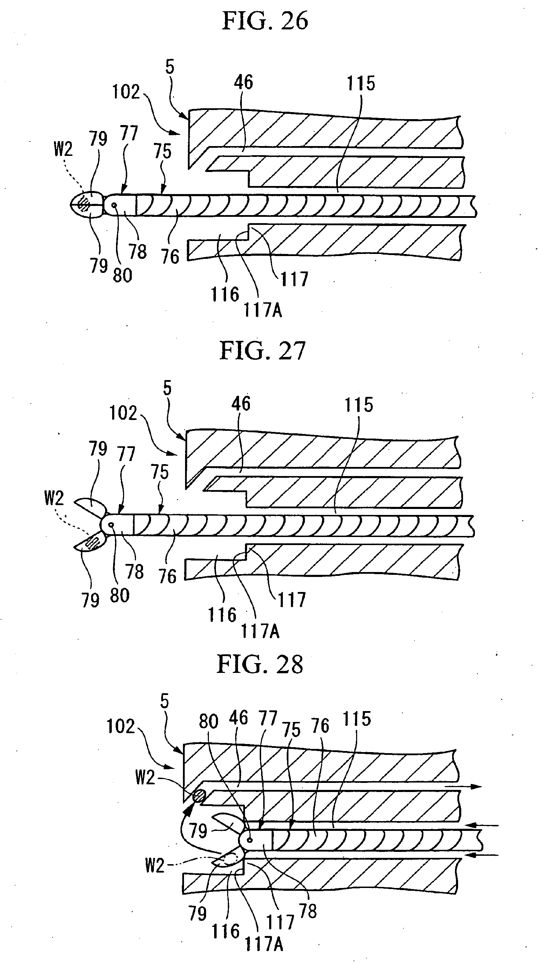

As illustrated in FIG. 26, subsequently extending the whole instrument 3 causes the distal end-treating section 77 to project from the distal end section of the endoscope 102. As illustrated in FIG. 27, subsequently manueuvering the maneuvering section 71 causes a pair of the autopsy cups 79 to open again. The state of grasped tissue W2 remains housed in the autopsy cups 79.

Retracting the whole instrument 3 having an open state of the autopsy cups 79 causes the autopsy cups 79 upon hitting a abutment surface 117A of the abutment section 117 to stop there since the autopsy cups 79 is open as illustrated in FIG. 28. The distal end of this state of distal end-treating section 77 is disposed at a distal position relative to the tissue-suctioning pipeline 46.

Similar maneuvering to the first embodiment, i.e., supplying liquid from the proximal end of the forceps channel 115 and suctioning through the tissue-suctioning pipeline 46 cause liquid introduced through the port 343B of the first pipeline system 20 to the forceps channel 115 to flow through a space between the forceps channel 115 and the instrument 3 into the chamber 116, thereby separating the grasped tissue W2 from the autopsy cups 79. The grasped tissue W2 and liquid are suctioned into a tissue-suctioning pipeline disposed distally relative to the autopsy cups 79, and captured by the tissue-grasping device 17. Serial biopsy repeats these operations.

The grasped tissue W2 can be collected by using pipelines formed in the endoscope 102 accordingn to the present embodiment since the chamber 116 provided ti the distal end section of the forceps channel 115 can accommodate the distal end-treating section 77 having an open state of the autopsy cups 79. Accordingly, the configuration in the instrument 3 is simplified; thus, cost reduction can be achieved. Also, the chamber 116 provided in the distal end section enables narrowing the rest of the part of the forceps channel 115; thus, the diameter of the endoscope insertion section 5 can be narrower than that of the first embodiment.
The distal end-treating section 77 ensured to be positioned proximally relative to the tissue-suctioning pipeline 46 allows the grasped tissue W2 to be collected desirably since the autopsy cups 79 making contact with the abutment surface 117A causes the distal end-treating section 77 to be positioned. Operational workload to a surgeon can be reduced since positioning can be achieved by merely abutting the distal end-treating section 77 to the abutment surface 117A. In addition, the grasped tissue W2 can be colledted while maintaining an inserted state of the instrument 3 without changing the configuration of the instrument 3.

The chamber 116 is not necessarily cylindrical, i.e., the chamber 116 may be rectangle, etc., in shape. A abutment section 117 can be formed in this case since the autopsy cups 79 is capable of opening and closing in the chamber 116. The abutment surface 117A may have a predetermined tilt angle relative to an axial line of the forceps channel 115 or may have a curved shape.

### (Third Embodiment)

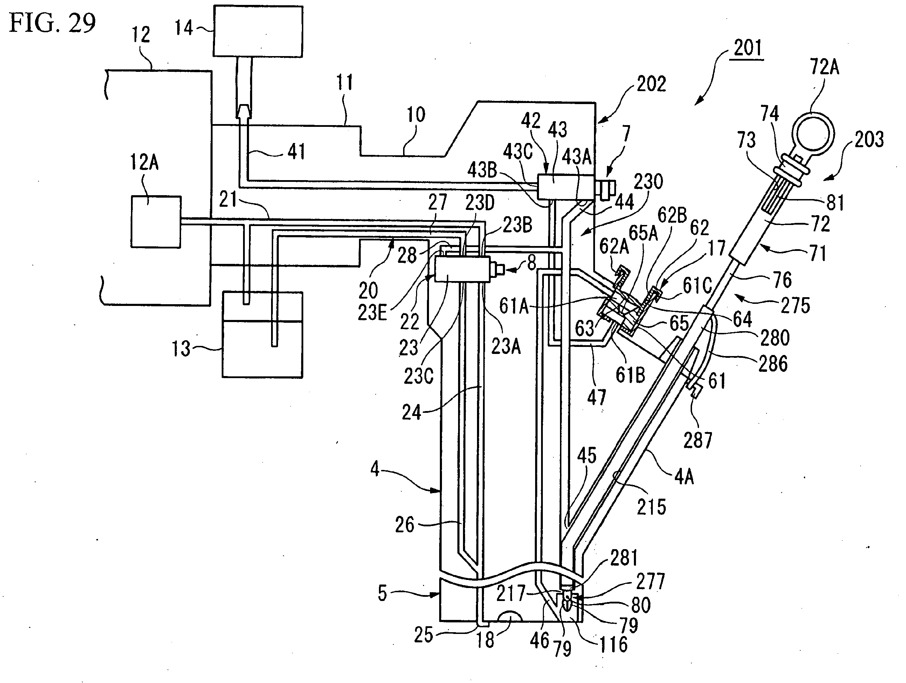

A third embodiment according to the present invention is explained as follows in detail with reference to FIGS. 29 to 36. Note that elements that are equivalent to those of the first embodiment will be assigned the same reference symbols and redundant explanations thereof will be omitted.

As illustrated in FIG. 29, an endoscope system 201 includes an endoscope 202 and an instrument 203 in configuration. The endoscope 202 has a first pipeline system 20 and a second pipeline system 230. THe second pipeline system 230 is provided with a forceps channel 215 having a regulating section, i.e., a abutment section 217 provided to the vicinity of the distal end of the forceps channel 215 so that the abutment section 217 has a ring shape and projects inward in a radial direction.
The surface on the proximal end of the abutment section 217 forms a abutment surface 217A that is substantially orthogonal to an axial line of the forceps channel 215. The portion distal relative to the abutment section 217 forms a chamber 116 having a tissue-suctioning pipeline 46 connected to the chamber 116 in a slanting manner. The diameter of the forceps channel 215 except the abutment section 217 is substantially the same until the connecting point 45 is reached.

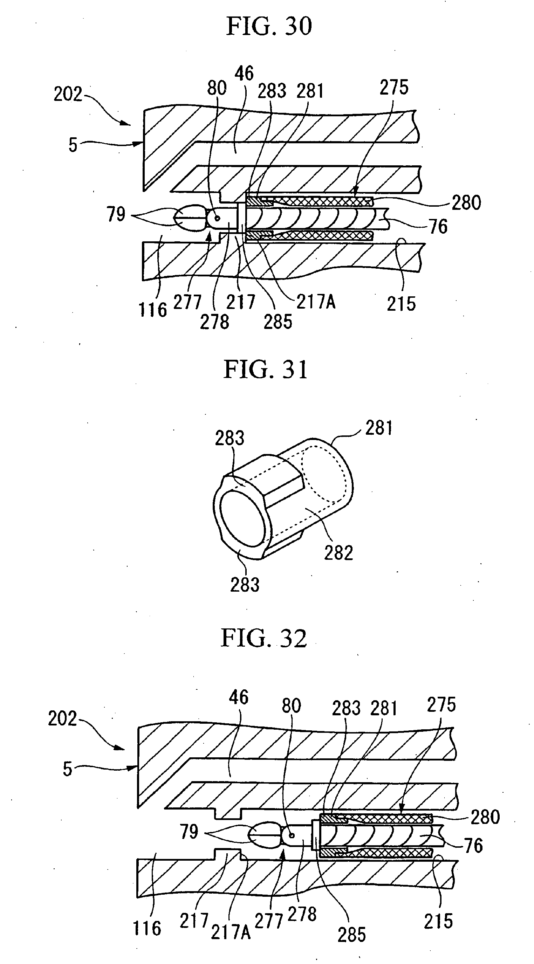

The insertion section 275 of the instrument 203 has an inner sheath that is a densely-wound coil sheath 76 as illustrated in FIGS. 29 and 30, and an outer sheath 280, covering the inner sheath and capable of freely sliding on the inner sheath. The outer sheath 280 operable as a distal-end-regulating section as explained later has a length longer than that of the forceps channel 215 but shorter than the coil sheath 76. Therefore, the proximal end section of the outer sheath 280 is exposed from the endoscope 202, and furthermore, the coil sheath 76 is extracted from the proximal end of the outer sheath 280. The outer sheath 280 has a tubular sheath main unit having a distal end chip 281 fixed to the distal end section sheath main unit using crimping method, etc.

As illustrated in FIG. 31, the distal end chip 281 has a cylindrical chip main unit 282. The inner diameter of the chip main unit 282 is more significant than the outer diameter of the coil sheath 76. The outer diameter of the chip main unit 282 is less significant than the inner diameter of the forceps channel 215 except the abutment section 217. Two projecting sections 283 projecting outward in a radial direction are provided to the distal end section of the chip main unit 282.
The projecting sections 283 are provided at an equal interval in a circumferential direction around the axial line of the distal end chip 281. The outer diameter of the insertion section 275 corresponding to the positions where the projecting section 283 are formed is more significant. The outer diameter of the projecting section 283 including the distal end chip 281 is more significant than the inner diameter of the abutment section 217 of the forceps channel 215 disposed in the endoscope 202 but less significant than the inner diameter of the forceps channel 215 provided at a distal position relative to the abutment section 217.

An end section of a distal-end-regulating section, i.e., an engagement member 286 is fixed to the proximal end section of the outer sheath 280 expoased outside of the endoscope 202. The engagement member 286 is made of a looped elastic member, etc. The other end section of the engagement member 286 is capable of engaging with a hook section 287 that is a regulating section provided to the vicinity of the lateral section 4A of the endoscope-maneuvering section 4 and to the vicinity of the forceps plug 16.

The hook section 287 assumues a downward hook in chape, i.e., slanting with respect to a vertical line, more specifically, the hook section 287 is slanting relative to the axial line of the forceps channel 215 and is directed to the distal end of the endoscope insertion section 5. Extending the other end section of the engagement member 286 produced from an elastic member and engaging the end to the hook section 287 cause the engagement member 286 to contract, thereby urging the outer sheath 280 toward the abutment section 217. The engagement member 286 and the hook section 287 are disposed so that the outer sheath 280 is continuously abutted to the abutment section 217.

In addition, a distal end-treating section 277 is provided to the distal end of the insertion section 275 of the instrument 203. The distal end-treating section 277 is provided with a pair of autopsy cups 79 capable of opening or closing at the forceps distal end section 278; and an increased-diameter section 285 having an increased diameter at the proximal end section of the forceps distal end section 278. The outer diameter of the increased-diameter section 285 is more significant than the inner diameter of the distal end chip 281 and less significant than the inner diameter of the abutment section 217 of the endoscope 202. It should be noted that the forceps distal end section 278 itself may have the outer diameter the same as that of the increased-diameter section 285.

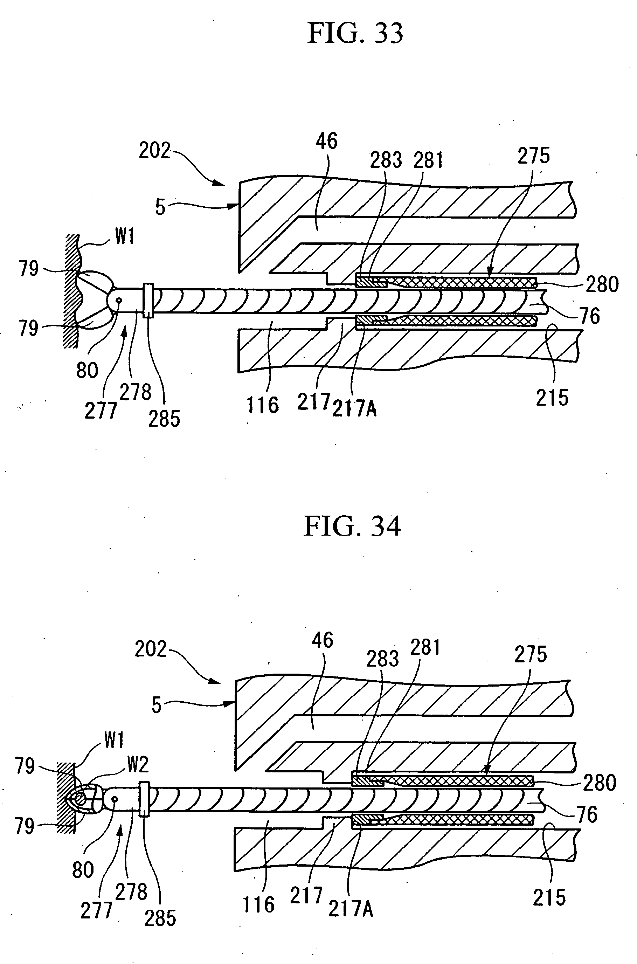

Operations in the present embodiment is explained next.
An instrument 203 is inserted into a forceps channel 215 of the endoscope 202 as illustrated in FIG. 32. The insertion of the instrument 203 causes the coil sheath 76 and the outer sheath 280 to be inserted together Since friction force hardly allows a maneuvering, e.g., an insertion or a retraction to move positions between the outer sheath 280 and the coil sheath 76 from each other
As illustrated in FIG. 30, the projecting section 283 of the distal end chip 281 makes contact with the abutment surface 217A although the distal end-treating section 277 of the distal end passes through the abutment section 217. Engaging this state of the engagement member 286 provided at the proximal end of the outer sheath 280 to the hook section 287 subsequently causes the outer sheath 280 to be fixed on the abutment surface 217A in the abutted state. Therefore, extending the coil sheath 76 does not cause the outer sheath 280 to move as illustrated in FIG. 33 but causes the coil sheath 76 to extend, thereby allowing the distal end-treating section 277 to project from the distal end section of the endoscope 202.

Maneuvering the maneuvering section 71 upon pressing a pair of opening state of autopsy cups 79 to the mucosa W1 to close the autopsy cups 79 causes the crank member 734 to be grasped. Subsequently gripping the coil sheath 76 and drawing to retract the coil sheath 76 from the endoscope 202 causes the distal end-treating section 277 retracted into the forceps channel 215, thereby tearing the living tissue grasped by the autopsy cups 79 from the mucosa W1, thus obtaining the grasped tissue W2.
The abutting state of the outer sheath 280 onto the abutment section 217 is maintained since the outer sheath 280 is urged by the engagement member 286 as illustrated in FIG. 30. Therefore, the distal end-treating section 277 is retracted until the increased-diameter section 285 makes contact with the distal end chip 281. The distal end of the autopsy cups 79 upon a contact made between the increased-diameter section 285 and the distal end chip 281 is disposed proximally relative to a connecting point of the tissue-suctioning pipeline 46 in the chamber 116.

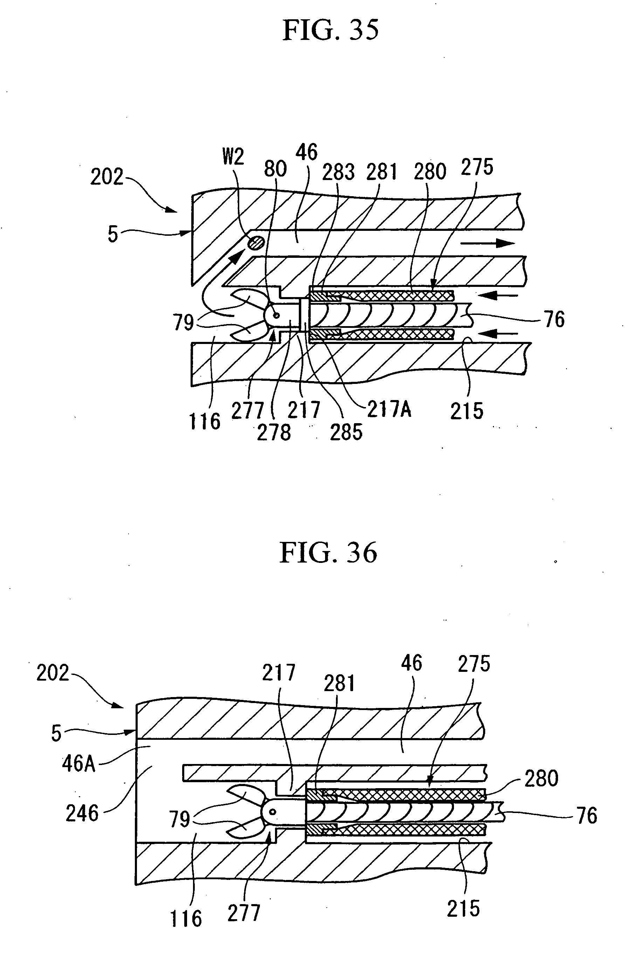

Water-supply and suctioning conducted similarly to the first embodiment cause the liquid passing from the first pipeline system 20 through the tissue water-supply pipeline 28 into the forceps channel 215 to flow through a space amoung an outer periphery of the chip main unit 282 of the distal end chip 281, the projecting section 283, and the abutment section 217 into the chamber 116, thereby suctioned to the tissue-suctioning pipeline 46 and drained. Therefore, opening a pair of the autopsy cups 79 and supplying liquid as illustrated in FIG. 35 cause the grasped tissue W2 to separate from the autopsy cups 79 and cause the separated tissue W2 to be introduced from the tissue-suctioning pipeline 46 to the tissue-grasping device 17 with the liquid, thereby captured by the filter 65. Serial biopsy repeats these operations.

In the present embodiment, the outer sheath 280 is provided to the instrument 203, and the distal end chip 281 making contact with the abutment section 217 is fixed to the distal end section of the outer sheath 280 while the abutment section 217 is provided to the forceps channel 215. Accordingly, causing the distal end chip 281 to make contact with the abutment section 217 and causing this state of the engagement member 286 provided on the proximal end of the outer sheath 280 to engage with the hook section 287 fix the position of the outer sheath 280, thereby positioning the distal end-treating section 277 in a retracting direction with reference to the position of the outer sheath 280.
Therefore, mere drawing of the coil sheath 76 allows capturing of the grasped tissue W2 and positioning of the distal end-treating section 277, thereby improving operability. The distal end-treating section 277 thus positioned facilitates and ensures collecting of the grasped tissue W2 that has undergone liquid-supply and suctioning.

A space significant for liquid-supply formed between the abutment section 217 and the distal end chip 281 allows the grasped tissue W2 to be collected reliably by using the liquid passing the outside of the instrument 203. Therefore, the structure of the instrument 203 can be simplified since no conventional pipelines need to be formed in the instrument 203. In addition, a retracted state of the coil sheath 76 can achieve reliable positioning of the distal end-treating section 277 since engaging the engagement member 286 attached to the proximal end of the outer sheath 280 to the hook section 287 is configured to allow the outer sheath 280 to be urged toward the abutment section 217.

The outer sheath 280 may be configured so that a lever provided in place fo the engagement member 286 is abutted to the abutment section 217 by hooking the lever to the lateral section 4A of the endoscope-maneuvering section 4.
The chamber 116 may be connected to the tissue-suctioning pipeline 46 through a communication path 246 in the endoscope 202 by disposing the chamber 116 of the forceps channel 215 in parallel with the tissue-suctioning pipeline 46 as illustrated in FIG. 36; disposing the opening of the distal end section 46A of the tissue-suctioning pipeline 46 onto the distal end section of the endoscope insertion section 5; and removing a wall section between the chamber 116 and the tissue-suctioning pipeline 46 to form the communication path 246. Functions and effects similar to the previously explained embodiments can be obtained since the liquid passing through the forceps channel 215 is absorbed through the communication path 246 into the tissue-suctioning pipeline 46.
A notch or a hole may be formed on the abutment section 217 to supply liquid to the chamber 216.

### (Fourth Embodiment)

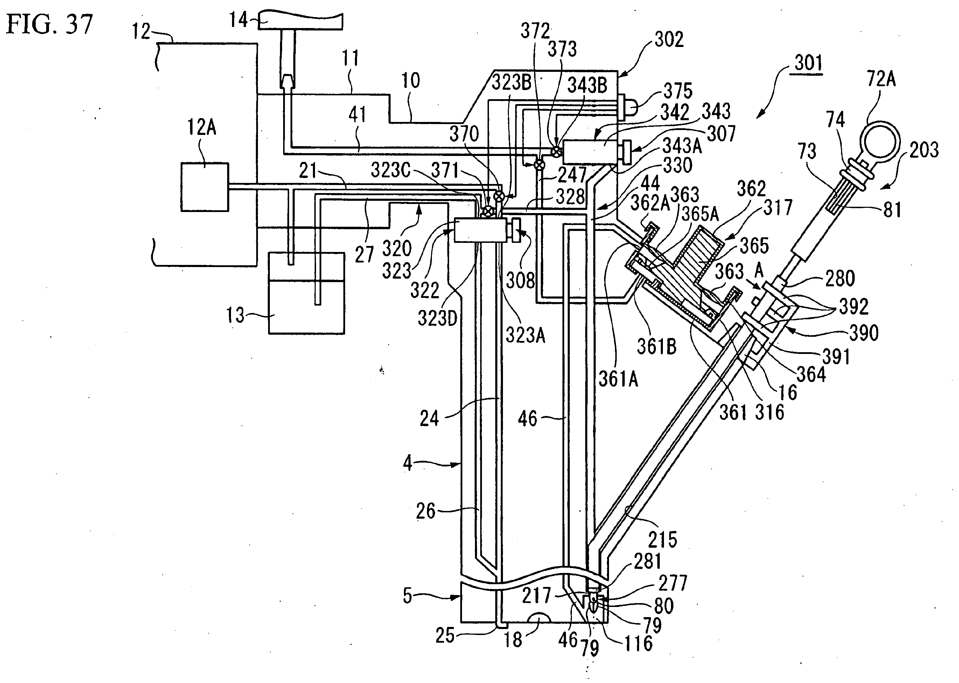

A fourth embodiment according to the present invention will be explained in detail with reference to FIGS. 37 to 44. Note that elements that are equivalent to those of the first embodiment will be assigned the same reference symbols and redundant explanations thereof will be omitted.

As illustrated in FIG. 37, an endoscope system 301 includes an endoscope 302 and an instrument 203 in configuration. The endoscope 302 has a first pipeline system 320 and a second pipeline system 330. The first pipeline system 320 has a air-supplying pipeline 21 connected to a air-supplying source 12A. The air-supplying pipeline 21 branching in a midway, and one of the branching parts is connected to a water-supplying tank 13, and the other to a second port 323B of the first switching device 322. A flow-passage-opening-and-closing section, i.e., a check valve 370 is provided between the branching point and the first switching device 322.

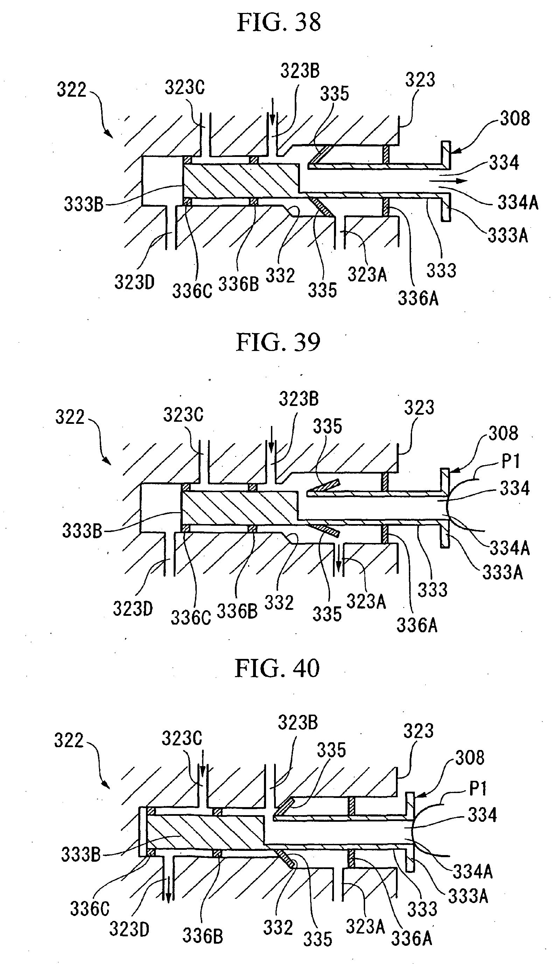

As illustrated in FIG. 38, the first switching device 322 switches flow paths and has a sleeve 323 having a air-and-water-supplying button 308 inserted therethrough. Four ports 323A to 323D are provided in order from the opening of the sleeve 323 in an axial line direction. A taper surface 332 opening toward the opening is formed between the first port 323A and the second port 323B. The air-and-water-supplying button 308 has a button main unit 333 having an exhaust hole 334 formed therein.
A leak hole 334A is an opening of the exhaust hole 334 formed on a head section 333A of a sleeve 323 where the button main unit 333 is exposed therefrom. The exhaust hole 334 further extends in the axial line direction and opens at a lateral section prior to reaching the distal end section 333B inserted into the sleeve 323. A check valve 335 is provided on an outer periphery that is distal relative to the opening. A packing 336A is fixed between the check valve 335 and the head-section 333A. Furthermore, two packings 336B and 336C are fixed at a predetermined interval distally relative to the check valve 335. These packings 336A to 336C form an airtight structure between the air-and-water-supplying button 308 and the sleeve 323.

The packing 336A is disposed between the first port 323A and the opening of the sleeve 323, and the check valve 335 is disposed between the first port 323A and the second port 323B as illustrated in FIG. 38. The packing 336B is disposed between the second port 323B and the third port 323C. The packing 336C is disposed between the third port 323C and the fourth port 323D. As illustrated in FIG. 39, blocking the leak hole 334A by a finger P1 and supplying air to the second port 323B cause the check valve 335 to open, thereby allowing the first port 323A to communicate to the second port 323B.

As illustrated in FIG. 40, presing the air-and-water-supplying button 308 until the check valve 335 makes contact with the taper surface 332 of the sleeve 323 decreases a pressure-receiving area of the check valve 335; therefore, the check valve 335 does not open if the leak hole 334A is blocked by a finger; thus, the first port 323A does not communicate wo the second port 323B. In this state, the packing 336A remains close to the opening relatie to the first port 323A; the packing 336B remains between the second port 323B and the third port 323C; and the packing 336C moves close to the blocked end of the sleeve 323 relative to the fourth port 323D. Therefore, this state of the third port 323C alone communicates to the fourth port 323D.

As illustrated in FIG. 37, an air-supplying pipeline 24 is connected to the first port 323A. A water-supplying pipeline 26 connected to the distal end section of the air-supplying pipeline 24 is further connected to the fourth port 323D. A water-supplying pipeline 27 is connected to the third port 323C. The water-supplying pipeline 27 is connected to the water-supplying tank 13. A tissue water-supply pipeline 328 branches off from a part of the water-supplying pipeline 27. The tissue water-supply pipeline 328 is connected to the second pipeline system 330. A flow-passage-opening-and-closing section, i.e., check valve 371 is provided in a middle of the tissue water-supply pipeline 328.

The second pipeline system 330 has a suction pipeline 41 connected to the suction source 14. The suction pipeline 41 subsequent to branching of the tissue-suctioning pipeline 247 is connected to the second port 343B of the second switching device 342. The tissue-suctioning pipeline 247 is connected to an opening section 361B of the casing 361 of the tissue-grasping device 317. In addition, a flow-passage-opening-and-closing section, i.e., check valve 372 is provided in a middle of the tissue-suctioning pipeline 247.
A flow-passage-opening-and-closing section, i.e., check valve 373 is provided to the suction pipeline 41 close to the second switching device 342 relative to a branching point of the tissue-suctioning pipeline 247. The check valves 372 and 373, and the check valves 370 and 371 of the first pipeline system 320 are electrically connected to a tissue-collecting suctioning-and-water-supplying switch 375 provided to the endoscope-maneuvering section 4. Turning off the suctioning-and-water-supplying switch 375 causes two check valves 370 and 373 to open and causes the check valves 371 and 372 alone to close. Turning of the suctioning-and-water-supplying switch 375 causes the check valve 370 and the check valve 373 to close and causes the check valve 371 and the check valve 372 to open.

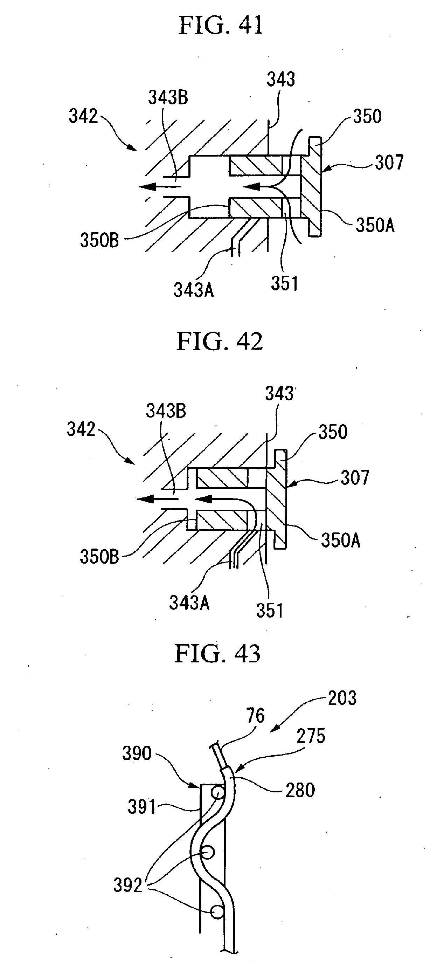

As illustrated in FIGS. 41 and 42, the second switching device 342 switches flow paths and has a sleeve 343 that allows a suction button 307 to be inserted therethrough. A first port 343A is provided to a lateral section of the sleeve 343. A second port 343B is provided to a blocked end section of the sleeve 343. The suction button 307 has a button main unit 350 that is substantially equal to an inner diameter of the sleeve 343. Provided to the button main unit 350 is a communication hole 351 that passes from the lateral section of the head section 350A to a distal end section 350B.

As illustrated in FIG. 41, an opening of the communication hole 351 close to the head section 350A relative to the retracted position of the suction button 307 is exposed outward, and the second port 343B opens outward. In contrast, as illustrated in FIG. 42, a pressed state of the suction button 307 causes the opening of the communication hole 351 close to the head section 350A to communicate to the first port 343A, and causes the first port 343A to communicate to the second port 343B.

As illustrated in FIG. 37, connected to the first port 343A is a suction pipeline 44 having a forceps channel 215 connected thereto. Formed on the distal end section of the forceps channel 215 is a chamber 116 having the distal end section of the tissue-suctioning pipeline 46 connected thereto in a slanting manner. The tissue-suctioning pipeline 46 is connected to the opening section having an opening on a lateral section of the casing 361 of the tissue-grasping device 317.

The tissue-grasping device 317 has bottomed cylinder casing 361 having an opening section 361A formed on a lateral section thereof. Formed on a bottom section of the casing 361 is an opening section 361B. A tissue-suctioning pipeline 46 is connected to the opening section 361 A, and a tissue-suctioning pipeline 247 is connected to the opening section 361B. A filter 365 capable of rotating around an axial line is inserted into the casing 361.
The central portion of the filter 365 projects along the axial line direction, and a plurality of tissue-grasping surfaces 365A surrounding the central portion are disposed in a circumferential direction at an equal interval. In addition, a lid 362 fixed to the casing 361 is inserted into the filter 365 while corresponding to the central portion. Provided to the lid 362 is a projecting section covering the central portion of the filter 365, and a plurality of lenses 363 surrounding the projecting section are disposed in a circumferential direction at an equal interval.
The lenses 363 provided to correspond to the tissue-grasping surface 365A of the filter 365 enable an enlarged view of observation to the grasped tissue W2 captured on the tissue-grasping surface 365A. It should be noted that an airtight structure is formed by the lid 362, an inner periphery of the casing 361, and a seal member 364, i.e., an O-ring, and that a jaw 362A engages the lid 362 to a flange of the casing 361. The lid 362 and the filter 365 rotate together, i.e., without a correlational shift since a fitting force between the lid 362 and the central portion of the filter 365 is more significant than the rotational force for rotating the lid 362.

An engagement member 390 is attached to a forceps plug 16 of the endoscope 302. The engagement member 390 has an engaging main unit 391 that extends in parallel with an axial line of the forceps channel 215. Three jaw section members 392 orthogonal to the axial line direction in side view extend from the engaging main unit 391 beyond the axial line of the forceps channel 215.
As illustrated in FIG. 43, the jaw section members 392, each having a cylindrical shape, are disposed offset from the longitudinal center line of the engaging main unit 391. Accordingly, passing the outer sheath 280 of the insertion section 275 of the instrument 203 through each jaw section member 392 causes the outer sheath 280 bending in a waveform to be fixed to the engagement member 390 by a friction force. It should be noted that each jaw section member 392 is not limited to a cylindrical shape and may be arbitrary in shape.

Operations in the present embodiment are explained as follows.
Inserting the instrument 203 into the endoscope 302 necessitates inserting of the insertion section 275 until the distal end chip 281 of the outer sheath 280 abuts an abutment section 217 of the endoscope 302. The distal end chip 281 upon abutting the abutment section 217 causes the outer sheath 280 to be positioned, thereby fixing this position of the outer sheath 280 by a friction force through the jaw section members 392. This prevents a position change of the outer sheath 280 due to the extending or retracting of the coil sheath 76.
The suctioning-and-water-supplying switch 375 is turned off to conduct an ordinary liquid-supply from the distal end of the endoscope insertion section 5. The air-and-water-supplying button 308 is further pressed as illustrated in FIG. 40. Air supplied from the air-supplying pipeline 21 to the water-supplying tank 13 causes liquid in the water-supplying tank 13 to flow into the water-supplying pipeline 27, followed by the third port 323C of the first switching device 322, the fourth port 323D, and the water-supplying pipeline 26, and to be ejected from the nozzle 25.

The suctioning-and-water-supplying switch 375 is turned off to conduct an ordinary air-supply from the distal end section of the endoscope insertion section 5. In addition, as illustrated in FIG. 39, the leak hole 334A of the retracted state of the air-and-water-supplying button 308 is blocked by a finger. Air flowing from the air-supplying source 12A through the air-supplying pipeline 21 into the second port 323B of the first switching device 322 opens the check valve 335 and further passes through the first port 323A and the air-supplying pipeline 24, thereby ejecting from the nozzle 25.

The suctioning-and-water-supplying switch 375 is turned off to conduct a suctioning alone. The suction button 307 is further pressed as illustrated in FIG. 42. Suctioningis conducted from the forceps channel 215 through the suction pipeline 44, second switching device 342, and the suction pipeline 41 since the first port 343A of the second switching device 342 is connected to the second port 343B.

Collecting the grasped tissue W2 in the maneuvering wire 81 necessitates tearing a part of the mucosa W1 by the autopsy cups 79, opening the autopsy cups 79 in the chamber 116, and conducting a suctioning through the tissue-suctioning pipeline 46 while supplying liquid from the forceps channel 215 similarly to the third embodiment. Turning on the suctioning-and-water-supplying switch 375 of this state of endoscope 302 causes the check valve 371 and the check valve 372 to open, and the check valve 370 and the check valve 373 to close. As illustrated in FIGS. 38 to 41, the buttons 307 and 308 each remains at the retracted position.

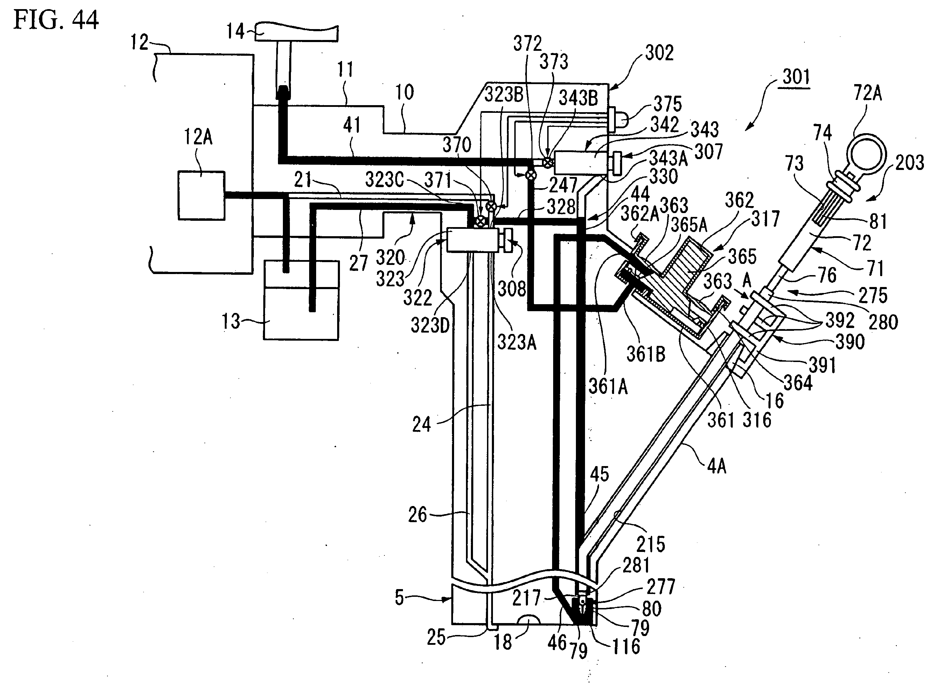

Accordingly, as illustrated in FIG. 44, the closing state of the check valve 370 provides air-supply from the air-supplying source 12A to the water-supplying tank 13, thereby commencing liquid-supply from the water-supplying tank 13 to the water-supplying pipeline 27. Liquid flows from the tissue water-supply pipeline 328 having an opening state of check valve 371 to the suction pipeline 44 since the third port 323C of the first switching device 322 is not connected other ports.
The closed state of the second switching device 342 of the suction pipeline 44 cause the liquid to flow into the forceps channel 215, thereby separating the grasped tissue W2 from the autopsy cups 79 in the chamber 116. The grasped tissue W2, suctioned with the liquid into the tissue-suctioning pipeline 46 and further introduced into the tissue-grasping device 317, is captured by the tissue-grasping surface 365A of the filter 365 disposed in the vicinity of the opening section 361A. The liquid passing through the tissue-grasping surface 365A is suctioned from the opening section 361B of the casing 361 into the tissue-suctioning pipeline 247 followed by the check valve 372, thereby drained from the suction pipeline 41.

Gripping and retracting the coil sheath 76 to tear the grasped tissue W2 from the mucosa W1 do not cause this state of outer sheath 280 to move since the outer sheath 280 is fixed to the endoscope 302 via the engagement member 390. Accordingly, the distal end of the autopsy cups 79 is automatically fixed at a predetermined position in the chamber 116 since the distal end chip 281 of the outer sheath 280 remains to abut onto the abutment section 217 of the forceps channel 215.
In a serial biopsy that repeats the above operations, the tissue-grasping device 317 having a plurality of tissue-grasping surfaces 365A rotates the filter 365 and the lid 362 every time the grasped tissue W2 is connected; therefore, a new tissue-grasping surface 365A is disposed toward the opening section 361A.

The present embodiment can achive a simple configuration of the instrument 203 since flow paths for conducting liquid-supply and suctioning to collect the grasped tissue W2 are provided in the endoscope 302. The tissue water-supply pipeline 328 that allows the first pipeline system 320 to communicate to the second pipeline system 330 and a configuration for switching a flow path by a plurality of check valves 370 to 373 and the suctioning-and-water-supplying switch 375 enables collecting of the grasped tissue W2 by switching, i.e., turning on or off the suctioning-and-water-supplying switch 375 based on a conventional endoscope based on conventional air-supply mechanism, liquid-supply function, and suctioning mechanism, thereby providing improved operability and effective serial biopsy.
In particular, a mere pressing of the suctioning-and-water-supplying switch 375 enables suctioning simultaneously with liquid-supply, or prior to liquid-supply. A single operation covering liquid-supply and suctioning provides improved operability and reduces workload to a surgeon.

The tissue-grasping device 317 provided to the endoscope 302 enables downsizing of the instrument 203 and reducing cost.
The position of the autopsy cups 79 in the chamber 116 can be fixed easily and reliably with a referenct to the position of the distal end of the outer sheath 280, and operability can be improved since the outer sheath 280 of the instrument 203 fixed by the engagement member 390 having three jaw section members 392 do not move by retracting the coil sheath 76 to collect the grasped tissue W2.
A component different from the third embodiment, i.e., the engagement member 390 is provided in the endoscope 302 can achieve more cost reduction for the instrument 203.

### (Fifth Embodiment)

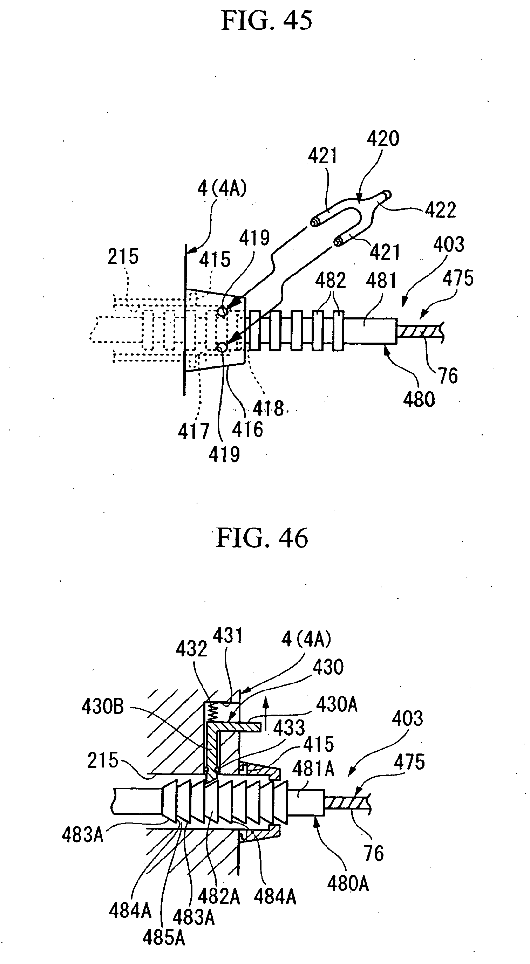

A fifth embodiment according to the present invention is explained as follows in detail with reference to FIGS. 45 to 55. The present embodiment has a feature in configuration where an outer sheath of an instrument is positioned and engaged to an endoscope. The other configuratios are similar to the third embodiment or the fourth embodiment.

As illustrated in FIG. 45, provided to a lateral section 4A of an endoscope-maneuvering section 4 is a forceps port 415 of a forceps channel 215. An outer periphery of the forceps port 415 is increased in diameter to form a flange. The forceps plug 416 that engages with a forceps port 415 of this type is an elastic truncated cone member that reduces in diameter from a bottom section making close contact with the endoscope-maneuvering section 4 toward in an axial line direction of the forceps channel 215. Formed in the forceps plug 416 is a insertion hole 417 that allows an insertion section 475 of the instrument 403 to be inserted therethrough. A diameter of the insertion hole 417 is substantially the same as the inner diameter of the forceps channel 215. A part cooresponding to the distal end section of the forceps plug 416 is reduced in diameter to form a reduced-diameter section 418.

Formed to the forceps plug 416 are two parallel key holes 419 that places an axial line of the insertion hole 417 therebetween. The key holes 419 each penetrating with the insertion hole 417 allows a key 420 to be inserted therethrough. The key 420 has a shape constituted by two parallel key insertion sections 421 forming a part of the U-letter shape and corresponding to the key holes 419; and a grasping section 422 extending from the U-letter shape to be grasped by a surgeon.

An outer sheath 480 capable of freely sliding on the outside of the coil sheath 76 is provided in the insertion section 475 of the instrument 403. The outer sheath 480 has an elastic tubular sheath main unit 481. A plurality of ring engagement sections 482 formed by increasing the outer peripheryies of the sheath main unit 481 are disposed at an equal interval in an axial line direction.
The outer diameter of the engagement section 482 is equal to or smaller than the inner diameter of the forceps channel 215, in addition, substantially the same as the diameter of the reduced-diameter section 418 of the insertion hole 417 of the forceps plug 416. An interval that disposes the engagement sections 482 in the axial line direction is substantially the same as the diameter of the key hole 419. The outer diameter of the sheath main unit 481 is substantially the same as a distance between two key holes 419. It should be noted that the engagement section 482 becomes a distal-end-regulating section; and the forceps plug 416 and the key 420 become a regulating section of the endoscope 302.

Operations in the present embodiment are explained as follows.
An instrument 403 is inserted into an endoscope 302 while the key holes 419 are removed from the forceps plug 416. The key 420 is inserted into the key holes 419 when the distal end chip 281 of the outer sheath 480 abuts the abutment section 217 of the distal end of the forceps channel 215. The key 420, inserted between the engagement sections 482 of the outer sheath 480, disposed to place the sheath main unit 481 therebetween in an axial line direction causes the outer sheath 480 to be fixed at this position.
Collecting of the grasped tissue W2 necessitates extending the coil sheath 76, grasping a living tissue of the mucosa W1 with the autopsy cups 79, and subsequently retracting the coil sheath 76 into the endoscope 302. The engagement between the key 420 and the engagement section 482 preventing a movement of this state of outer sheath 480 allows the coil sheath 76 alont to extend or retract, thereby causing the distal end chip 281 of the outer sheath 480 to make contact with and the abutment section 217 to be fixed there. Therefore, the distal end-treating section 277 retracted into the chamber 116 stops upon abutting the distal end chip 281.

The grasped tissue W2 is collected from an opening state of the autopsy cups 79 by suctioning from the tissue-suctioning pipeline 46 while conducting liquid-supply by using the forceps channel 215 similarly to the previous embodiments. Collecting all the necessary grasped tissue W2 and removing the instrument 403 necessitate removing of the key 420 from the key holes 419. Accordingly, the released engamement of the engagement section 482 allows the instrument 403 retracted with the outer sheath 480 to be removed from the endoscope.

The present embodiment allows the outer sheath 480 to be abutted to the abutment section 217 of the forceps channel 215 and fixed there since the key holes 419 that allow the key 420 to be inserted therethrough are formed on the forceps plug 416; and since the engagement section 482 capable of engaging with the key 420 is provided to the outer sheath 480. Therefore, the autopsy cups 79 can be positioned easily and reliably in the chamber 116 since retracting of the coil sheath 76 to collect the grasped tissue W2 does not cause movement of the outer sheath 480.
Therefore, complex adjustment of positioning the autopsy cups 79 can be overcome, and effective therapeutic maneuvering can be conducted. In addition, a plurality of engagement sections 482 disposed in an axial line direction facilitate adjusting of the insertion amount of the outer sheath 480, thereby allowing the outer sheath 480 to make contact with the abutment section 217 reliably.

A modified example of the present embodiment is explained as follows.
As illustrated in FIG. 46, provided to a lateral section 4A of an endoscope-maneuvering section 4 in the vicinity of the forceps port 415 of the forceps channel 215 is a housing section 431 for housing a ratchet 430 so that the ratchet 430 can freely slide in the housing section 431. An L-letter shape of the ratchet 430 is constituted by: a lever section 430A extending in parallel with an axial line direction of the forceps channel 215 so that a part of the lever section 430A is exposed outward; and a jaw section 430B extending in a directin orthogonal to the axial line of the forceps channel 215.
The jaw section 430B is urged by an elastic material 432, e.g., a coil spring so that the distal end of the jaw section 430B can project into the forceps channel 215. A part of the distal end of the jaw section 430B, directed to the forceps port 415 of the forceps channel 215, is cut in a slanting manner. A seal member 433, e.g., an O-ring is attached to surround an outer periphery of the jaw section 430B; thus, a liquid-tight structure is formed among the ratchet 430, the housing section 431, and the forceps channel 215.

The instrument 403 has an outer sheath 480A having an elastic tubular sheath main unit 481A. Formed on the proximal end section of the sheath main unit 481A is a predetermined length of tooth-shaped engagement section 482A disposed in an axial line direction. The engagement section 482A has a taper surface 483A where the diameter thereof reduces toward the distal end; and an engagement surface 484A that is orthogonal to an axial line. The engagement surface 484A and the taper surface 483A for a recessed section 485A that is capable of engage with the distal end section of the jaw section 430B of the ratchet 430.

Insertion of the instrument 403 causes an outer sheath 480A to be inserted while the taper surface 483A of the engagement section 482A pushes the ratchet 430 away. This state of the ratchet 430 overrides on the outer periphery of the taper surface 483A while contracting the hook section 287 having a restoring force that causes the ratchet 430 to enter a next recessed section 485A. Repeating these operations without manual maneuvering of the ratchet 430 cause further insertion of the instrument 403. Insertion is suspended upon a contact of the distal end chip 281 of the outer sheath 480A to the abutment section 217.
On the other hand, the engagement surface 484A abuts the jaw section 430B of the ratchet 430 in a retracting direction of the outer sheath 480A. An attempt to extract the outer sheath 480A will not be successful because the engagement surface 484A and the surface of the jaw section 430B abutting the engagement surface 484A are orthogonal to the axial line direction; and so the ratchet 430 engages with the engagement section 482A.

Therefore, positioning of the autopsy cups 79 during collecting of the grasped tissue W2 can be conducted easily and reliably because the distal end chip 281 continuously makes contact to the abutment section 217 if the coil sheath 76 is retracted while the living tissue is grasped by the autopsy cups 79. The instrument 403 is removed by drawing the lever section 430A in a direction indicated by an arrow shown in the drawing away from the instrument 403; releasing the engagement with the ratchet 430; and removing the insertion section 475.
Providing the engagement section 482A that serves as a distal-end-regulating section for the outer sheath 480A and the ratchet 430 that serves as a regulating section for the endoscope 202 enables swift maneuverging since an operation for fixing the outer sheath 480A is not necessary when the instrument 403 is inserted.

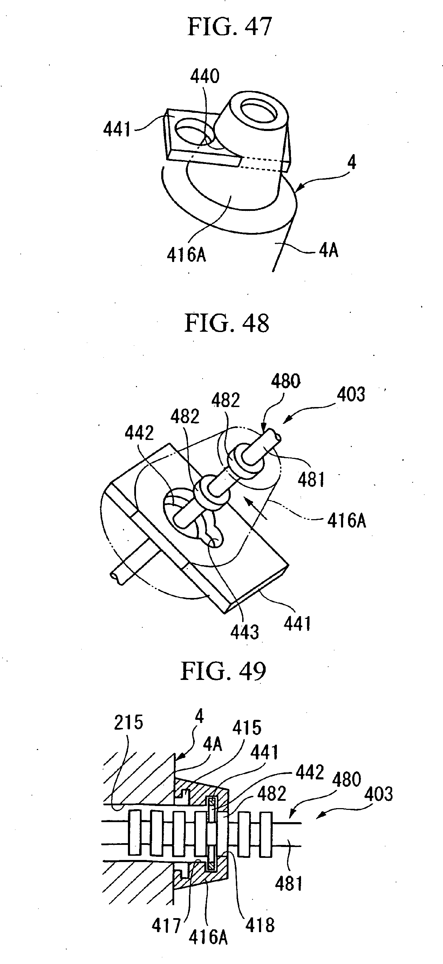

As illustrated in FIG. 47, a slit 440 orthogonal to the axial line direction may be formed on the forceps plug 416A, and a catching plate 441 capable of freely sliding may be press-fit in to the slit 440. As illustrated in FIG. 48, the catching plate 441 is formed to be elongate in shape so that an increased-diameter section 442 penetrating in the axial line direction of the forceps plug 416A partly overlap a reduced-diameter section 443 that is less significant in diameter than the increased-diameter section 442 in a longitudinal direction of the catching plate 441.
In contrast, projecting-engagement sections 482 disposed at an interval equvalent to or greater than the thickness of the cover 450 are provided on the outer sheath 480 of the instrument 403 in the axial line direction. The diameter of the engagement section 482 is greater than the diameter of the reduced-diameter section 443 but smaller than the diameter of the increased-diameter section 442. The outer diameter of the sheath main unit 481 is smaller than the diameter of the reduced-diameter section 443.

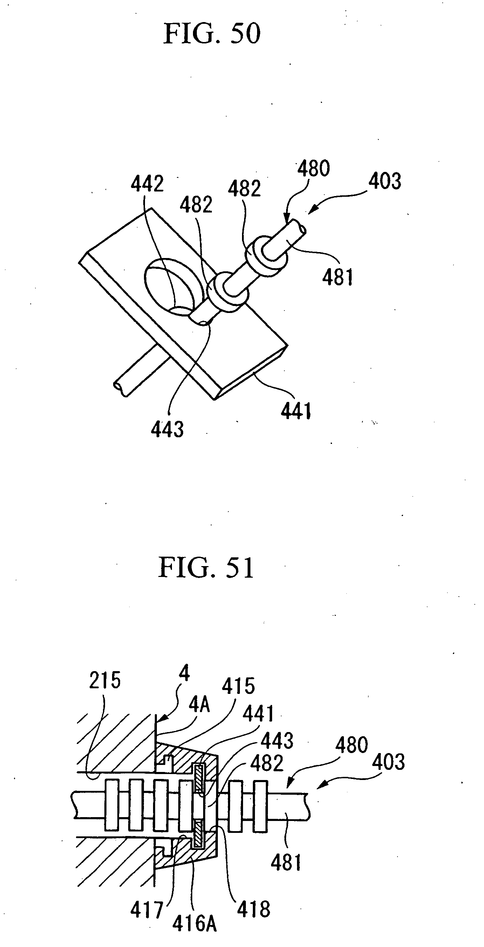

As illustrated in FIGS. 48 and 49, insertion of the instrument 403 necessitates coinciding the center of the increased-diameter section 442 to the center of the insertion hole 417 by sliding the catching plate 441. The outer sheath 480 having a greater diameter than that of the increased-diameter section 442 is unconditionally inserted therethrough. Subsequently, the catching plate 441 is slid after the distal end chip 281 of the outer sheath 480 abuts the abutment section 217; thus, the center of the reduced-diameter section 443 is coincided with the center of the insertion hole 417 as illustrated in FIGS. 50 and 51.
Movement of the outer sheath 480 is prevented since the engagement section 482 of the outer sheath 480 cannot pass through the reduced-diameter section 443. Removing of the instrument 403 from the endoscope necessitates sliding the catching plate 441 similarly; causing the increased-diameter section 442 to coincide with the insertion hole 417; and removing the instrument 403 together with the outer sheath 480.

Since the distal-end-regulating section, i.e., the engagement section 482 is provided to the outer sheath 480; and the regulating sections, i.e., the slit 440 and the catching plate 441 are provided to the forceps plug 416A of the endoscope 202, mere sliding of the catching plate 441 of this case can switch a fixed state and a movable state of the outer sheath 480, thereby facilitating the operation.

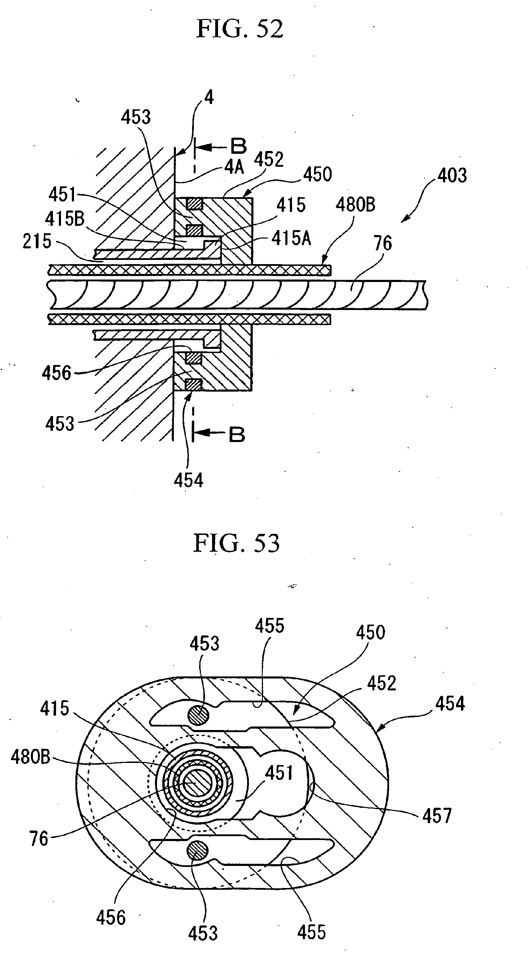

As illustrated in FIGS. 52 and 53, a cover 450 provided to the outer sheath 480B in place of the forceps plug 416 may be attached to the forceps port 415 of the forceps channel 215. The outer sheath 480B is fixed on the outer periphery of the outer sheath 480B, and a recessed section 451 capable of accommodating the flange section of the forceps port 415 therein is formed directed to the endoscope 302.
A ring lateral section 452 defined by a recessed section 451 has a partly notched section before reaching to the distal end surface making contact to the endoscope-maneuvering section 4 provides two supporting section 453 disposed in a ridial direction. In addition, catching plates 454 capable of freely sliding and having a supporting section 453 therebetween are inserted into the notched section.

The catching plate 454 having an oval shape has two elongated holes 455 that are formed to allow the supporting section 453 to pass therethrough. An increased-diameter section 456 and a reduced-diameter section 457 formed between the elongated holes 455 are connected in a longitudinal direction. The increased-diameter section 456 is through hole that allows the flange section, i.e., the outer periphery of the forceps port 415 to be inserted therethrough. The reduced-diameter section 457 is a through hole that is smaller than the diameter of the flange section 415A of the forceps port 415 allows the proximal end section 415B of the forceps port 415 that is smaller than the diameter of the flange section 415A. The elongated hole 455 benging inward in the vicinity of the center of the increased-diameter section 456 does not allow the catching plate 454 to move with an insignificant force.

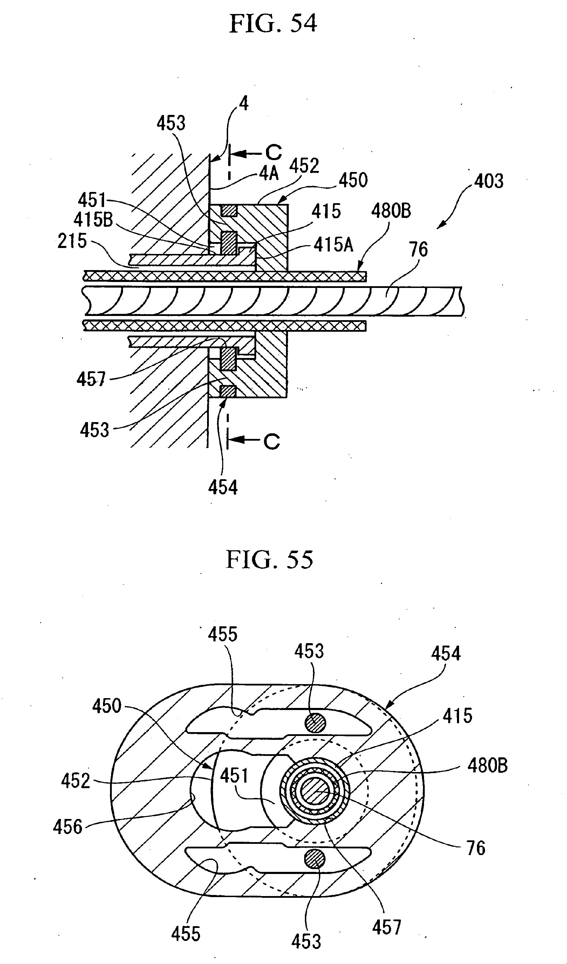

The insertion section 275 is inserted through the forceps channel 215 while the increased-diameter section 456 coincides with the recessed section 451 as illustrated in FIG. 52. Inserting until the distal end chip 281 of the outer sheath 480B abuts the abutment section 217 causes the cover 450 to be pressed onto the forceps port 415. Subsequently sliding the catching plate 454 as illustrated in FIGS. 54 and 55 causes the reduced-diameter section 457 to coincide with the recessed section 451.
This causes the forceps port 415 to be supported by the catching plate 454 and the cover 450, thereby fixing the outer sheath 480B via the cover 450. Therefore the autopsy cups 79 can be positioned in the chamber 116 easily because the outer sheath 480B does not move if the coil sheath 76 is taken out.

Since distal-end-regulating sections, i.e., the cover 450 and the catching plate 454 are provided to the outer sheath 480B of this case; and a regulating section, i.e., the flange section 415A of the forceps port 415 is provided to the endoscope 202, mere sliding of the catching plate 454 allows the outer sheath 480B to be positioned relative to the endoscope 202 and fixed there easily.

### (Sixth Embodiment)

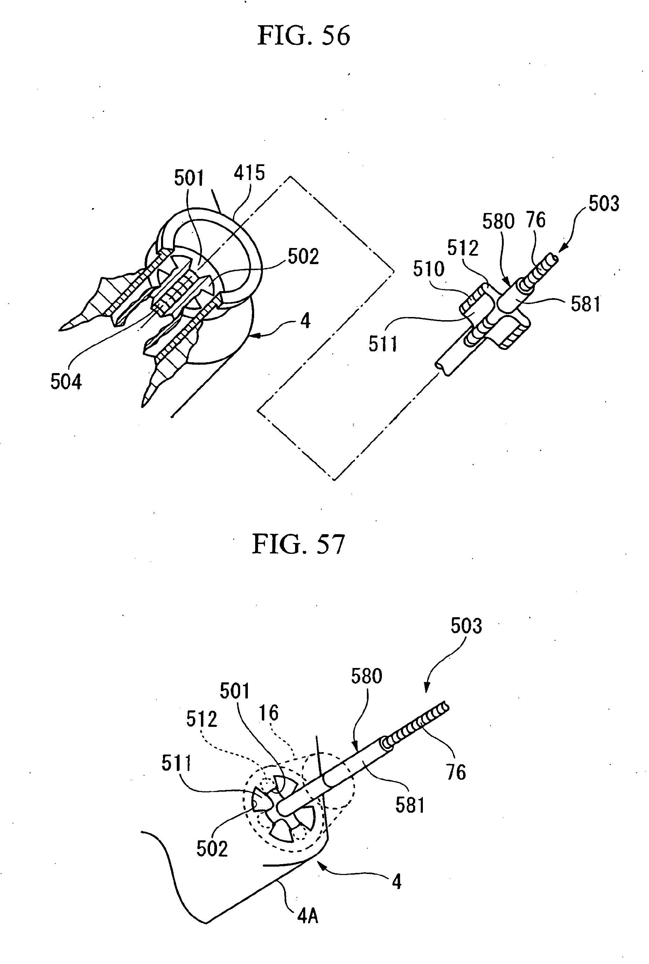

A sixth embodiment according to the present invention will be explained in detail with reference to FIGS. 56 and 57. The present embodiment is characterizedin a configuration where an outer sheath of an instrument is positioned and engaged to an endoscope. Other features are the same as those of the third embodiment or the fourth embodiment.

As illustrated in FIGS. 56 and 57, a regulating section, i.e., a female-thread section 501 is formed on an inner periphery in the vicinity of the forceps port 415 of a forceps channel 215. A plurality of slits 502 formed in a circumference direction of the female-thread section 501 in parallel with an axial direction. Projecting sections 504 defined by the slits 502 have female threads on the inner periphery thereof.

An outer sheath 580 of the instrument 503 has a tubular sheath main unit 581. A male-thread section 510 that serves as a distal-end-regulating section is fixed to the proximal end section of the sheath main unit 581. A plurality of recessed sections 511 are formed in a circumference direction of the male-thread section 510. Male threads are formed on an outer periphery of projecting sections 512 defined by the recessed sections 511. The recessed sections 511 are formed to stay away from the projecting sections 504 of the female-thread section 501.

Therapeutic maneuvering using an instrument 503 is conducted while a forceps plug 16 is attached to a forceps channel 215. The outer sheath 580 is inserted through the forceps channel 215 so that the projecting section 512 of theh male-thread section 510 enters between the projecting sections 504 of the female-thread section 501. Upon abutting the distal end chip 281 onto the abutment section 217, the outer sheath 580 is rotated around the axial line. This causes the male-thread section 510 to be screwed into the female-thread section 501.
The outer sheath 580 fixed to the forceps channel 215 does not permit movement of the distal end chip 281 if the coil sheath 76 is drawn; therefore, the distal end of the autopsy cups 79 is positioned upon abutting the distal end chip 281. Removing the instrument 503 from the endoscope 202 necessitates rotating the outer sheath 580 around the axial line and releasing the engagement between the projecting section 504 of the female-thread section 501 and the projecting section 512 of the male-thread section 510. The projecting section 512 can be removed while maintaining this state, i.e., the projecting sections 512 of the male-thread section 510 is accommodated in the slits 502 of the male-thread section 510.

The position of the distal end-treating section 277 can be fixed at a position that facilitates collecting of the grasped tissue W2 since the outer sheath 580 screwed into the forceps channel 215 can be fixed therein according to the present embodiment. Therefore, the grasped tissue W2 can be collected easily and reliably while maintaining an inserted state of the instrument 503.
The recessed sections between the female-thread sections 501 and the projecting sections between the male-thread sections 510 enable insertion and retraction while preventing the interference between the projecting sections 504 of the female-thread sections 501 and the projecting sections 512 of the male-thread sections 510. In addition, when fixing, the outer sheath 580 can be fixed with an insignificant force. Changing screwing-degree can adjust a diviation in length between the forceps channel 215 and the outer sheath 580 of the instrument 503, and this case of distal end chip 281 can be abutted to the abutment section 217 reliably.

The abutment section 217 that is preferable to be provided to the forceps channel 215 may not be provided, and instead, positioning of the distal end-treating section 277 may be conducted by abutting the distal end-treating section 277 to the outer sheath 580 based to a reference point where the male-thread section 510 is screwed into the female-thread section 501.

### (Seventh Embodiment)

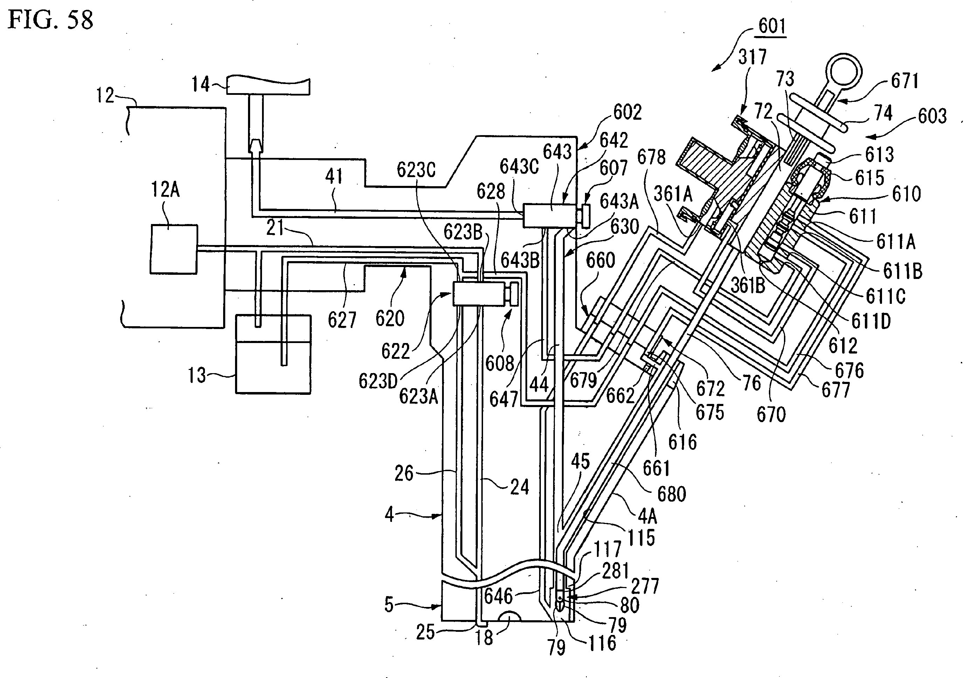

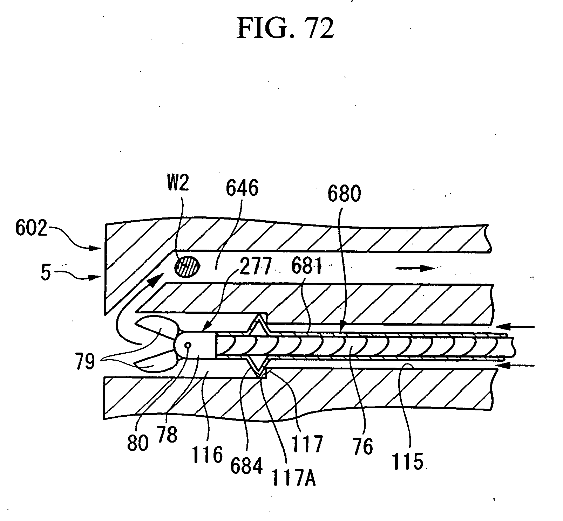

A seventh embodiment according to the present invention is explained as follows in detail with reference to FIGS. 58 to 81. Note that elements that are equivalent to those of the first embodiment will be assigned the same reference symbols and redundant explanations thereof will be omitted.

As illustrated in FIG. 58, an endoscope system 601 includes an endoscope 602 and an instrument 203 in configuration. The endoscope 602 has a first pipeline system 620 and a second pipeline system 630. A part of these pipeline systems are connected to a suctioning-and water-supplying button 610 and a tissue-grasping device 317 provided to the instrument 603. The first pipeline system 620 extending from a air-supplying source 12A is connected to a second port 623B of a first switching device 622 in the first pipeline system 620.
A air-supplying pipeline 24 is connected to a first port 623A of the first switching device 622. A water-supplying pipeline 26 is connected to a fourth port 623D. A water-supplying pipeline 627 is connected to a third port 623C. The water-supplying pipeline 627 subsequent to branching off to a tissue water-supply pipeline 628 is connected to a water-supplying tank 13. The tissue water-supply pipeline 628 has an opening in a connector 660 provided on a lateral section 4A of an endoscope-maneuvering section 4.

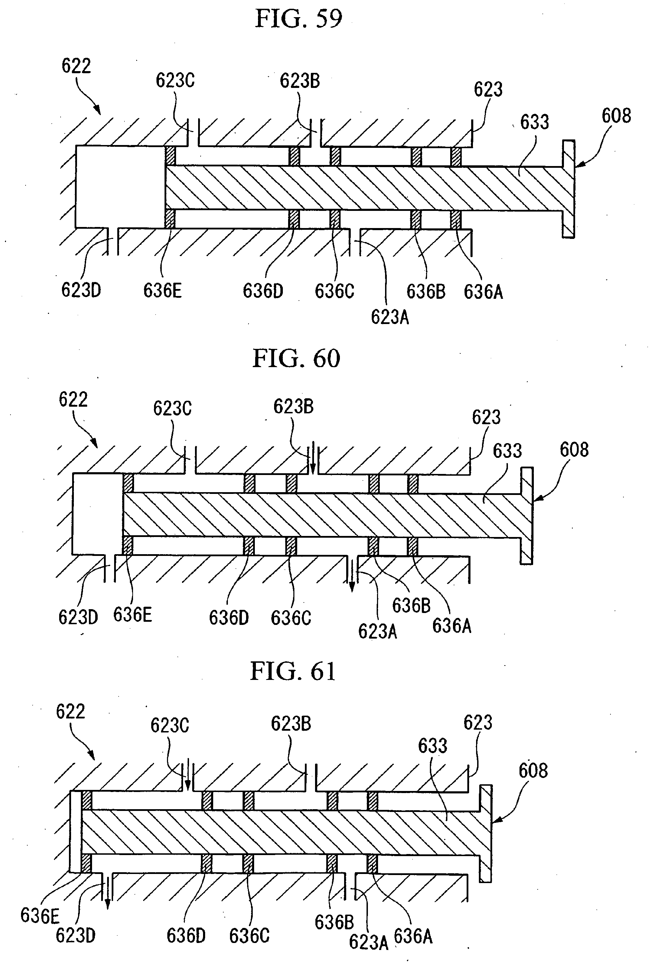

As illustrated in FIGS. 59 to 61, the first switching device 622 that undertake switching of flow paths has a sleeve 623 provided with four ports. A air-and-water-supplying button 608 is inserted into an opening of the sleeve 623. The air-and-water-supplying button 608 has a water-tight structure and a liquid-tight structure by a button main unit 633 that is narrower than the inner diameter of the sleeve 623; and a plurality of packings 636A to 636E fixed in a longitudinal direction of the button main unit 633.

FIG. 59 illustrates two packings 636A and 636B are disposed in the vicinity of the opening relative to the first port 623A; and the packing 636C disposed between the first and second port 623A,623B. The packing 636D is disposed between the second and third ports 623B and 623C. The packing 636E is disposed between the third and fourth ports 623C, 623D. All the ports 623C and 623D are separated in this case.
As illustrated in FIG. 60, a first press to the air-and-water-supplying button 608 cases the packing 636A and the packing 636B to stay in the vicinity of the opening section relative to the first port 623A, thereby allowing the packing 636C to move to between the second and third ports 623B and 623C; and causes the packing 636D so say between the second and third ports 623B and 623C, thereby causing the packing 636E to stay between the third and fourth ports 623C, 623D. In this case, the first port 623A is connected to the second port 623B.
A second press, i.e., further pressing the air-and-water-supplying button 608 as illustrated in FIG. 61 causes the packing 636A to stay in the vicinity of the opening section relative to the first port 623A, thereby causing the packing 636B to move to between the first and second ports 623A and 623B; and causes the packing 636C and the packing 636D to stay between the second and third ports 623B and 623C. The packing 636E moves across the fourth port 623D to the other, i.e., the block end of the sleeve 623. In this case, the third port 623C is connected to the fourth port 623D.

As illustrated in FIG. 58, a second pipeline system 630 has a suction pipeline 41 that is connected to a suction source 14. The suction pipeline 41 is connected to a third port 643C of the second switching device 642. A suction pipeline 44 is connectedt to the first port 643A of the second switching device 642. The suction pipeline 44 is connected to a forceps channel 115. A tissue-suctioning pipeline 646 connected in a slanting manner to a chamber 116 of the forceps channel 115 has an opening in the connector 660 provided to the lateral section 4A of the endoscope-maneuvering section 4.
A tissue-suctioning pipeline 647 is connected to the second port 643B of the second switching device 642. The tissue-suctioning pipeline 647 has an opening in the connector 660 provided to the lateral section 4A of the endoscope-maneuvering section 4. A forceps plug 616 is attached to the forceps channel 115. A pipe way 661 is formed in the forceps plug 616. The pipe way 661 communicating to the connector 660 through a bridge 662 has an opening in the connector 660.

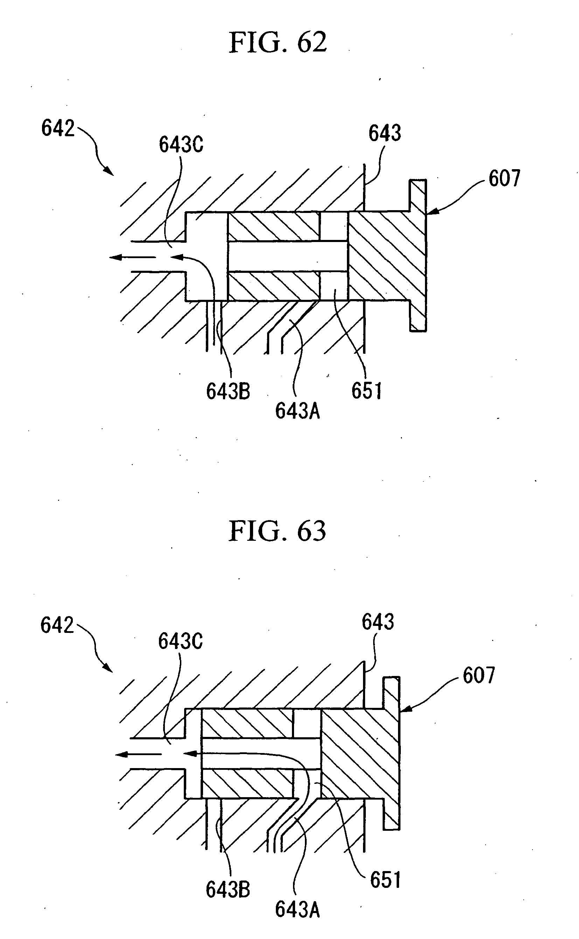

As illustrated in FIGS. 62 and 63, the second switching device 642 that undertake switching of flow paths has a sleeve 643 provided with three ports. An end of the sleeve 643 is blocked and the other end opens so as to allow the suction button suction button 607 to freely extend or retract therethrough. The outer diameter of the suction button 607 that is substantially the same as the inner diameter of the sleeve 643 form an airtight structure. In addition, a communication hole 651 is formed in the suction button 607. The communication hole 651 runs from an opening formed on the lateral section through the suction button 607 and opens at the distal end section directed to the blocked end of the sleeve 643.

FIG. 62 shows a porition so that the first port 643A is blocked, and the second port 643B communicates to the third port 643C. As illustrated in FIG. 63, pressing the suction button 607 causes the first port 643A to communicate to the third port 643C and causes the second port 643B to be blocked.

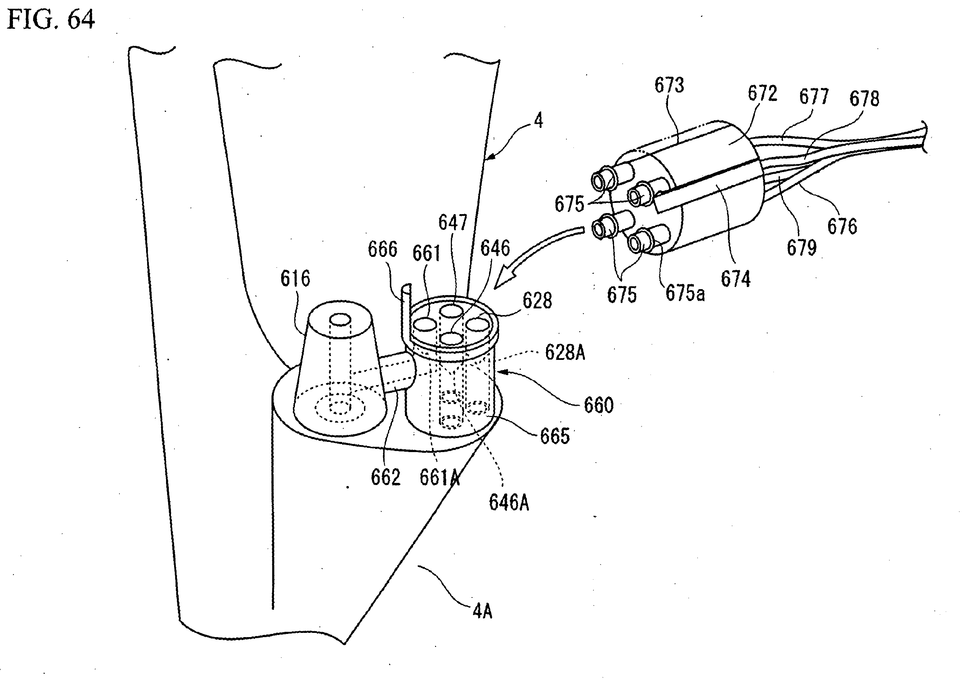

FIG. 64 shows an example of the connector 660. The connector 660 has a cylindrical connector main unit 665 fixed on the laterat section 4A of the endoscope-maneuvering section 4. Ends of pipe ways 628, 646, 647, and 661 extends in parallel from the connector main unit 665. An engagement section 666 is provided to project from an exterior of the connector main unit 665.
The engagement section 666 is configured to engage with a notched section 673 formed on an outer periphery section of a manifold 672 of the maneuvering section 671 of the engagement section 666. Check valves 628A, 646A, and 661 A provided in the vicinities of the openings of three pipe ways 628, 646, and 661 can maintain airtight condition in pipelines inward relative to the check valves 628A, 646A, and 661A.

As illustrated in FIG. 58, a tissue-grasping device 317 and a suctioning-and-water-supplying switch 610 are fixed on the maneuvering section main unit 72 of the maneuvering section 671 of the instrument 603. A manifold 672 is provided to have four tubes 676 to 679 extending from the suctioning-and-water-supplying switch 610.

Provided to project from a lateral section of the manifold 672 is a jaw section 674 that engages to a gap section of the connector main unit 665 of the connector 660. Four engagement pipes 675 are provided to receive the ends of the pipe ways 628, 646, 647, and 661. The engagement pipes 675 are disposed to coincide with the ends of the pipe ways 628, 646, 647,and 661. The outer diameters of the engagement pipe 675 smaller than the opening diameters of the ends of the pipe ways 628, 646, 647,and 661 of the connector 660 allow the engagement pipes 675 enter the ends of the pipe ways 628, 646, 647,and 661. O-rings 675a attached to the engagement pipe 675 ensure airtight condition between the engagement pipes 675 and the pipe ways 628, 646, 647,and 661. Tubes 676 to 679 each are connected to the engagement pipe 675 respectively.

That is, the tissue water-supply pipeline 628 of the first pipeline system 620 can be connected to the water-supplying tube 676 through the engagement pipe 675. The water-supplying tube 676 is connected to the second port 611B of the suctioning-and-water-supplying switch 610. The end section of the side hole 661 of the forceps channel 115 of the second pipeline system 630 can be connected to the water-supplying tube 677 through the engagement pipe 675. The water-supplying tube 677 is connected to the first port 611A of the suctioning-and-water-supplying switch 610.
The end section of the tissue-suctioning pipeline 646 can be connected to the suction tube 678 through the engagement pipe 675. The suction tube 678 is connected to the opening section 361A of the casing 361 of the tissue-grasping device 317. The end section of the tissue-suctioning pipeline 647 can be connected to the suction tube 679 through the engagement pipe 675. The suction tube 679 is connected to a fourth port 611D of the suctioning-and-water-supplying switch 610.

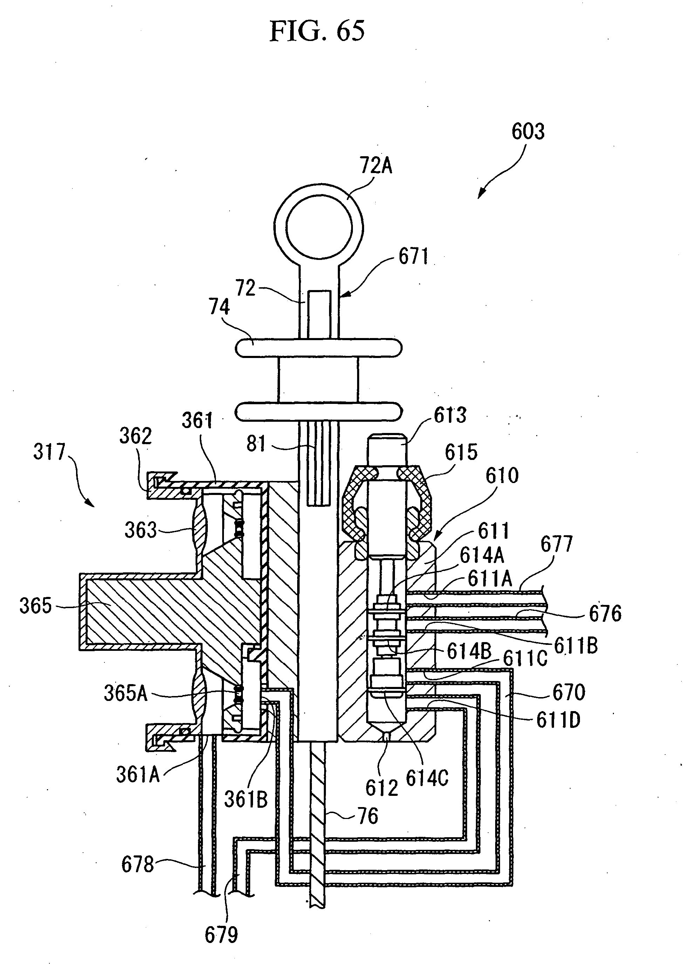

As illustrated in FIG. 65, the suctioning-and-water-supplying switch 610 has a sleeve 611 provided with four ports 611A to 611D. A suction tube 670 is connected to the third port 611C. The suction tube 670 is connected to an opening section 361B of a bottom section of the tissue-grasping device 317. An end section of the sleeve 611 is tapered, i.e., reduced in diameter and has a leak hole 612 formed thereon. A switch main unit 613 capable of freely sliding is inserted from the opening formed on the other end section.
A plurality of valving elements 614A to 614C are fixed on the switch main unit 613 in a longitudinal direction. An elastic material 615 is attached to a portion of the switch main unit 613 that is exposed outward from the sleeve 611. This elastic material 615 urges the switch main unit 613 in a retracting-direction relative to the sleeve 611.

A valving element 614A is positioned between the first port 611 A and the second port 611B and a valving element 614B is positioned between the second port 611B and third port 611C as long as the switch main unit 613 is not pressed. The fourth port 611D communicating to the leak hole 612 is released to an ambient while the valving element 614C does not allow this state of the fourth port 611 D tot communicate to the third port 611C.

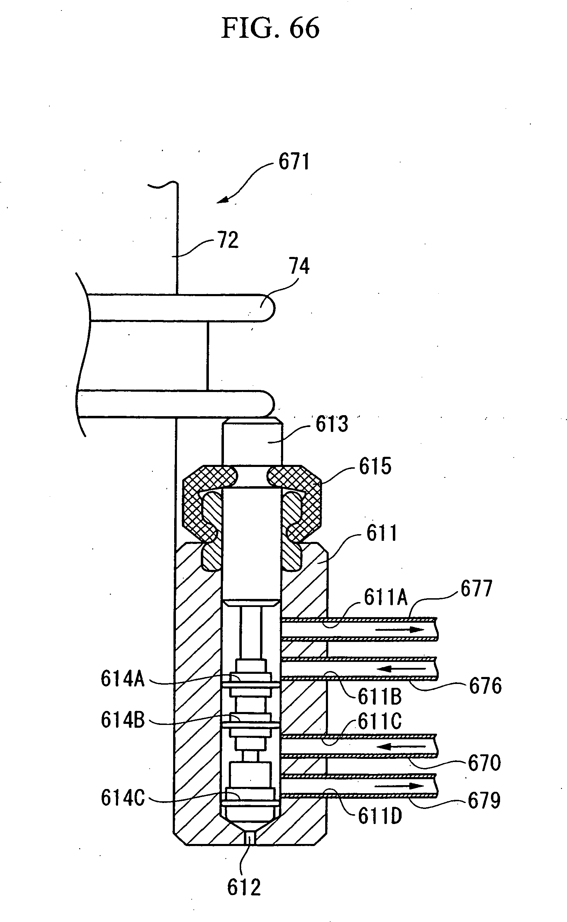

FIG. 66 shows the switch main unit 613 pressed by the slider 74; the first port 611A communicating to the second port 611B; and the third port 611C communicating to the fourth port 611D. In addition, the leak hole 612 is blocked by the distal end section of the switch main unit 613 upon making contact to the taper of the sleeve 611.

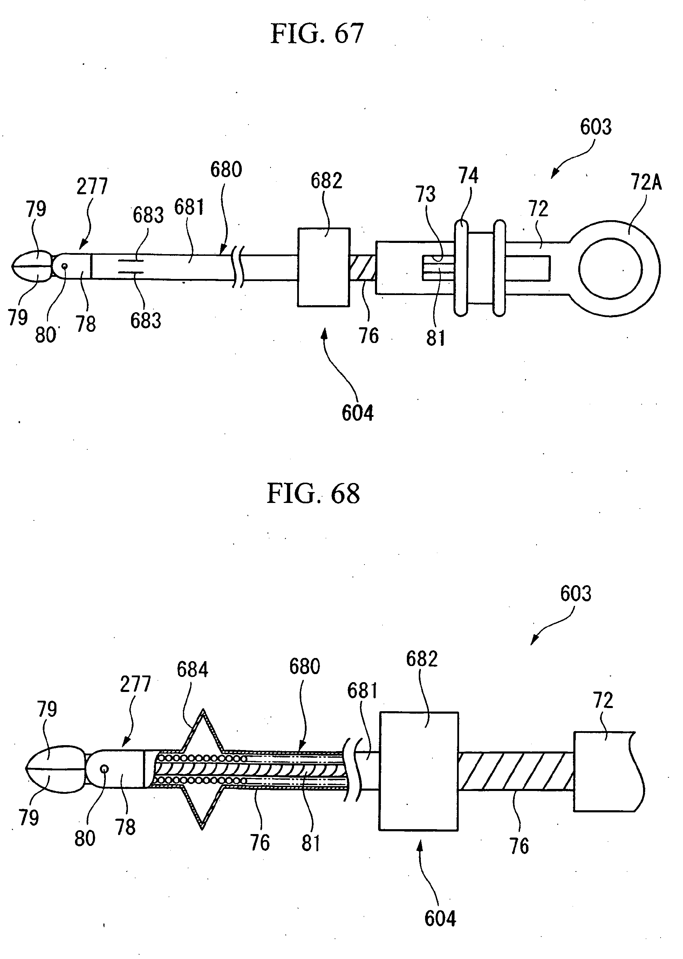

As illustrated in FIGS. 58 and 67, an insertion section 604 extends from a maneuvering section main unit 72 of a maneuvering section 671 of the instrument 603. A maneuvering wire 81 capable of freely extending or retracting is inserted through a densely-wound coil sheath 76 in the insertion section 604. In addition, an outer sheath 680 capable of freely sliding is provided to cover the outer periphery of the coil sheath 76.
The outer sheath 680 has a tubular sheath main unit 681. A slider 682 having an increased diameter and provided to the proximal end section of the sheath main unit 681 enables, when grasped, extending or retracting of the outer sheath 680 relative to the coil sheath 76. The distal end section of the sheath main unit 681 of the outer sheath 680 is fixed to a distal end-treating section 77.

A plurality of slits 683 are formed in the vicinity of the distal end of the outer sheath 680. Each one of, for example, four or more slits 683 that are in parallel to each other, extends in a longitudinal direction. As illustrated in FIG. 68, advancing of the slider 682 causes the positions corresponding to the slits 683 formed there to bend and project outward since the distal end section of the outer sheath 680 is fixed to the distal end-treating section 277 while the sheath main unit 681 pushed toward the distal end. This allows a part of the outer sheath 680 to serve as increased-diameter sections 684 (freely-projecting-and-recessing sections) projecting outward relative to the outer diameter of the distal end-treating section 277.

The maximum diameter of the outer sheath 680 upon projecting the increased-diameter section 684 is smaller than the diameter of the chamber 116 of the tissue water-supply pipeline 28 and greater than a diameter of a section in the vicinity of the proximal end relative to the chamber 116, i.e., the inner diameter of the abutment section 117. Therefore, the forceps channel 115 allows a non-pressed state of the outer sheath 680 to be inserted therethrough.

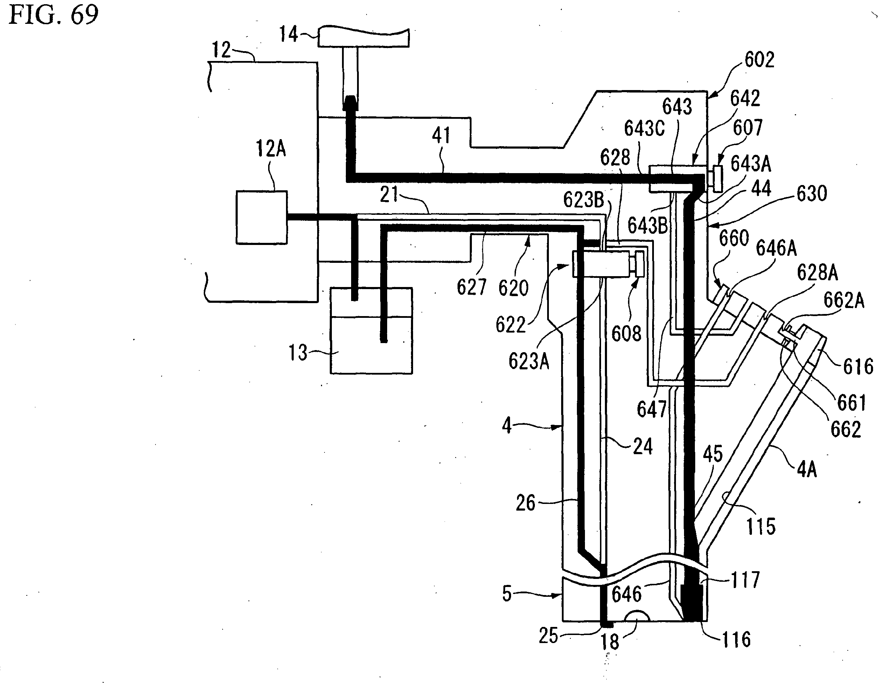

Operations in the present embodiment are explained as follows.
As illustrated in FIG. 69, a discrete use of the endoscope 602 necessitates a tissue water-supply pipeline 628 to be blocked by a check valve 628A and a tissue-suctioning pipeline 646 to be blocked by a tissue-suctioning pipeline 646. The forceps channel 115 is blocked by the forceps plug 616 and the check valve 661 A of the pipe way 661. Therefore, ordinary water-supply from the distal end section of the endoscope 602 can be conducted by discharging water having passed through the water-supplying pipeline 627, the first switching device 622, the water-supplying pipeline 26 from the nozzle 25.
On the other hand, ordinary suctioning is conducted by connecting the suction pipeline 41 to the forceps channel 115 through the second switching device 642 and the suction pipeline 44, and suctioning from the chamber 116 of the distal end section.

As illustrated in FIG. 64, inserting of the instrument 603 through the forceps channel 115 necessitates attaching the packings 636A to 636E to the connector 660. The engagement pipes 675 of the manifold 672 are inserted into the pipe ways 628, 646, 647,and 661; the check valves 628A, 646A, and 661A are opened; and the tubes 676 to 679 are connected to the corresponding pipe ways 628, 646, 647,and 661.

As illustrated in FIG. 65, the ports 611A to 611D are separated since the switch main unit 613 of an initial state of the suctioning-and-water-supplying switch 610 is at a retracted position; therefore, the suction tube 679 connected to the fourth port 611D is released to an ambient through the leak hole 612. In this state, ordinary water-supply or suctioning can be conducted by the endoscope 602 similarly to the case of FIG. 69.

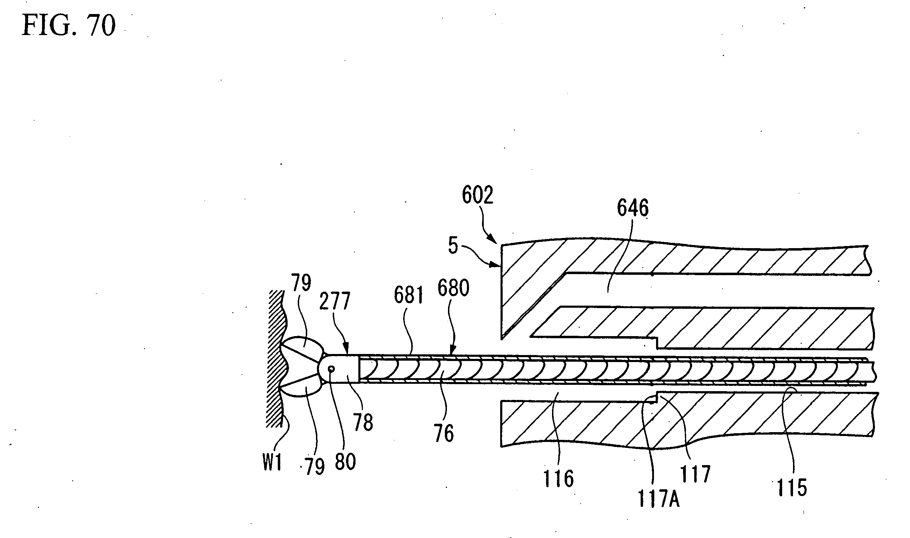

Conductinf of biopsy necessitates advancing the whole instrument 603. As illustrated in FIG. 70, this state of outer sheath 680 in conjunction projects from the distal end section of the endoscope 602. An increased-diameter section 684 is formed by grasping a part of living tissue of a mucosa W1 by opening and closing the autopsy cups 79; and advancing the slider 682 of the outer sheath 680. Retracting the whole instrument 603 maintaining the current state of the increased-diameter section 684 causes the living tissue grasped by the autopsy cups 79 to be tomed, thereby obtaining a grasped tissue W2. Subsequently the distal end-treating section 77 is retracted into the forceps channel 115. This state of the increased-diameter section 684 abuts on the abutment section 117 of the chamber 116.
Accordingly, the increased-diameter section 684 operative as a distal-end-regulating section causes the distal end-treating section 277 to be positioned in the vicinity of the proximal end relative to the tissue-suctioning pipeline 646 in the chamber 116.

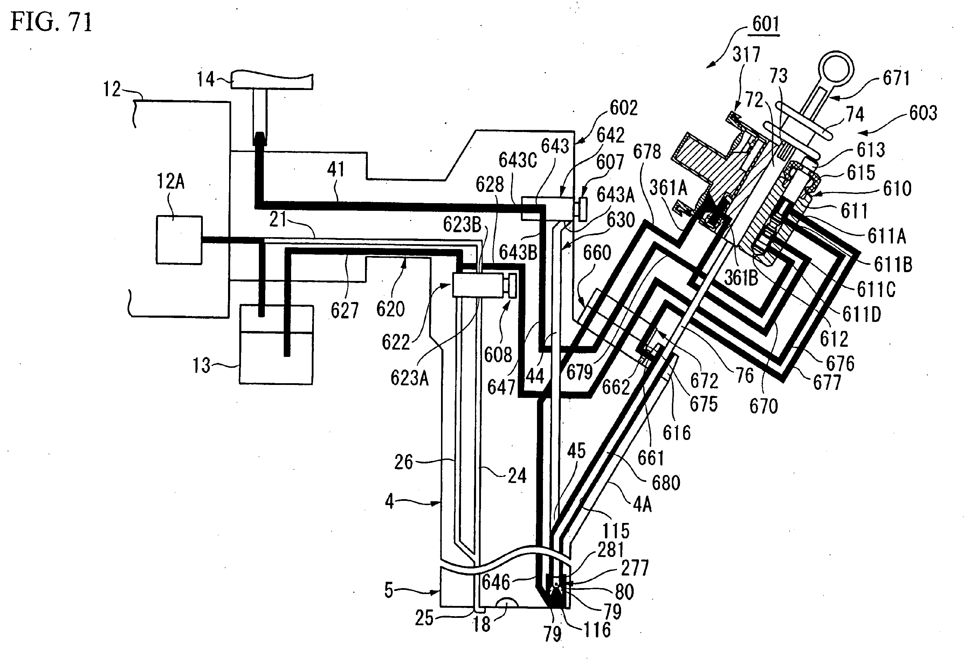

As illustrated in FIG. 71, advancing of the slider 74 of the maneuvering section 671 allows a pair of the autopsy cups 79 connected through the maneuvering wire 81 to open. Simultaneously, the suctioning-and-water-supplying switch 610 pressed by the slider 74 convinces water-supply and suctioning. That is, water-supply is conducted through the water-supplying pipeline 627, the tissue water-supply pipeline 628, the water-supplying tube 676, the second port 611 B of the suctioning-and-water-supplying switch 610, the first port 611 A, the water-supplying tube 677, the pipe way 661 of the forceps plug 616, and the forceps channel 115.
Suctioning is conducted through the tissue-suctioning pipeline 646, the suction tube 678, the tissue-grasping device 317,the suction tube 670,the suctioning-and-water-supplying switch 610,the suction tube 679,the tissue-suctioning pipeline 647,the second switching device 642, and the suction pipeline 41 since the third port 611C is connected to the fourth port 611D in the suctioning-and-water-supplying switch 610.

This results in that liquid passing through between the abutment section 117 and the increased-diameter section 684 and flowing into the chamber 116 causes the grasped tissue W2 to be separated from the autopsy cups 79, thereby suctioning togethere with the grasped tissue W2 from the tissue-suctioning pipeline 646. Serial biopsy repeats these operations. Removing of the instrument 603 from the endoscope 602 necessitates retracting the outer sheath 680; restoring the increased-diameter section 684 to a straightened state; and removing the whole instrument 603.

The present embodiment can provide a simple configuration of instrument 603 since the tissue-grasping device 317 and the suctioning-and-water-supplying switch 610 are provided to the instrument 603 so that the grasped tissue W2 can be collected by using pipelines of the endoscope 602.
Mere opening or closing of the autopsy cups 79 allows water-supply and suctioning operations for collecting the grasped tissue W2, thereby improving operability since turning on the suctioning-and-water-supplying switch 610 upon maneuvering the slider 74 of the maneuvering section 671 commences water-supply and suctioning.
The pipe ways 628, 646, 647,and 661 can be connected to the tubes 676 to 679 correctly since positioning of components relatively can be obtained coinciding the engagement section 666 with the notched section 673 when attaching the manifold 672 to the connector 660.

Proposed for modified examples of the present embodiment are as follows.

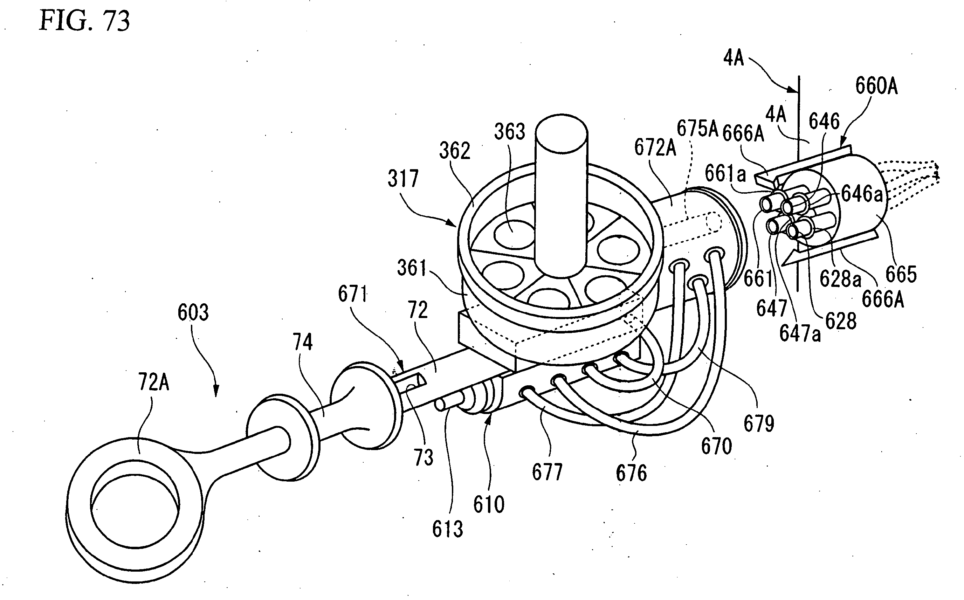

FIG. 73 proposes another example of manifold. A manifold 672A is fixed to a tissue-grasping device 317 of the instrument 603 as illustrated in FIG. 73. The manifold 672A that is attached to the connector 660A of the endoscope-maneuvering section 4 has four recessed sections 675A that can accommodate the end sections of the pipe ways 628, 646, 647,and 661. It should be noted that FIG. 73 shows a piece of recessed section 675A.
Each recessed section 675A having one of the tubes 676 to 679 is configured to be connected to the pipe ways 628, 646, 647,and 661 similarly to the previous explanation. Airtight state in the pipe ways 628, 646, 647,and 661 having O-rings 628a, 646a, 647a,and 661a is maintained upon connecting to the recessed section 675A. End sections of the pipe ways 628, 646, 647,and 661 project from the connector main unit 665 of the connector 660A, and two jaw sections 666A extend from the lateral section of the connector main unit 665. Engaging of the jaw section 666A to the manifold 672A allows the connector 660A to be fixed.

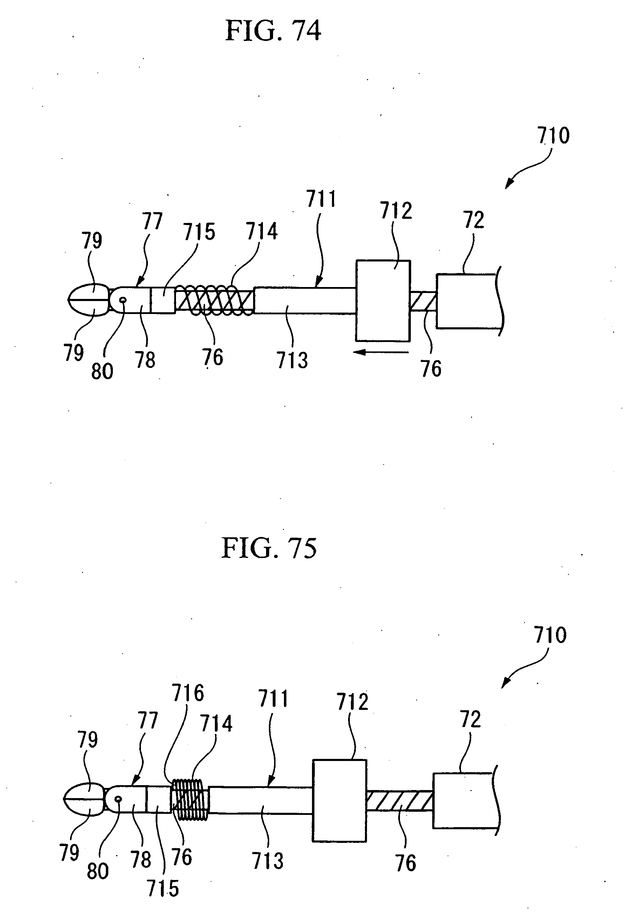

FIG. 74 proposes another example of increased-diameter section. The outer sheath 711 of the instrument 710 illustrated in FIG. 74 is formed by a slider 712; a sheath main unit 713; and a distal end sheath section 715 joined to the distal end of the sheath main unit 713 via spiral wires 714 (freely-projecting-and-recessing section). The distal end sheath section 715 is fixed to the distal end-treating section 77. The length of the distal end sheath section 715 in an axial line direction is substantially the same as the length between the distal end of the outer sheath 680 and a position where the slits 683 are formed.
The wires 714 disposed at a predetermined interval are wound around the outer periphery of the coil sheath 76 spirally. Accordingly, advancing the slider 712 and moving the sheath main unit 713 toward the distal end sheath section 715 cause the wires 714 to overlap and bulge in a radial direction, thereby forming the increased-diameter section 716. The increased-diameter section 716 of the outer sheath 711 that is operable as a distal-end-regulating section in this configuration can obtain the same effects as those previously explained.

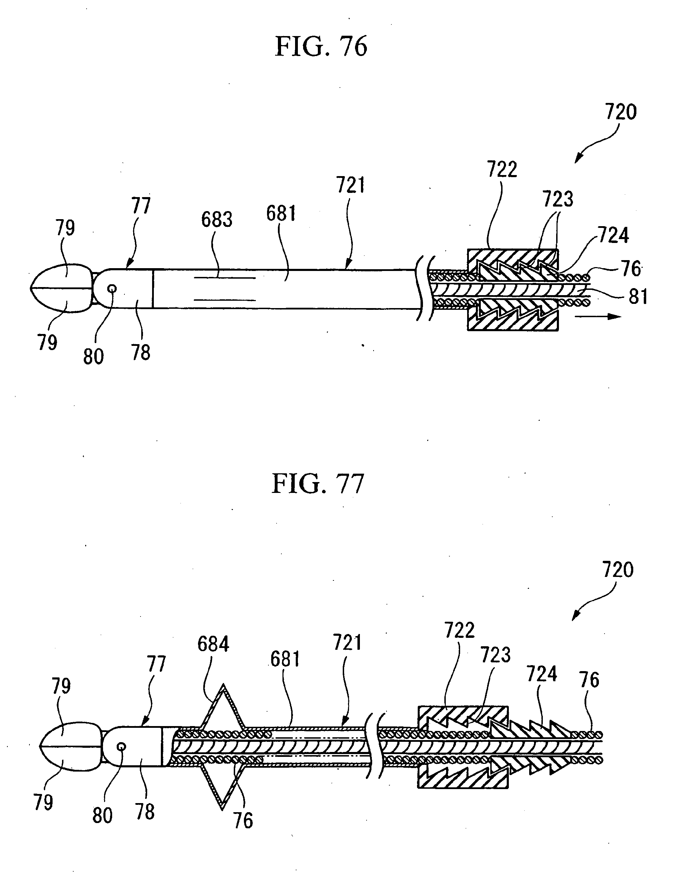

As illustrated in FIG. 76, a ratchet section 723 may be formed on an inner periphery of an elastic cylindrical member, i.e., a slider 722 of an outer sheath 721 of an instrument 720 fixed to a sheath main unit 681. The ratchet section 723 has a sawtooth formed by inclining surfaces that incline relative to an axial line and open toward the distal end; and orthogonal surfaces that are orthogonal with respect to the axial line direction.
On the other hand, a sawtooth-shaped engagement section 724 capable of engaging with the ratchet section 723 is formed to the coil sheath 76. An initial state of the engagement section 724 housed in the slider 722 does not cause the portion where slits 683 of the outer sheath 721 are formed to be deformed. Therefore, this state of the instrument 720 can be inserted through an endoscope.

The instrument 720 upon grasping a living tissue of a mucosa W1 by autopsy cups 79 retracts a coil sheath 76 with momentum while maintaining a slider 722 of the outer sheath 721. As illustrated in FIG. 77, this results in that the engagement section 724 is retracted partly from the slider 722, thereby pressing the sheath main unit 681 of the outer sheath 721 relatively. The distal end section of the sheath main unit 681 and the distal end section of the coil sheath 76 that are fixed to the distal end-treating section 77 cause the sheath main unit 681 to deform outward in a radial direction at a position where the slits 683 are formed, thereby forming an increased-diameter section 684. Accordingly, abutting the increased-diameter section 684 thereto enagles positioning of the distal end-treating section 77.

The engagement obtained between the ratchet section 723 and the engagement section 724 in a retracting direction of the coil sheath 76 relative to the outer sheath 721 does not cause the position of the coil sheath 76 relative to the outer sheath 721 to change if a force for drawing the coil sheath 76 is released, thereby maintaining an open state of the increased-diameter section 684. Therefore, retracting the whole instrument 720 causes the grasped tissue W2 to be torn and the distal end-treating section 77 to be retracted in to the forceps channel 115, thereby suspending the increased-diameter section 684 upon abutting to the abutment section 117.
Removing the instrument 720 from the endoscope 602 necessitates moving the engagement section 724 retracted from the slider 722 to the initial position as illustrated in FIG. 76, and the sheath main unit 681 of the outer sheath 721 to be drawn back. Accordingly, this allows this state of the instrument 720 to be removed from the endoscope 602 since the increased-diameter section 684 closes and a portion where the slits 683 are formed does not deform.

Operations by the instrument 720 are simple since captureing the grasped tissue W2 necessitates advancing the outer sheath 721 relatively, thereby enabling to form the increased-diameter section 684. In addition, operabiity is improved since releasing the outer sheath 721 does not cause the increased-diameter section 684 to restore.

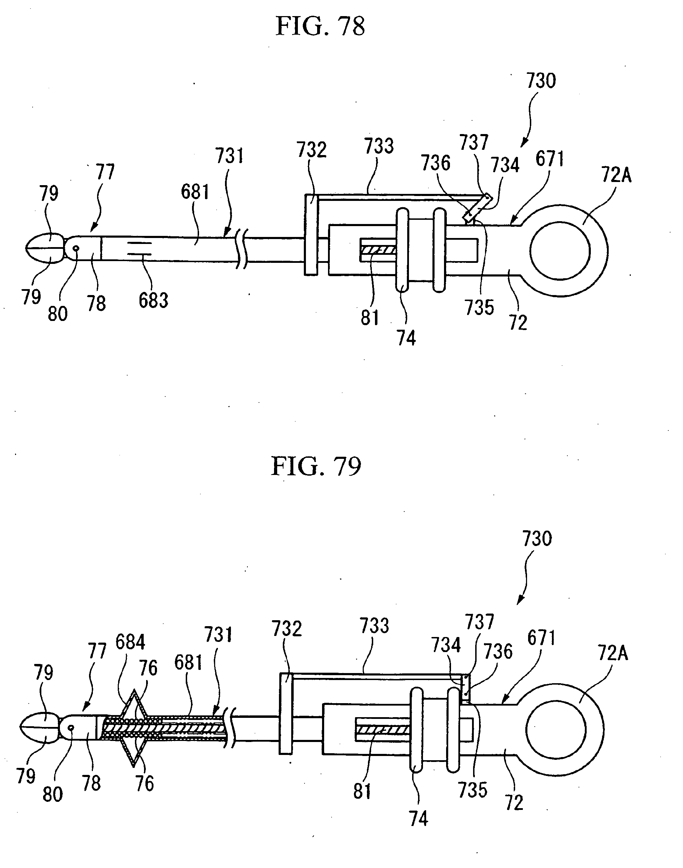

As illustrated in FIG. 78, the proximal end section of a shaft 733 fixed to a slider 732 of an outer sheath 731 of an instrument 730 may be connected to a maneuvering section main unit 72 of a maneuvering section 671 via a crank member 734. An end section of the crank member 734 is supported rotatively by a pin 736 at a projection 735 provided in the vicinity of a ring 72A relative to the moveing area of the slider 74 when the autopsy cups 79 is opened or closed. The other end section of the crank member 734 is joined rotatively to a shaft 733 via a pin 737 in further vicinity of the ring 72A relative to a supporting position of the pin 736. Therefore, an end surface of an initial state of the crank member 734 is positioned in the vicinity of the slider 74 beyond the pin 736.

As illustrated in FIG. 79, further retracting the slider 74 from the closed state of the cup 79 causes the end section of the crank member 734 to be pressed by the filter 65, thereby rotating the crank member 734 around the pin 736. This results in that the other end section of the crank member 734 to move toward the front, thereby causing the slider 732 of the outer sheath 731 to be pressed toward the distal end via the shaft 733. This results in causing the outer sheath 731 to advance, thereby forming the increased-diameter section 684. Maintaining the retracted state of the slider 74 of the maneuvering section 671 maintains a projecting state of the increased-diameter section 684.
On the other hand, slightly advancing the slider 74 of the maneuvering section 671 and separateing the slider 74 from the crank member 734 release the power that presses the crank member 734, thereby causing the slider 732 to move back to the proximal end with a force that restores the increased-diameter section 684 and obtaining a flat outer sheath 731.

The increased-diameter section 684 used for therapeutic maneuveringis formed by drawing the slider 74 of the maneuvering section 671; grasping the grasped tissue W2 by the autopsy cups 79; and further drawing the slider 74. Drawing this state of the whole instrument 730 causes the grasped tissue W2 to be tom. Subsequently, water-supply and suctioning are conducted upon abutting the increased-diameter section 684 ot the abutment section 117 of the forceps channel 115.
Advancing the slider 74 of the maneuvering section 671 and opening the autopsy cups 79 while maintaining the position of the instrument 730 cause the grasped tissue W2 to be separated from the autopsy cups 79 by the supplied water, thereby collecting the grasped tissue W2 from the tissue-suctioning pipeline 646. The whole instrument 730 is drawn and removed from the endoscope 602 upon capturing the whole grasped tissue W2 while maintaining a closed state of the autopsy cups 79 and a separated state of the slider 74 from the crank member 734.

Opening and closing the autopsy cups 79 once upon forming the increased-diameter section 684 of the instrument 730 causes the crank member 734 to rotate, thereby re-flattening the increased-diameter section 684; therefore, operations of the instrument 730 are simple; thus, the instrument 730 can be removed without conducting additional operations.

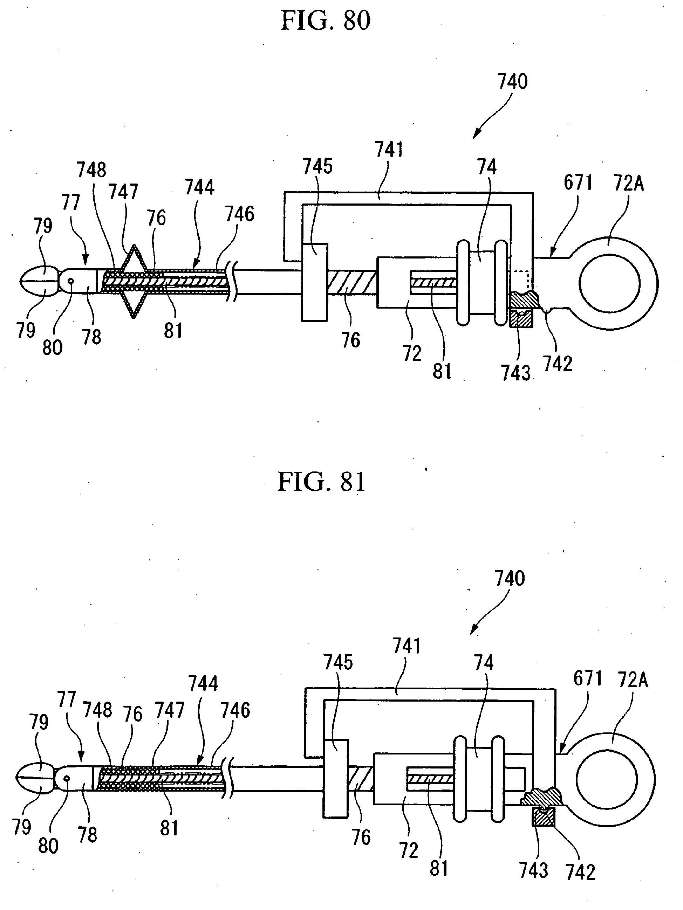

As illustrated in FIG. 80, the proximal section of the hook 41 capable of freely moving in an axial direction may be attached between the slider 74 of the maneuvring section main unit 72 of the instrument 740 and the ring 72A. A projecting section 742 is provided to project from the maneuvering section main unit 72. A hole formed on the hook 41 allows the maneuvering section main unit 72 to pass therethrough. The projecting section 742 can engage with a recessed section 743 formed on an inner periphery of the hole. The hook 41 extends toward the distal end while avoiding interfering the movement of the slider 74. Its distal end section abuts from the distal end onto the distal end surface of the slider 745 of the outer sheath 744.

Extending from the slider 745 of the outer sheath 744 is a tubular sheath main unit 746. The distal end of the sheath main unit 746 is connected to a sheath distal end section 748 via an increased-diameter section 747 (freely-projecting-and-recessing section). The sheath distal end section 748 is fixed to a distal end-treating section 77. The increased-diameter section 747 is formed by a deformed coil produced by a molding method to project outward in a radial direction in a natural condition, or a leaf spring, etc.
As illustrated in FIG. 81, engaging the recessed section 743 of the hook 41 of the instrument 740 with the projecting section 742 of the maneuvering section main unit 72 causes the distal end section of the hook 41 to draw the slider 745 of the outer sheath 744 toward the maneuvering section 671, thereby straightening the increased-diameter section 747 that is as if drawn by the sheath main unit 746.

Maneuvering necessitates inserting the recessed section 743 engaged with the projecting section 742 and the flattened state of the increased-diameter section 747 through the forceps channel 115 of the endoscope 602, opening and closing the autopsy cups 79, and grasping the grasped tissue W2. Releasing this state of engagement between the recessed section 743 and the projecting section 742 releases the force that deforms to flatten the increased-diameter section 747; thereby causing the increased-diameter section 747 to project. Retracting the whole instrument 740 causes the grasped tissue W2 to be torn and the increased-diameter section 747 to abut on the abutment section 117 of the forceps channel 115. Subsequently opening the autopsy cups 79 while conducting water-supply and suctioning cause the grasped tissue W2 to be collected.
Removing of the instrument 740 necessitates retracting the hook 41 and engaging the recessed section 743 with the projecting section 742. Since the distal end section of the hook 41 causing the slider 745 of the outer sheath 744 to retract extends the increased-diameter section 747, this state of the whole instrument 740 is removed. Retraction of the hook 41 and the engagement between the recessed section 743 and the projecting section 742 may be accomplished by drawing the hook 41 with the slider 74 or drawing the hook 41 alone.

Operability of the instrument 740 is improved since the outer sheath 744 is provided with the increased-diameter section 747 that projects in a natural state is configured to flatten the increased-diameter section 747 by engaging the slider 745 of the outer sheath 744 to the hook 41 and drawing the hook 41 toward the ring 72A, thereby enabling operations for opening and closing operations of the autopsy cups 79 and positioning the distal end-treating section 77 by advancing and retracting the slider 74 of the maneuvering section 671.

### (Eighth Embodiment)

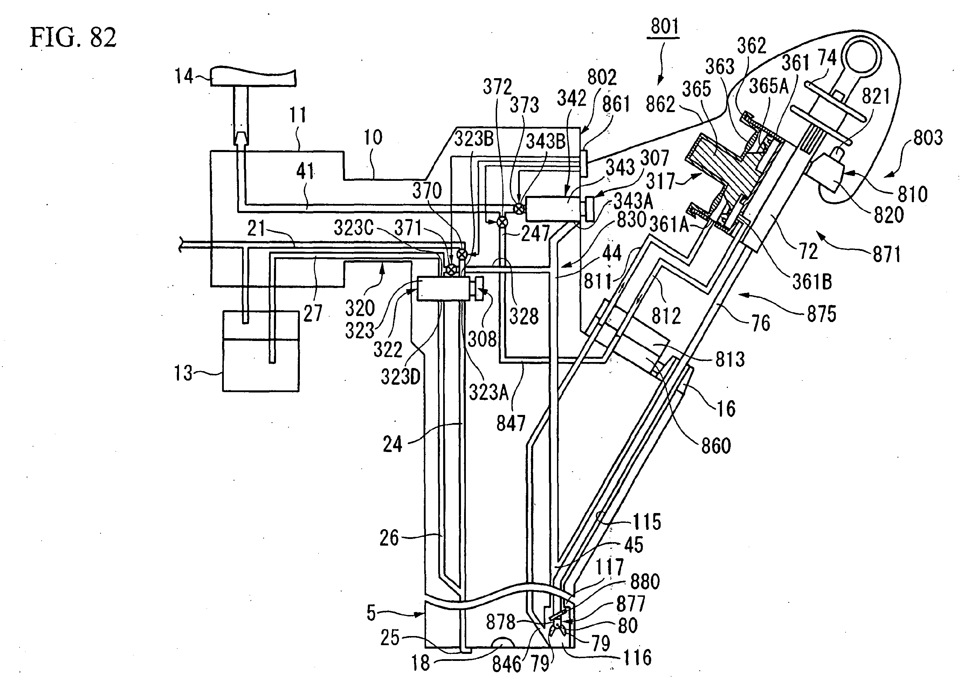

An eighth embodiment according to the present invention is explained as follows in detail with reference to FIGS. 82 to 86. Note that elements that are equivalent to those of the first embodiment will be assigned the same reference symbols and redundant explanations thereof will be omitted.

As illustrated in FIG. 82, an endoscope system 801 includes an endoscope 802 and an instrument 203 in configuration. Provided to a maneuvering section 871 of the instrument 803 are a tissue-grasping device 317 and a suctioning-and-water-supplying switch 810. Suction tubes 811, 812 each are connected to an opening section 361A of a lateral section of a casing 361 of the tissue-grasping device 317 and to an opening section 361B of a bottom section of the casing 361. The suction tubes 811 and 812 are inserted into a manifold 813.
A sleeve 820 of the suctioning-and-water-supplying switch 810 is fixed on a maneuvering section main unit 72. A switch main unit 821 capable of undertaking a press onto the sleeve 820 is projected toward the slider 74. An insertion section 875 has a densely-wound coil sheath 76 having a distal end-treating section 877 formed on the distal end of the coil sheath 76. The diameter of a forceps distal end section 878, operable as a distal-end-regulating section, of the instrument 803 is greater than the outer diameter of the coil sheath 76.

The endoscope 802 has a first pipeline system 320 and a second pipeline system 830. The configuration of the first pipeline system 320 is the same as that of the fourth embodiment. Only tissue-suctioning pipelines 846 and 847 of the second pipe way system 830 are different from those of the fourth embodiment. That is, the tissue-suctioning pipeline 847 branchig off from a suction pipeline 41 has a check valve 372 provided in the middle of the pipeline, and has an opening on a connector 860 provided to a lateral section 4A of the endoscope-maneuvering section 4.
Similarly, the tissue-suctioning pipeline 846, connected to the chamber 116 of the forceps channel 115 in a slanting manner, has an opening in the connector 860. Only the tissue-suctioning pipeline 846 has a check valve (not shown in the drawing) provided to an end section within the connector 860. Although the connector 860 and the corresponding manifold 813 provided to the instrument 803 are modified to have a different number of connector 660 and the manifold 672 according to the seventh embodiment, mechanisms are similar.

Signal lines for controlling the check valves 370 to 373 are connected to the connector 861 provided in the endoscope-maneuvering section 4. A signal line 862 extending from the suctioning-and-water-supplying switch 810 of the instrument 803 can be attached to the connector 861.

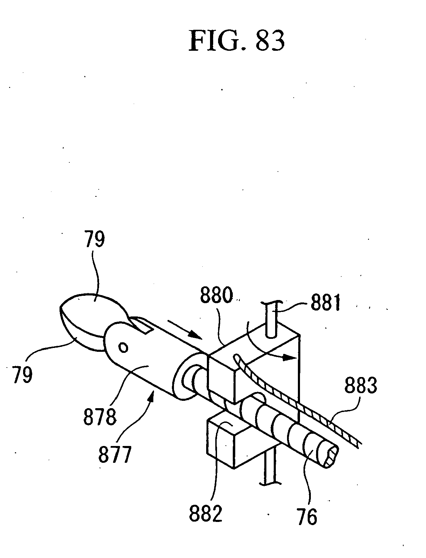

A rasing stand 880 is a regulating section provided to the chamber 116 of the forceps channel 115. As illustrated in FIGS. 82 and 83, the freely rotative rasing stand 880 is supported by a ratative shaft 881 in the chamber 116. The ratative shaft 881 extends in a direction orthogonal to an axial line direction of the forceps channel 115. The rasing stand 880 has a distal end section standing from a starting point, i.e., a pivotally supported proximal end section. The rasing stand 880 and a slit 882 provided thereto be directed the distal end section as a whole form an angular U-shape. The slit 882 has a size that allows the coil sheath 76 of the instrument 803 to be inserted therethrough but the distal end-treating section 877.

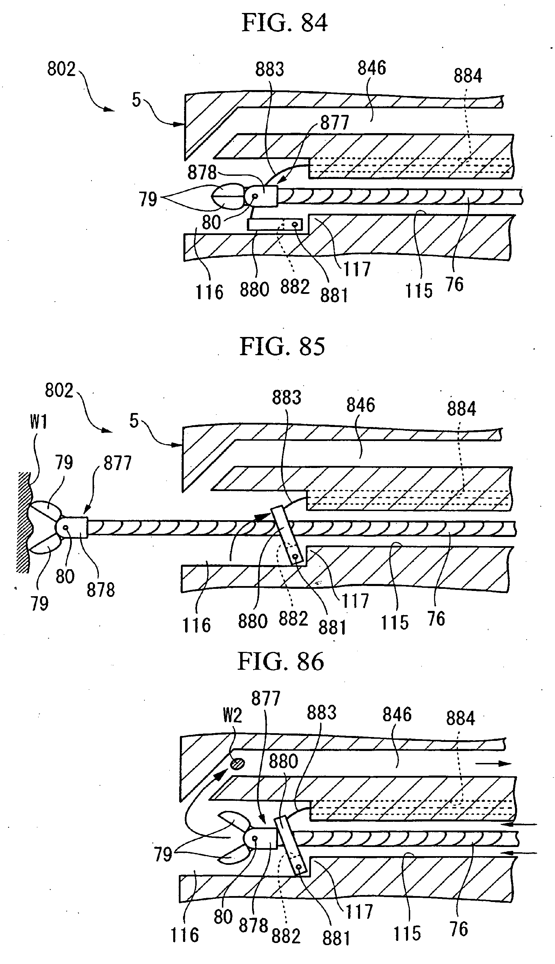

As illustrated in FIG. 84, attached to the distal end section of the rasing stand 880 is a raising wire 883 that passes through the maneuvering channel 884 and is attached to a lever, not shown in the drawing, of the endoscope-maneuvering section 4. The position where the ratative shaft 881 of the rasing stand 880 is installed and the position of an opening of the distal end of the maneuvering channel 884 are configured to place the forceps channel 115 therebetween.
Therefore, maneuvering of the raising wire 883 allows the rasing stand 880 to move to a retracting position where the rasing stand 880 is substantially in parallel to the axial line of the forceps channel 115 and to a standing position where the rasing stand 880 stands in a slanting manner to cross the axial line direction of the forceps channel 115.

Operations in the present embodiment are explained next as follows.
An uninserted state of the instrument 803 causes the check valve 370 and the check valve 373 to be in opening states and causes the check valve 371 and the check valve 372 to be in closing states. In addition, an end section of the tissue-suctioning pipeline 846 is blocked by a check valve. Therefore the endoscope 802 alone can conduct ordinary water-supply, air-supply, and suctioning.

Conducted upon inserting the instrument 803 are attaching the manifold 813 to the connector 860, connecting the suction tube 811, 812 to the corresponding tissue-suctioning pipe ways 846 and 847, and connecting the second pipe way system 830 to the tissue-grasping device 317. Similarly, connecting the signal line 862 from the suctioning-and water-supplying button 810 to the connector 861 allows the suctioning-and water-supplying button 810 to provide opening-and-closing control to the check valves 370 to 373.
The rasing stand 880 disposed at the retracting position in the vicinity of the distal end of the endoscope 802 retracting position causes the distal end-treating section 877 to project into the chamber 116. A part of the living tissue of the mucosa W1 is grasped upon projecting the instrument 803 pushed while maintaining the closed state of the pair of the autopsy cups 79 from the distal end of the endoscope 802.
The raising wire 883 is drawn upon maneuvering of a lever, not shown in the drawing. The rasing stand 880 rotates around the axial line and stands up as illustrated in FIG. 85. As illustrated in FIG. 83, the distal end-treating section 877 upon abutting to the rasing stand 880 is suspended since fully retracting the instrument 803 in order to tear the grasped tissue W2 allows the coil sheath 76 to pass through the slit 882 but does not allow to pass through the distal end-treating section 877. Accordingly the position of the distal end of the distal end-treating section 877 is fixed.

Advancing the slider 74 of the maneuvering section 871 and opening a pair of the autopsy cups 79 cause the slider 74 to simultaneously press the suctioning-and-water-supplying switch 810. The control signal sent to the endoscope 802 through the signal line 862 causes the check valve 371 and the check valve 372 to open and the check valve 370 and the check valve 373 to close. Water is supplied from the water-supplying tank 13 to the forceps channel 115 through the water-supplying pipeline 27, the tissue water-supply pipeline 328 and the suction pipeline 44. The liquid flowing flowing into the chamber 116 through a space defined by the abutment section 117, the instrument 803, and the rasing stand 880 causes the grasped tissue W2 to be separated from the autopsy cups 79.

The grasped tissue W2 separated from the autopsy cups 79 and suctioned into the tissue-suctioning pipeline 846 through the suction tube 811 is captured at a tissue-grasping surface 365A of the tissue-grasping device 317. The liquid or water sent to pass through the tissue-grasping surface 365A, the suction tube 812, and the tissue-suctioning pipeline 847, and the suction pipeline 41 is drained. Retracting the slider 74 sebsequent to fully capturing the tissue W causes the autopsy cups 79 to close. Accordingly, the suctioning-and-water-supplying switch 810 is turned off; thus, water-supply and suctioning are suspended. Subsequently, the rasing stand 880 is moved back to the retracting position, and after that, the instrument 803 is fully retracted to remove the instrument 803 from the endoscope 802.

Providing the water-supplying pipeline and the suctioning pipeline in the endoscope 802 according to the present embodiment can simplify the configuration of the insertion section 875 of the instrument 803. Mere opening and closing of the autopsy cups 79 by using the slider 74 permits water-supply and suctioning since the suctioning-and-water-supplying switch 810 that undertakes controlling of the check valves 370 to 373 that change flow paths is provided at a position that allows the slider 74 of the maneuvering section 871 of the instrument 803 to maneuver the suctioning-and-water-supplying switch 810, thereby facilitating operations.

Providing the rasing stand 880 to the chamber 116 of the forceps channel 115 allows the position of the distal end of the suction pipeline 114 to be fixed by raising the rasing stand 880. Accordingly, the position of the distal end of the instrument 803 can be fixed without providing a specific mechanism to the instrument 803. It is preferable to raise the rasing stand 880 in a slanting manner to obtain an adequate flow rate of water-supply. The abutment section 117 may not be provided to the forceps channel 115, i.e., the diameter of the forceps channel 115 until reaching the connecting point 45 may be the same as that of the chamber 116.

### (Ninth Embodiment)

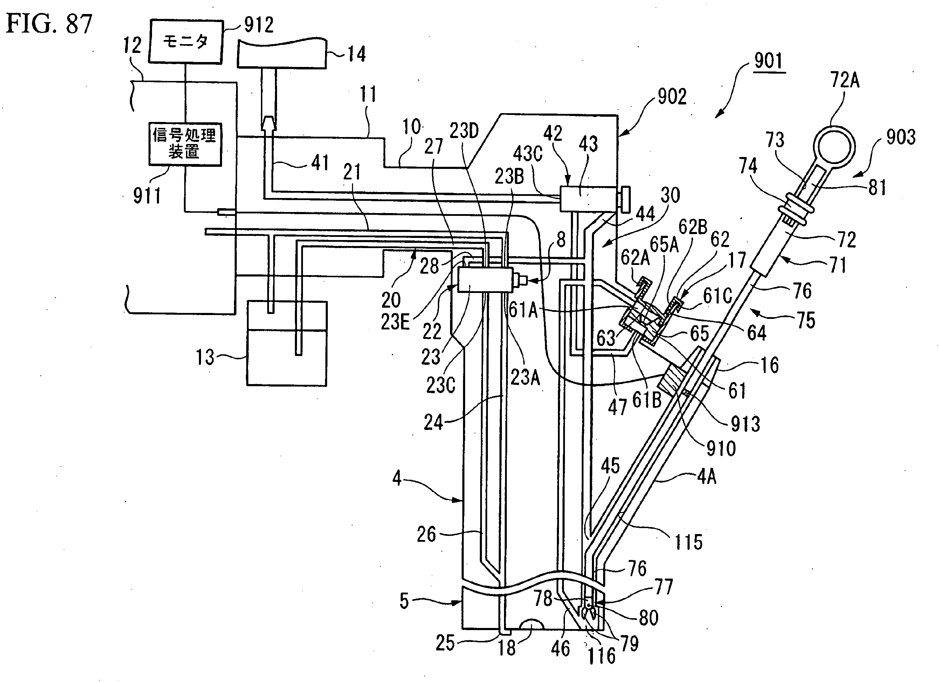

A ninth embodiment according to the present invention is explained as follows in detail with reference to FIGS. 87 to 90. Note that elements that are equivalent to those of the first embodiment will be assigned the same reference symbols and redundant explanations thereof will be omitted.
As illustrated in FIG. 87, an endoscope system 901 includes an endoscope 902 and an instrument 203 in configuration. The endoscope 902 is attached to the endoscope-maneuvering section 4 so that a detecting section of a photo-sensor 910 is exposed in a forceps channel 115. Output from the photo-sensor 910 is connected to a signal-processing device 911 in the controlling device 12. The signal-processing device 911 is connected to a monitor 912.

A mark 913 is an identification member provided to the insertion section 75 of the instrument 903. The mark 913 is produced from a high reflectivity material. The mark 913 of this type is provided at a position that allows the photo-sensor 910 to detect the mark 913 when the distal end-treating section 77 is disposed in the chamber 116 and the distal end of the autopsy cups 79 is disposed in the vicinity of the proximal end relative to the tissue-suctioning pipeline 46.

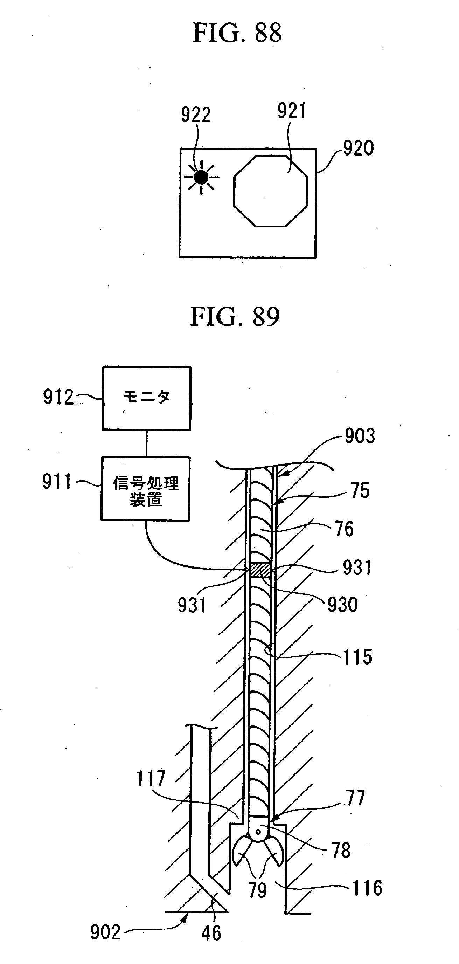

FIG. 88 shows an example of a monitor display 620 output on a monitor 912. Provided on the monitor display 620 are a display section 921 that displays an image of the inside of a body of a patient captured by an image-pickup unit provided to the distal end section of the endoscope 902; and a lamp 922. The lamp 922 is configured to light up when the photo-sensor 910 detects the mark 913. Altertively, the mark 913 may fully include the insertion section 75, or a part of the insertion section 75 may be free of the mark 913. In these cases, the lamp 922 lights up as long as the mark 913 is not detected by the photo-sensor 910.

Operations in the present embodiment are explained next as follows.
The instrument 903 is inserted into the endoscope 902, the autopsy cups 79 are opened and closed, and then the grasped tissue W2 is grasped. The instrument 903 is fully retracted and the grasped tissue W2 is torn from the mucosa W1, and then the distal end-treating section 77 is retracted into the forceps channel 115. The mark 913 provided to the insertion section 75 is detected by the photo-sensor 910 in the course of retracting of the distal end-treating section 77. the instrument 903 is suspended at the current position when process conducted by the signal-processing device 911 causes the lamp 922 of the monitor 912 to light up.
The autopsy cups 79 are opened while maintaining water-supply and suctioning, and the grasped tissue W2 is sent through the tissue-suctioning pipeline 46 and captured by the tissue-grasping device 17. After the grasped tissue W2 is fully captured, the autopsy cups 79 are closed and the instrument 903 is removed.

Mere observing of the monitor display 620 enables positioning of the distal end of the distal end-treating section 77 since the present embodiment is configured to detect the position of the distal end-treating section 77 by providing the mark 913 serving for a distal-end-regulating section to the instrument 903 and providing the photo-sensor 910 serving for a regulating section to the endoscope 902. The grasped tissue W2 can be collected reliably.

Another configuration in place of lighting up of the lamp 922 is a buzzer sound notification, etc. A lamp disposed in the vicinity of the forceps plug 16 may be easily acknowledged by a surgeon who maneuvers the instrument 903. A mark may similar to the mark 913 may be provided to the distal end of the insertion section 75 of the instrument 903, and a photo-sensor 910 may be provided to the distal end of the forceps channel 115. Positioning accuracy in this case can be improved since vatiations of the instrument 903 and the forceps channel 115 in length hardly affect the positioning accuracy.

A conductive material 930 serving for a distal-end-regulating section may be provided as illustrated in FIG. 89 in place of the mark. Sensors provided to the endoscope 902 in this case are two electrical contacts 931 that project from the forceps channel 115. The conductive material 930 and the electrical contact 931 are positioned so that electric current is applied to the twoelectrical contacts 931 through the conductive material 930 when the distal end-treating section 77 is disposed in the vicinity of the proximal end relative to a connecting position of the tissue-suctioning pipeline 46 in the chamber 116. That is, the two electrical contacts 931 are connected electrically via the conductive material 930 of the insertion section 75 in the course of retracting the instrument 903 maintaining the grasped state of the grasped tissue W2.
The signal-processing device 911 lights up the lamp 922 (cf. FIG. 88) of the monitor 912. The autopsy cups 79 are opened prior tor subsequent to water-supply and suctioning, and the grasped tissue W2 is sent through the tissue-suctioning pipeline 46 and captured by the tissue-grasping device 17. The endoscope system in this case can obtain the same effects as those of the previously-explained embodiments. Mere providing of electrical contacts can achieve low cost production.

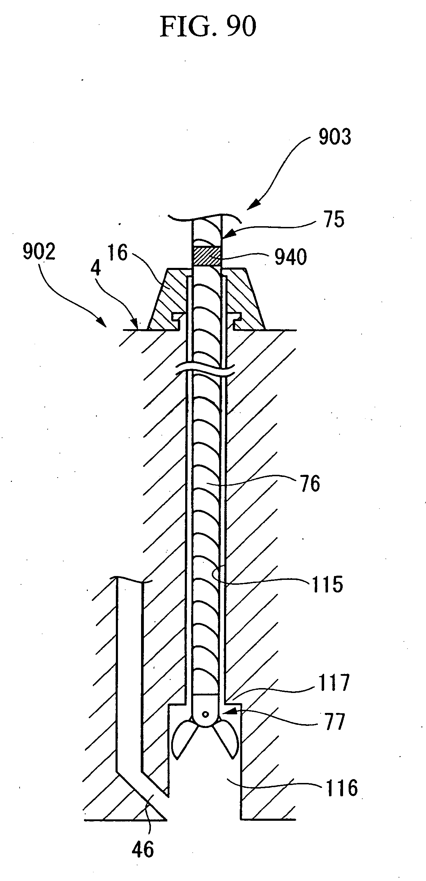

A distal-end-regulating section that can be observed in visual inspection may be a mark 940 provided to the insertion section 75 of the instrument 903 as illustratedin FIG. 90. The mark 940 is provided to be exposed outward from the forceps plug 16 when the distal end-treating section 77 is disposed in the vicinity of the proximal end relative to the connecting point of the tissue-suctioning pipeline 46 in the chamber 116. Maneuvering of grasping the grasped tissue W2 convinces with projecting of the distal end-treating section 77 from the distal end section of the endoscope 902.
Retracting the instrument 903 fully and tearing the grasped tissue W2 cause the mark 940 retracted in the forceps plug 16 to be exposed outward in accordance with retraction of the coil sheath 76. The retraction of the instrument 903 is suspended at the current position, and then the autopsy cups 79 are opened prior or subsequent to water-supply and suctioning. Accordingly, the grasped tissue W2 passing through the tissue-suctioning pipeline 46 is captured by the tissue-grasping device 17. The endoscope system in this case can obtain the same effects as those of the previously-explained embodiments, thereby additionally realizing low cost production and facilitating visual inspection for observing the position of the distal end-treating section 77 by a surgeon who maneuvers the instrument 903.

### (Tenth Embodiment)

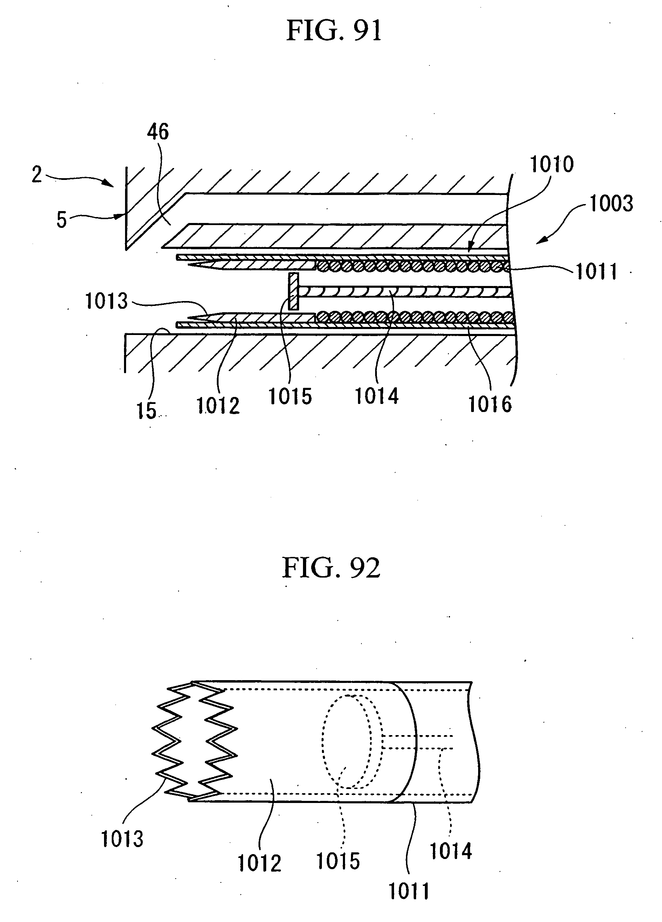

A tenth embodiment according to the present invention is explained as follows in detail with reference to FIGS. 91 to 95. Note that elements that are equivalent to those of the first embodiment will be assigned the same reference symbols and redundant explanations thereof will be omitted.

As illustrated in FIG. 91, the instrument 1003 has a long length of elastic insertion section 1010. A ring cutter 1012 is secured to the distal end section of a densely-wound coil sheath 1011 of the insertion section 1010. As illustrated in FIG. 92, a blade section 1013 provided to the distal end of the cutter 1012 having a sharp wave-shaped edge formed in a circumferential direction.
As illustrated in FIGS. 91 and 92, a maneuvering wire 1014 capable of freely advancing and retracting is inserted through the coil sheath 1011. A pusher 1015 is secured to the distal end of the maneuvering wire 1014. In addition, an outer sheath 1016 is capable of freely sliding on the outer periphery of the coil sheath 1011. Configuration associated with the maneuvering section 71 of the instrument 1003 and the endoscope 2 is the same as that of the first embodiment.

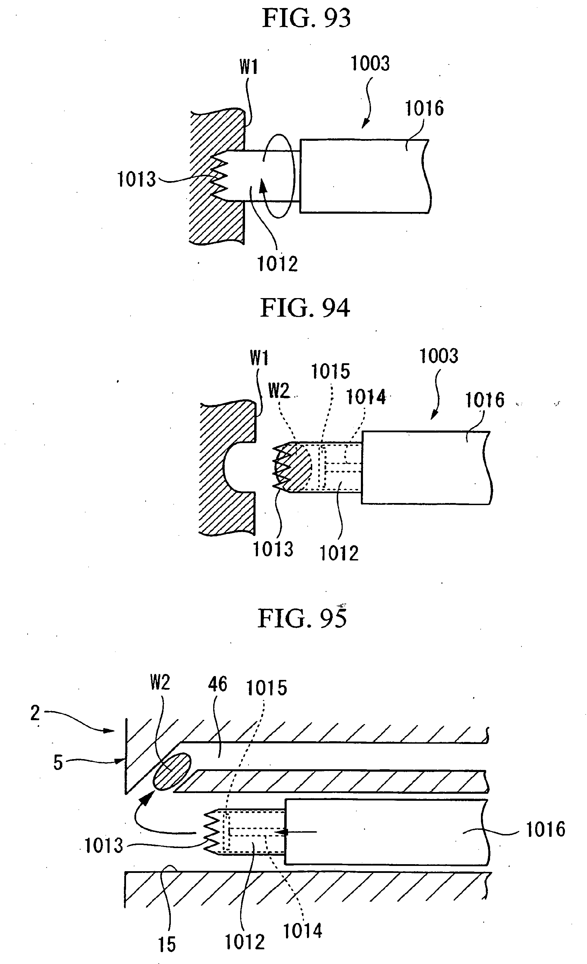

The pusher 1015 is retracted and the outer sheath 1016 is advanced to cover the cutter 1012 prior to inserting of the instrument 1003 into the forceps channel 15. Subsequently the maneuvering section main unit 72 is projected from the outer sheath 1016 at the moment of abutting the cutter 1012 onto the mucosa W1. The instrument 1003 upon abutting the cutter 1012 onto the mucosa W1 is rotated around an axial line as illustrated in FIG. 93. After that, fully retracting the instrument 1003 as illustrated in FIG. 94 causes the grasped tissue W2 to be captured into the cutter 1012.

Water-supply and suctioning are started upon retracting the cutter 1012 in the vicinity of the proximal end relative to the tissue-suctioning pipeline 46 in the endoscope 2, and then the slider 74 is advanced. The grasped tissue W2 in the cutter 1012 is squeezed out when the pusher 1015 advances. The grasped tissue W2 together with supplied water or liquid is suctioned into the tissue-suctioning pipeline 46 and captured by the tissue-grasping device 17. Serial collection of the grasped tissue W2 repeats these operations. The instrument 1003 is removed from the endoscope 2 upon finishing all the collection.

The present embodiment taking advantage of pipelines provided to the endoscope 2 and allowing the grasped tissue W2 to be collected in the exterior of the body of a patient can simplify the configuration of the instrument 1003 and achieving cost reduction. Greater diameter of the endoscope insertion section 5 can be prevented since an increased-diameter section is not necessary to be formed to the forceps channel 15.

### (Eleventh Embodiment)

An eleventh embodiment of the present invention is explained in details as follows with reference to the drawings. Note that elements that are equivalent to those of the first embodiment will be assigned the same reference symbols and redundant explanations thereof will be omitted.

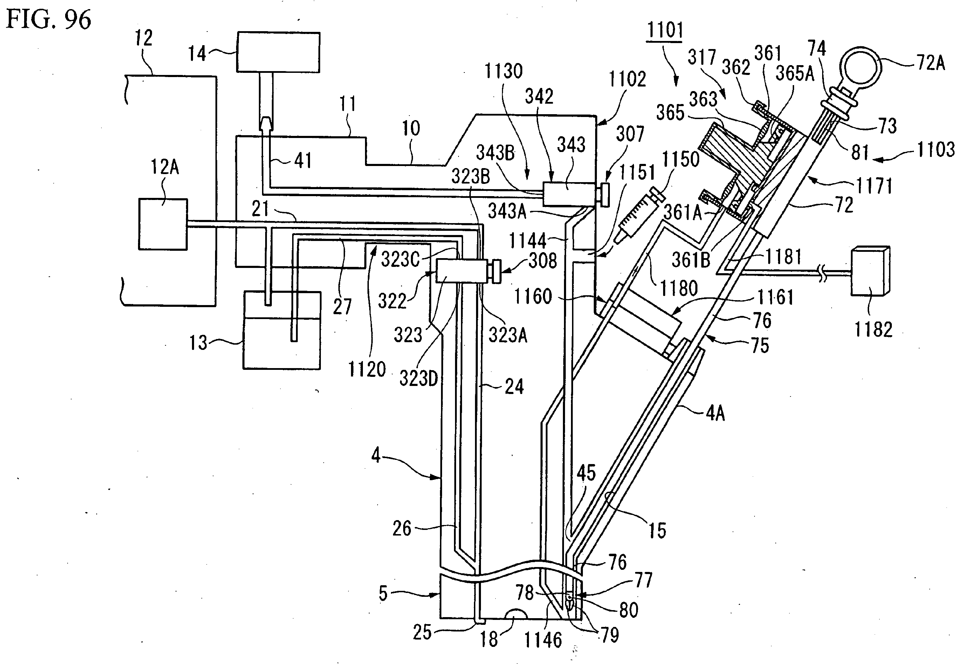

As illustrated in FIG. 96, an endoscope system 1101 includes an endoscope 1102 and an instrument 1103 in configuration. The endoscope 1102 has a first pipeline system 1120 and a second pipeline system 1130. A·air-supplying pipeline 21 of the first pipeline system 1120 is connected to a second port 323B of the first switching device 322. A first port 323A of the first switching device 322 is connected to an air-supplying pipeline 24.
The check valve 371 has a configuration illustrated in FIGS. 38 to 40. A water-supplying pipeline 26 is connected to the distal end of the air-supplying pipeline 24. The water-supplying pipeline 26 is connected to the fourth port 323D of the first switching device 322. A water-supplying pipeline 27 connected to the third port 323C of the first switching device 322 enables water-supply from the water-supplying tank 13.

The second pipeline system 1130 has a suction pipeline 41 connected to the suction source 14. The suction pipeline 41 is connected to the second port 343B of the second switching device 342. The second switching device 342 has a configuration illustrated in FIGS. 41 and 42. A suction pipeline 1144 is connectedt to the first port 343A of the second switching device 342. The suction pipeline 1144 is connected to a forceps channel 15. An outer port 1151 capable of undertaking insertion of the water-supplying syringe 1150 is formed in the middle of the suction pipeline 1144.
Preferably a check valve is provided to the outer port 1151 so that air-tight condition can be maintained in the suction pipeline 114 when the syringe 1150 is detached. In addition, a tissue-suctioning pipeline 1146 is connected to the distal end of the forceps channel 15 in a slanting manner. The tissue-suctioning pipeline 1146 has an opening in the connector 1160 provided to a lateral section 4A of the endoscope-maneuvering section 4. A connector 1161 of the instrument 1103 is attached to the opening. The configuration of the connectors 1160 and 1161 is similar to that o the connectors in the fourth embodiment.

A long length of insertion section 75 extends from a maneuvering section 1171 of the instrument 1103. A distal end-treating section 77 is provided to the distal end of the insertion section 75. The maneuvering section 1171 has a maneuvering section main unit 72. A tissue-grasping device 317 is fixed to the maneuvering section main unit 72. A suction tube 1180 is connected to an opening section 361A of a lateral section of a casing 361 of the tissue-grasping device 317. The suction tube 1180 is connected to a connector 1161.
The connector 1161 is configured to be capable of engaging with a connector 1160 of the endoscope 1102 and to cause the suction tube 1180 to communicate to the tissue-suctioning pipeline 1146. A suction tube 1181 is connected to an opening section 361B of a bottom section the casing 361 of the tissue-grasping device 317. A suctioning device 1182 is connected to the suction tube 1181 separately.

Operations in the present embodiment are explained next as follows. Ordinary air-supply is conducted by using the air-supplying pipeline 21, the first switching device 322, and the air-supplying pipeline 24 of the first pipeline system 1120. Ordinary water-supply is conducted through the water-supplying pipeline 27 of the first pipeline system 1120, the first switching device 322, the water-supplying pipeline 26, and the nozzle 25 of the distal end of the air-supplying pipeline 24. Also, ordinary suctioning operation is conducted through the suction pipeline 41 of the second pipeline system 1130, the second switching device 342, the suction pipeline 1144, and the forceps channel 15.

Collecting of the grasped tissue W2 by the endoscope system 1101 commences retracting of the instrument 1103 having grasped state of the grasped tissue W2 in the autopsy cups 79 fully into the forceps channel 15. The maneuvering section 1171 is maneuvered upon retracting the distal end sections of the autopsy cups 79 into the vicinity of proximal end relative to the connecting section of the tissue-suctioning pipeline 1146 to open the autopsy cups 79. Suctioning is started by driving this state of the suctioning device 1182. The suction button 307 of the second switching device 342 is positioned as illustrated in FIG. 41, and after that, the syringe 1150 is attached to the outer port 1151 of the suction pipeline 1144 of the second pipeline system 1130, and the liquid in the syringe 1150 is injected to the suction pipeline 1144.

The liquid injected from the syringe 1150 and flowing through the suction pipeline 1144 and the forceps channel 15 washes the grasped tissue W2 of the autopsy cups 79 of the distal end and causes the grasped tissue W2 to separate from the autopsy cups 79, thereby the grasped tissue W2 is suctioned into the tissue-suctioning pipeline 1146. The grasped tissue W2 is also suctioned into the tissue-suctioning pipeline 1146 through the suction tube 1180 is captured at a tissue-grasping surface 365A of the tissue-grasping device 317. Serial collection of the grasped tissue W2 repeats these operations. The instrument 1103 is removed from the endoscope 1102 upon finishing all the collection.

The present embodiment configured to supply water through the syringe 1150 to collect the grasped tissue W2 and suction the grasped tissue W2 by using a separately provided suctioning device 1182 can simplify configuration of pipelines in the endoscope 1102. Burden sharing enabling suctioning of the instrument 1103 and water-supply of the endoscope 1102 can reduce burden to a surgeon who maneuvers the instrument 1103.

It should be noted that the present invention is not limited to the above embodiment, i.e., the present invention can be applied to various aspects.
For example, the embodiments can be combined to obtain an endoscope, an endoscope instrument, and an endoscope system. A more specific example may be a configuration combining an endoscope having a first and second pipelines using check valves 370 to 371 and a forceps channel 15. Also, an instrument may have a tissue-grasping device; and one of a suctioning switch and a water-supplying switch.

Preferably, the chamber 116 of the forceps channels 15, 115, and 215 that permits opening and closing of the autopsy cups 79 of the distal end-treating sections 77 and 277 is produced from a material, e.g., metal or hard plastic that is harder than the proximal end of the forceps channels 15, 115, and 215. Accordingly, the forceps channels 15, 115, and 215 are prevented from waring when the autopsy cups 79 during opening and closing abuts to an inner surfaces of the forceps channel 15, 115, and 215.

The diameter of the opening of the distal end of the channel 115 may be reduced as long as the distal end-treating sections 77 and 277 can pass therethrough. The reduced diameter of the distal end section of the chamber 116 facilitates suctioining of supplied water or liquid.
The tissue-suctioning pipelines 46, 646, 846, and 1146 may be connected in various directions, e.g., a direction orthogonal to an axial direction instead of slant connection to the distal end sections of the forceps channels 15, 115, and 215.

### Industrial Applicability

The present invention can be applied to an endoscope or an endoscope system for collecting a living tissue.

## Claims

1. An endoscope (2) comprising:
an endoscope insertion section (5) used to be inserted into a human body; and
an endoscope-maneuvering section (4) maneuvered by a surgeon in the exterior of the human body, a channel (15) that allows insertion of an endoscope instrument (3) therethrough being formed from a distal end section of the endoscope insertion section (5) to the endoscope-maneuvering section (4), wherein
a tissue-suctioning pipeline (46) and the channel (15) are connected to each other in the vicinity of an opening of the distal end of the channel, and the tissue-suctioning pipeline (46) being capable of connecting to a suction source (14) that suctions a living tissue captured by the endoscope instrument (3).

2. The endoscope (2) according to Claim 1, wherein a space (116) is formed in the vicinity of the opening of the distal end of the channel (15), the space (116) being capable of allowing a forceps section (77) provided to the distal end of the endoscope instrument (3) to open and close therein.

3. The endoscope (2) according to Claim 2, wherein a regulating section (117) is provided for regulating the position of the forceps section (77) of the endoscope instrument (3) within the space (116).

4. The endoscope (2) according to Claim 3, wherein the regulating section (117) is a abutment section that is provided to project into the channel (15) and allows a part of the endoscope instrument (3) to abut thereon.

5. The endoscope (2) according to Claim 3, wherein the regulating section (117) is provided in the vicinity of the opening section of the channel of the endoscope-maneuvering section (4), the regulating section (117) being configured to be capable of engaging with a part of the endoscope instrument (3).

6. The endoscope (2) according to Claim 3, wherein the regulating section (117) has an abutment surface (117A) capable of abutting to the endoscope instrument (3) in a direction orthogonal to an axial line of the channel (15), the direction indicating retraction of the endoscope instrument (3).

7. The endoscope (2) according to Claim 3, wherein the regulating section (117) has a raising stand provided to be capable of freely raising in the channel (15), wherein a long length of sheath (76) of the endoscope instrument (3) can be inserted through the raising stand, and having a notch to engage the forceps section (77) provided at the distal end of the sheath (76).

8. The endoscope (2) according to Claim 2, wherein a sensor (910) for detecting the position of the forceps section (77) of the endoscope instrument (3) is provided in the channel (15), the sensor (910) being directed to the inside of the channel (15).

9. The endoscope (2) according to Claim 2, wherein the space (116) is formed by widening the distal end section of the channel (15).

10. The endoscope (2) according to Claim 2, wherein the space (116) is formed from a material harder than the other part of the channel (15).

11. The endoscope (2)according to Claim 1, wherein a tissue-grasping device (17) for capturing a living tissue collected by the endoscope instrument (3) is provided between the tissue-suctioning pipeline (46) and the suction source (14).

12. The endoscope (2) according to Claim 1, wherein a tissue water-supply line (28) is provided that is capable of connecting to a water-supplying tank (13) and supplying a liquid stored in the water-supplying tank (13) to the channel (15).

13. The endoscope (2) according to Claim 12 having a synchronization structure that conducts water-supply using the tissue water-supply line (28) and suctioning using the tissue-suctioning pipeline (46) synchronously.

14. The endoscope (2) according to Claim 1, wherein suction amount of the tissue-suctioning pipeline (46) is greater than water-supply amount of the tissue water-supply line (28).

## Patentansprüche

1. Endoskop (2) umfassend:
einen Endoskopeinführabschnitt (5), der dazu verwendet wird, in einen menschlichen Körper eingeführt zu werden; und
einen Endoskop-Bewegungsabschnitt (4), der durch einen Operateur außerhalb des menschlichen Körpers bewegt wird, einen Kanal (15), der das Einführen eines Endoskop-Instruments (3) **dadurch** ermöglicht und von einem distalen Endabschnitt des Endoskopeinführabschnitts (5) bis zu dem Endoskop-Bewegungsabschnitt (4) gebildet ist, wobei
eine Gewebe-Absaugrohrleitung (46) und der Kanal (15) miteinander in der Nähe einer Öffnung des distalen Endes des Kanals verbunden sind und die Gewebe-Absaugrohrleitung (46) dazu geeignet ist, mit einer Absaugquelle (14) verbunden zu sein, die ein von dem Endoskop-Instrument (3) gegriffenes lebendiges Gewebe absaugt.

2. Endoskop (2) nach Anspruch 1, wobei ein Raum (116) in der Nähe der Öffnung des distalen Endes des Kanals (15) gebildet ist, wobei der Raum (116) dazu geeignet ist, es einem an dem distalen Ende des Endoskop-Instruments (3) vorgesehenen Zangenabschnitt (77) zu ermöglichen, sich darin zu öffnen und zu schließen.

3. Endoskop (2) nach Anspruch 2, wobei ein Regulierungsabschnitt (117) zum Regulieren der Position des Zangenabschnitts (77) des Endoskop-Instruments (3) innerhalb des Raums (116) vorgesehen ist.

4. Endoskop (2) nach Anspruch 3, wobei der Regulierungsabschnitt (117) ein Anstoßabschnitt ist, der vorgesehen ist, um in den Kanal (15) vorzustehen und es einem Teil des Endoskop-Instruments (3) zu ermöglichen, daran anzustoßen.

5. Endoskop (2) nach Anspruch 3, wobei der Regulierungsabschnitt (117) in der Nähe des Öffnungsabschnitts des Kanals des Endoskop-Bewegungsabschnitts (4) vorgesehen ist, wobei der Regulierungsabschnitt (117) dazu ausgebildet ist, dazu geeignet zu sein, mit einem Teil des Endoskop-Instruments (3) zu verrasten.

6. Endoskop (2) nach Anspruch 3, wobei der Regulierungsabschnitt (117) eine Anstoßoberfläche (117A) aufweist, die dazu geeignet ist, mit dem Endoskop-Instrument (3) in einer Richtung senkrecht zu einer axialen Linie des Kanals (15) aneinander zu stoßen, wobei die Richtung ein Zurückziehen des Endoskop-Instruments (3) anzeigt.

7. Endoskop (2) nach Anspruch 3, wobei der Regulierungsabschnitt (117) einen Erhöhungssockel aufweist, der vorgesehen ist, um dazu geeignet zu sein, sich frei in dem Kanal (15) zu erheben, wobei eine lange Länge der Hülse (76) des Endoskop-Instruments (3) durch den Erhöhungssockel eingeführt werden kann, und eine Kerbe aufweist, um in den an dem distalen Ende der Hülse (76) vorgesehenen Zangenabschnitt (77) einzugreifen.

8. Endoskop (2) nach Anspruch 2, wobei ein Sensor (910) zum Bestimmen der Position des Zangenabschnitts (77) des Endoskop-Instruments (3) in dem Kanal (15) vorgesehen ist, wobei der Sensor (910) in das Innere des Kanals (15) gerichtet ist.

9. Endoskop (2) nach Anspruch 2, wobei der Raum (116) gebildet ist durch Weiten des distalen Endabschnitts des Kanals (15).

10. Endoskop (2) nach Anspruch 2, wobei der Raum (116) gebildet ist aus einem Material, das härter ist als der andere Teil des Kanals (15).

11. Endoskop (2) nach Anspruch 1, wobei ein Gewebe-Greifgerät (17) zum Ergreifen eines durch das Endoskop-Instrument (3) gesammelten lebenden Gewebes zwischen der Gewebe-Absaugrohrleitung (46) und der Absaugquelle (14) vorgesehen ist.

12. Endoskop (2) nach Anspruch 2, wobei eine Gewebe-Wasserversorgungsleitung (28) vorgesehen ist, die dazu geeignet ist, mit einem Wasserversorgungstank (13) verbunden zu sein und eine in dem Wasserversorgungstank (13) gespeicherte Flüssigkeit an den Kanal (15) zu liefern.

13. Endoskop (2) nach Anspruch 12 mit einer Synchronisierungsstruktur, die eine Wasserversorgung unter Verwendung der Gewebe-Wasserversorgungsleitung (28) und ein Absaugen unter Verwendung der Gewebe-Absaugrohrleitung (46) synchron ausführt.

14. Endoskop (2) nach Anspruch 1, wobei eine Absaugmenge der Gewebe-Absaugrohrleitung (46) größer ist als eine Wasserversorgungsmenge der Gewebe-Wasserversorgungsleitung (28).

## Revendications

1. Endoscope (2) comprenant :
une section d'insertion d'endoscope (5) utilisée pour être insérée à l'intérieur d'un corps humain ; et
une section de manoeuvre d'endoscope (4) manoeuvrée par un chirurgien à l'extérieur du corps humain, un canal (15) qui permet l'insertion d'un instrument d'endoscope (3) à travers celui-ci étant formé d'une section d'extrémité distale de la section d'insertion d'endoscope (5) jusqu'à la section de manoeuvre d'endoscope (4), dans lequel
une canalisation d'aspiration de tissu (46) et le canal (15) sont connectés l'un à l'autre à proximité d'une ouverture de l'extrémité distale du canal, et la canalisation d'aspiration de tissu (46) étant apte à se connecter à une source d'aspiration (14) qui aspire un tissu vivant capturé par l'instrument d'endoscope (3).

2. Endoscope (2) selon la revendication 1, dans lequel un espace (116) est formé à proximité de l'ouverture de l'extrémité distale du canal (15), l'espace (116) étant apte à permettre qu'une section de forceps (77) prévue sur l'extrémité distale de l'instrument d'endoscope (3) s'ouvre et se ferme dans celui-ci.

3. Endoscope (2) selon la revendication 2, dans lequel une section de régulation (117) est prévue pour réguler la position de la section de forceps (77) de l'instrument d'endoscope (3) à l'intérieur de l'espace (116).

4. Endoscope (2) selon la revendication 3, dans lequel la section de régulation (117) est une section de butée qui est prévue pour se projeter à l'intérieur du canal (15) et permet qu'une partie de l'instrument d'endoscope (3) vienne buter sur celle-ci.

5. Endoscope (2) selon la revendication 3, dans lequel la section de régulation (117) est prévue à proximité de la section d'ouverture du canal de la section de manoeuvre d'endoscope (4), la section de régulation (117) étant configurée pour être apte à s'engager avec une partie de l'instrument d'endoscope (3).

6. Endoscope (2) selon la revendication 3, dans lequel la section de régulation (117) a une surface de butée (117A) apte à venir buter sur l'instrument d'endoscope (3) dans une direction orthogonale à une ligne axiale du canal (15), la direction indiquant le retrait de l'instrument d'endoscope (3).

7. Endoscope (2) selon la revendication 3, dans lequel la section de régulation (117) a un support surélevé prévu de façon à être apte à se surélever librement dans le canal (15), dans lequel une longueur longue de gaine (76) de l'instrument d'endoscope (3) peut être insérée à travers le support surélevé, et ayant une entaille pour engager la section de forceps (77) prévue au niveau de l'extrémité distale de la gaine (76).

8. Endoscope (2) selon la revendication 2, dans lequel un capteur (910) pour détecter la position de la section de forceps (77) de l'instrument d'endoscope (3) est prévu dans le canal (15), le capteur (910) étant dirigé vers l'intérieur du canal (15).

9. Endoscope (2) selon la revendication 2, dans lequel l'espace (116) est formé par un élargissement de la section d'extrémité distale du canal (15).

10. Endoscope (2) selon la revendication 2, dans lequel l'espace (116) est formé d'un matériau plus dur que l'autre partie du canal (15).

11. Endoscope (2) selon la revendication 1, dans lequel un dispositif de préhension de tissu (17) pour capturer un tissu vivant collecté par l'instrument d'endoscope (3) est prévu entre la canalisation d'aspiration de tissu (46) et la source d'aspiration (14).

12. Endoscope (2) selon la revendication 1, dans lequel une conduite d'alimentation d'eau pour tissu (28) est prévue qui est apte à se connecter à un réservoir d'alimentation d'eau (13) et à amener un liquide stocké dans le réservoir d'alimentation d'eau (13) jusqu'au canal (15).

13. Endoscope (2) selon la revendication 12, ayant une structure de synchronisation qui conduit une alimentation d'eau en utilisant la conduite d'alimentation d'eau pour tissu (28) et aspire en utilisant la canalisation d'aspiration de tissu (46) de façon synchrone.

14. Endoscope (2) selon la revendication 1, dans lequel une quantité d'aspiration de la canalisation d'aspiration de tissu (46) est plus grande qu'une quantité d'alimentation d'eau de la conduite d'alimentation d'eau pour tissu (28).
